# FASCICULE DE BREVET EUROPEEN

(11) **EP 1 756 119 B1**
(45) Date de publication et mention de la délivrance du brevet: **10.10.2007**
(21) Numéro de dépôt: 05778643.6
(22) Date de dépôt: 15.06.2005
(51) Int. Cl.: C07D 497/10, C07D 295/14, C07D 277/40, C07D 241/26, C07D 213/56, C07D 207/16

(54) **COLLECTIONS DE COMPOSES TRACABLES ET LEURS UTILISATIONS**
kOLLEKTION NACHWEISBARER VERBINDUNGEN UND DEREN VERWENDUNG
COLLECTION OF TRACEABLE COMPOUNDS AND USES THEREOF

(30) Priorité: 15.06.2004 FR 0406465
(43) Date de publication de la demande: 28.02.2007
(73) Titulaire: Centre National de la Recherche Scientifique, 75794 Paris Cedex (FR); Université Louis Pasteur de Strasbourg, F-67000 Strasbourg (FR)
(72) Inventeur: HIBERT, Marcel, François, Louis, F-67114 Eschau (FR); FRANCHET, Christel, Anne, F-67120 Ernolsheim-Bruche (FR); GALZI, Jean-Luc, F-67500 Weitbruch (FR); PATTUS, Franc, Emile, Jean, F-67000 Strasbourg (FR); GUILLIER, Fabrice, Yves, F-67118 Greispolsheim (FR)
(74) Mandataire: Grosset-Fournier, Chantal Catherine
(86) Numéro de dépôt international: PCT/FR2005/001501
(87) Numéro de publication internationale: WO 2006/003329

(56) Documents cités:
- WO-A-02/072613
- FR-A- 2 841 246

## Description

La présente invention a pour objet des collections de composés traçables, notamment de composés fluorescents, ainsi que leurs utilisations, notamment dans le cadre de la détermination de ligands d'un récepteur dont on ne connaît pas de ligand ou dont on ne connaît pas de ligand utilisable pour les études de liaison d'affinité spécifique.

Le gène codant pour une protéine fluorescente issue de la méduse *Aequorea victoria,* la protéine fluorescente verte (green fluorescent protein ou GFP) a été déchiffré par Prasher et al. (Gene, 1992, 111:229-233). La GFP est une protéine monomérique. Elle acquiert ses propriétés de fluorescence par un mécanisme autocatalytique de formation du fluorophore.

De nombreux médicaments et substances naturelles exercent leur action en interagissant avec des protéines régulatrices appelées récepteurs, impliquées dans de nombreuses fonctions physiologiques des organismes, et les altérations de leurs fonctions sont la cause de nombreuses pathologies. L'accessibilité des récepteurs aux agents pharmacologiques endogènes ou exogènes, naturels ou synthétiques, depuis l'extérieur de la cellule conduit à les considérer comme des cibles de choix pour la recherche de molécules biologiquement actives, en particulier de molécules présentant des pouvoirs thérapeutiques potentiels.

Le décryptage du génome humain donne accès à la séquence de centaines de protéines nouvelles dont on ne connaît ni le ligand endogène ni la fonction biologique. Ces protéines, dites "orphelines", constituent les sites potentiels d'action de médicaments.

Les méthodes existantes à ce jour ne permettent de détecter que des molécules agonistes qui activent une fonction associée à la protéine. Ce type d'essai fonctionnel conduit à de nombreux faux positifs coûteux en temps et en argent. Par ailleurs, les molécules antagonistes qui inhiberaient la fonction associée à la protéine ne peuvent pas être détectées directement par ces méthodes. Or, les antagonistes présentent la plupart du temps le meilleur potentiel thérapeutique.

La présente invention a pour but de proposer un procédé de détermination d'un ligand quelconque, qu'il soit endogène ou exogène, permettant de s'affranchir de la recherche systématique du ligand endogène ou naturel.

L'un des buts de l'invention est de fournir un procédé de criblage pour déterminer les premiers ligands d'un récepteur orphelin sans connaître son ligand endogène.

La présente invention concerne l'utilisation d'une collection de composés répondant à la formule générale (I) suivante : 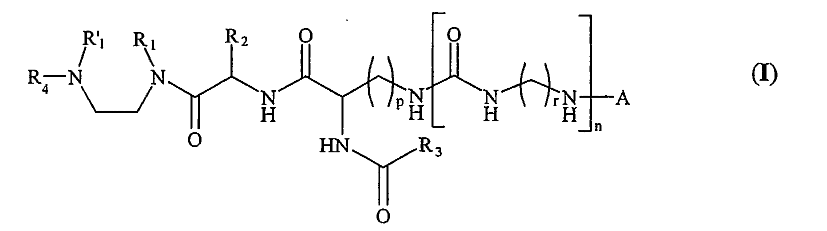 dans laquelle :
- n est égal à 0 ou 1,
- p représente un nombre entier variant de 1 à 6, et est de préférence égal à 4,
- r représente un nombre entier variant de 1 à 12, et est de préférence égal à 4,
- R₁ et R'₁ représentent indépendamment l'un de l'autre un atome d'hydrogène, un groupe alkyle comprenant de 1 à 6 atomes de carbone, ou un groupe aromatique, notamment un groupe benzyle ou un groupe phénéthyle, éventuellement substitués par un atome d'halogène, un groupe méthoxy ou un groupe alkyle comprenant de 1 à 6 atomes de carbone,
ou R₁ et R'₁ peuvent former un cycle comprenant de 2 à 4 atomes de carbone, notamment de 2 atomes de carbone,
- R₂ représente une chaîne latérale d'acide aminé ou d'un dérivé d'acide aminé,
- R₃ représente un groupe dérivé d'un acide carboxylique, porteur d'une entité basique, notamment choisi parmi les groupes suivants : 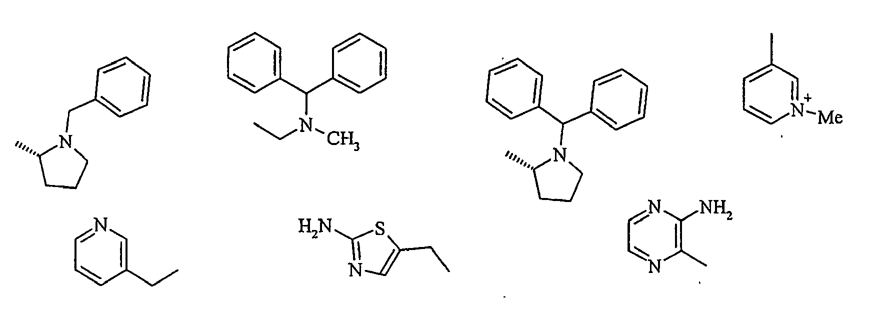
- R₄ représente un groupe alkyle comprenant de 1 à 10 atomes de carbone, notamment de 1 à 6 atomes de carbone, un groupe phényle substitué par un groupe alkyle comprenant de 1 à 10 atomes de carbone, notamment de 1 à 6 atomes de carbone, R₄ étant de préférence un groupe méthyle, un groupe benzyle ou un groupe allyle, et
- A représente un atome d'hydrogène, un groupe protecteur ou un groupement traceur, notamment un fluorophore, un colorant ou un "quencher",
pour la détermination *in vitro,* par étude de liaison, de ligands de récepteurs dont on ne connaît pas de ligand ou dont on ne connaît pas de ligand utilisable pour effectuer des essais de liaison d'affinité spécifique.

L'expression "collection de composés" désigne un ensemble de molécules pouvant être préparées selon un protocole commun.

L'expression "groupe traceur" désigne une entité chimique fluorescente ou une entité chimique possédant des propriétés de colorant ou d'absorption de certaines longueurs d'onde.

L'expression "récepteur dont on ne connaît pas de ligand" désigne toute macromolécule biologique ("récepteur") pour laquelle on ne connaît pas de molécules endogène ou exogène venant s'y lier de manière réversible.

L'expression "essai de liaison d'affinité spécifique" désigne la mesure de la force d'interaction entre deux molécules, à savoir un récepteur et son ligand.

La présente invention implique la conception, la synthèse et le criblage de collections de molécules fluorescentes sur des récepteurs rendus eux-mêmes fluorescents notamment par fusion à la protéine fluorescente verte GFP.

La présente invention concerne également un composé de formule (I) suivante : 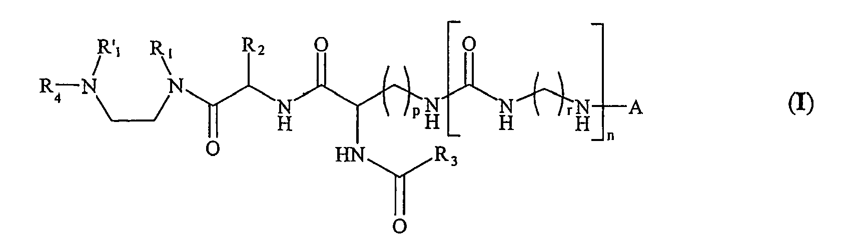 dans laquelle :
- n est égal à 0 ou 1,
- p représente un nombre entier variant de 1 à 6, et est de préférence égal à 4,
- r représente un nombre entier variant de 1 à 12, et est de préférence égal à 4,
- R₁ et R'₁ représentent indépendamment l'un de l'autre un atome d'hydrogène, un groupe alkyle comprenant de 1 à 6 atomes de carbone, ou un groupe aromatique, notamment un groupe benzyle ou un groupe phénéthyle, éventuellement substitués par un atome d'halogène, un groupe méthoxy ou un groupe alkyle comprenant de 1 à 6 atomes de carbone,
ou R₁ et R' ₁ peuvent former un cycle comprenant de 2 à 4 atomes de carbone, notamment de 2 atomes de carbone,
- R₂ représente une chaîne latérale d'acide aminé ou d'un dérivé d'acide aminé,
- R₃ représente un groupe dérivé d'un acide carboxylique, porteur d'une entité basique, notamment choisi parmi les groupes suivants : 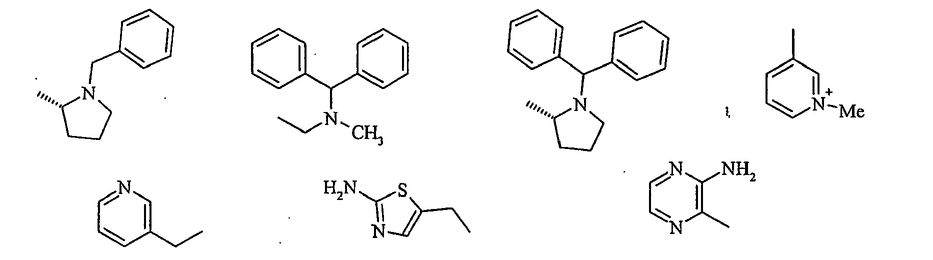
- R₄ représente un groupe alkyle comprenant de 1 à 10 atomes de carbone, notamment de 1 à 6 atomes de carbone, un groupe phényle substitué par un groupe alkyle comprenant de 1 à 10 atomes de carbone, notamment de 1 à 6 atomes de carbone, R₄ étant de préférence un groupe méthyle, un groupe benzyle ou un groupe allyle, et
- A représente un atome d'hydrogène, un groupe protecteur ou un groupement traceur, notamment un fluorophore, un colorant ou un "quencher".

La présente invention concerne un composé de formule (I) telle que définie ci-dessus, caractérisé en ce que R₂ représente la chaîne latérale d'un acide aminé ou dérivé d'acide aminé choisi dans le groupe comprenant : l'alanine, la glutamine, la leucine, la glycine, le tryptophane, la β-alanine, la phénylalanine, la 4-chloro-phénylalanine, l'acide isonipécotinique, l'acide 4-aminométhylbenzoïque, l'acide 3-tétrahydroisoquinoléinique et l'histidine libre ou benzylée.

Plus précisément, l'acide aminé ou dérivé d'acide aminé dont la chaîne latérale correspond à R₂ est choisi parmi :

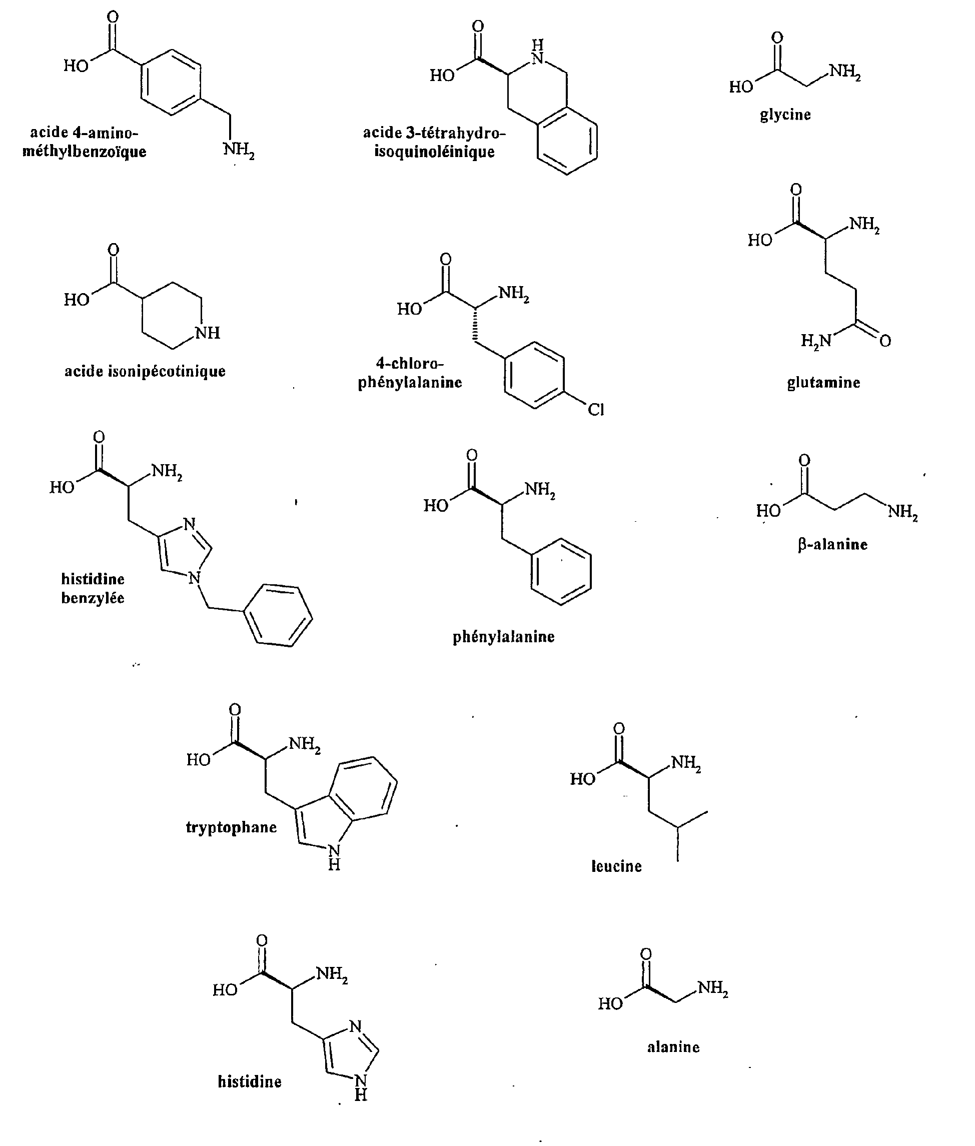

Un composé avantageux selon l'invention est un composé de formule (I) telle que définie précédemment dans laquelle n est égal à 0.

Un tel composé répond à la formule (I-a) suivante :

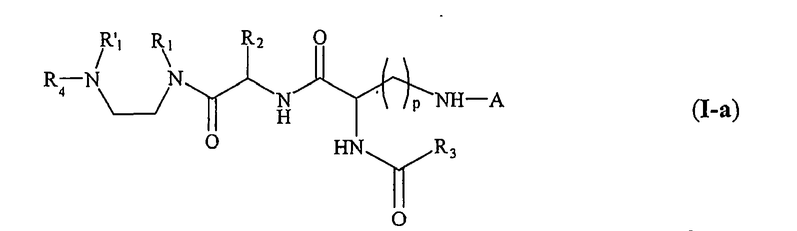

Un tel composé est avantageux dans la mesure où il présente une faible masse moléculaire.

Un composé avantageux selon l'invention est un composé de formule (I) telle que définie précédemment dans laquelle n est égal à 1.

Un tel composé répond à la formule (I-b) suivante :

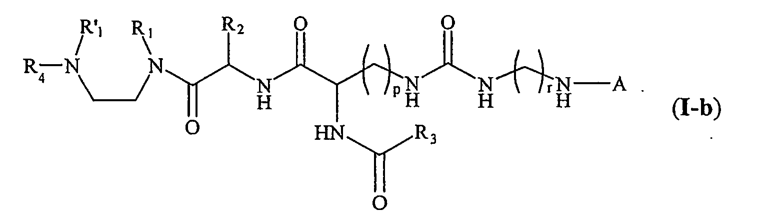

Un tel composé est avantageux dans la mesure où il présente une faible masse moléculaire.

Un composé avantageux selon l'invention est un composé de formule (I-a) telle que définie ci-dessus, caractérisé en ce que A représente un atome d'hydrogène ou un groupe protecteur (GP), notamment un groupe Boc.

Un tel composé répond à l'une des formules suivantes :

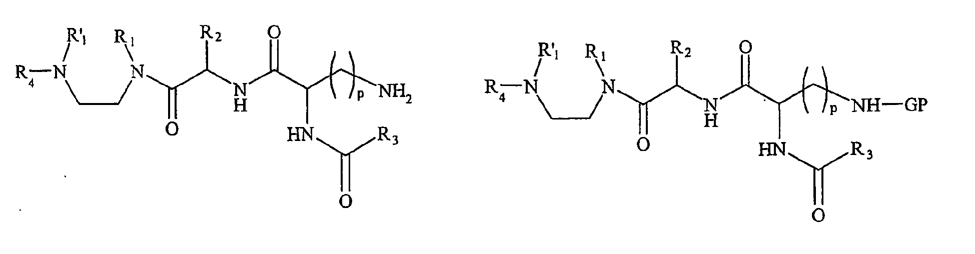

Selon un mode de réalisation avantageux de la présente invention, la présente invention concerne un composé tel que défini ci-dessus répondant à l'une des formules suivantes : 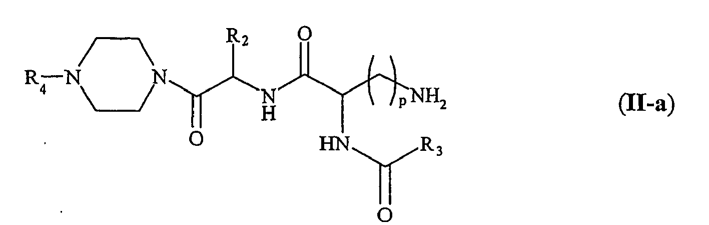 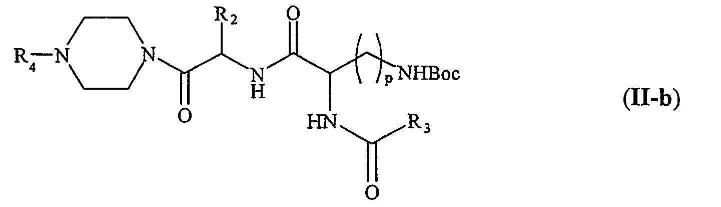 dans lesquelles R₂, R₃, R₄ et p sont tels que définis ci-dessus, p étant de préférence égal à 4.

Les composés susmentionnés de formule (II-a) ou (II-b) sont avantageux dans la mesure où ils présentent des motifs pipérazine, porteurs d'activité biologique.

La présente invention concerne également un composé tel que défini ci-dessus, de formule (I-a) ou (I-b), caractérisé en ce que A représente un groupe fluorophore choisi parmi le groupe comprenant les dérivés du Bodipy et de la lissamine.

Selon un mode de réalisation avantageux, les composés de l'invention de formule (I-a) ou (I-b) sont caractérisés en ce que A représente l'un des groupes suivants :

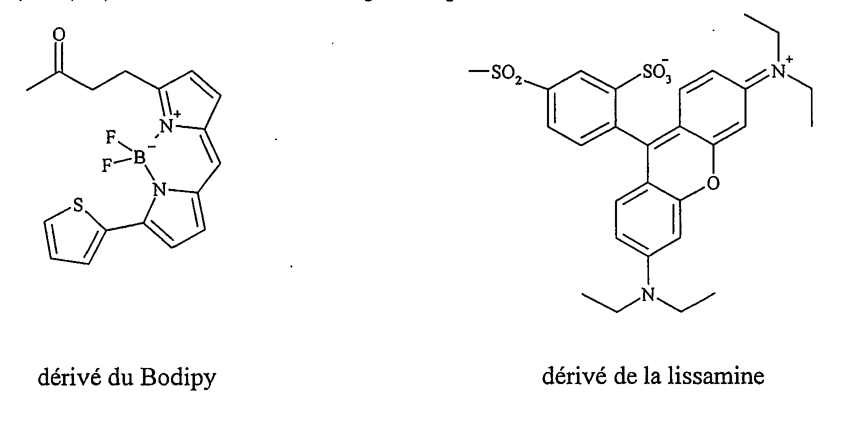

Un composé particulièrement avantageux selon l'invention est un composé tel que défini ci-dessus, répondant à la formule (III) suivante : 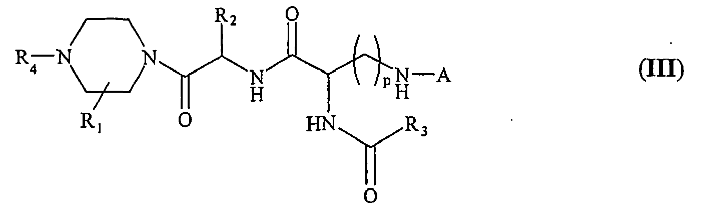 dans laquelle :
- R₁, R₂, R₃, R₄ et p sont tels que définis ci-dessus dans la formule (I), et
- A est tel que défini ci-dessus, c'est-à-dire qu'il représente un groupe fluorophore choisi parmi les dérivés du Bodipy et de la lissamine.

Selon un mode de réalisation avantageux de l'invention, la présente invention concerne un composé de formule (III), caractérisé en ce que p = 4 et répondant à la formule suivante : 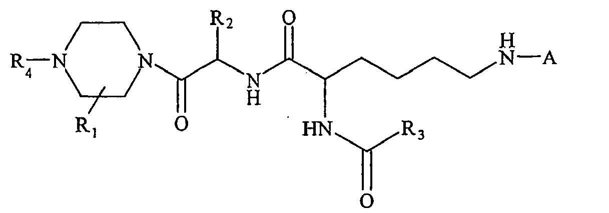 dans laquelle R₁, R₂, R₃ et R₄ sont tels que définis ci-dessus.

La présente invention concerne également un composé tel que défini ci-dessus, répondant à la formule (III-a) suivante : 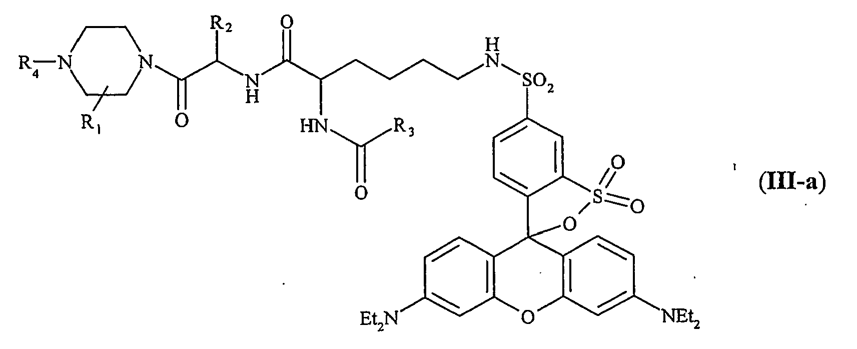 dans laquelle R₁, R₂, R₃ et R₄ sont tels que définis ci-dessus.

Un composé particulièrement avantageux selon l'invention est un composé tel que défini ci-dessus, répondant à la formule (IV) suivante : 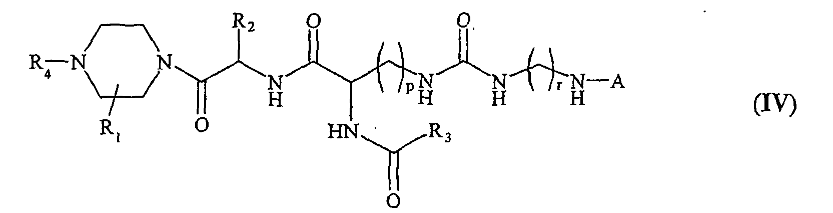 dans laquelle R₁, R₂, R₃, R₄, A, p et r sont tels que définis ci-dessus.

Un composé avantageux de l'invention est un composé de formule (IV) susmentionnée dans laquelle p = 4, et répondant à la formule suivante :

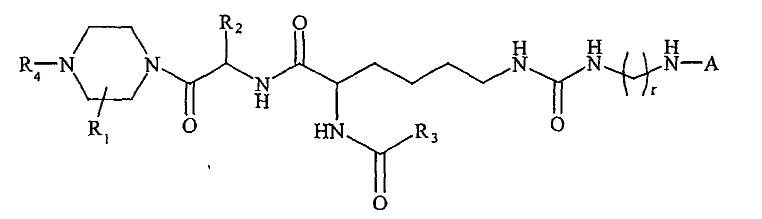

Un composé avantageux de l'invention est un composé de formule (IV) susmentionnée dans laquelle r = 4, et répondant à la formule suivante :

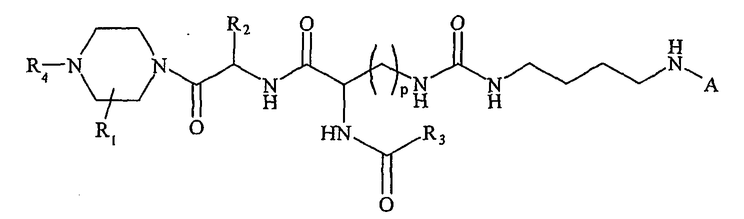

Un composé avantageux de l'invention est un composé de formule (IV) susmentionnée dans laquelle p et r sont égaux à 4, et répondant à la formule suivante :

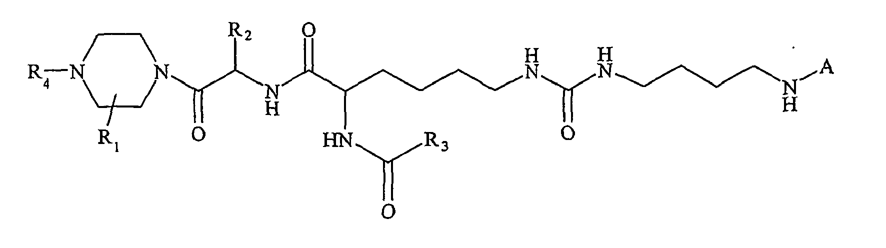

Un composé avantageux de l'invention est un composé tel que défini ci-dessus, répondant à la formule (IV-a) suivante : 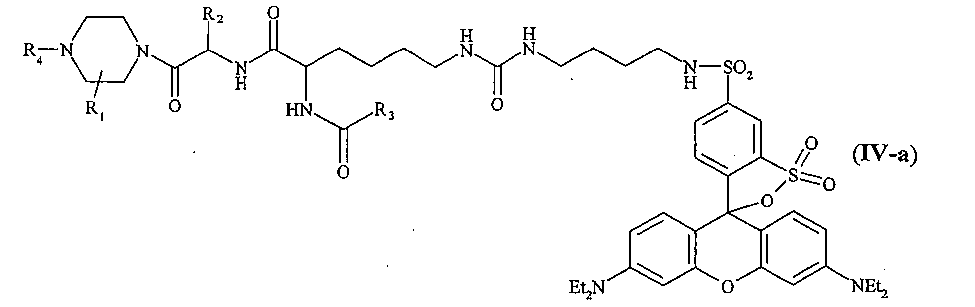 dans laquelle R₁, R₂, R₃ et R₄ sont tels que définis ci-dessus.

La présente invention concerne également une collection comprenant une pluralité de composés tels que définis ci-dessus.

L'expression "collection" désigne l'ensemble des molécules pouvant être préparées selon un protocole commun.

La présente invention concerne également une collection comprenant une pluralité de composés tels que définis ci-dessus, répondant à la formule (III-a).

La présente invention concerne également une collection comprenant une pluralité de composés tels que définis ci-dessus, répondant à la formule (IV-a).

La présente invention concerne également l'utilisation d'une collection telle que définie ci-dessus, pour la détermination *in vitro,* par étude de liaison, de ligands de récepteurs dont on ne connaît pas de ligand ou dont on ne connaît pas de ligand utilisable pour effectuer des études de liaison spécifique.

La présente invention concerne un procédé de criblage de ligands de récepteurs dont on ne connaît pas de ligand ou dont on ne connaît pas de ligand utilisable, ledit procédé comprenant les étapes suivantes :
- la mise en présence d'une collection de composés traçables selon l'invention, avec des cellules transfectées par une construction contenant la fusion de la séquence codant pour une protéine fluorescente, avec la séquence nucléotidique codant pour un récepteur dont on ne connaît pas de ligand ou dont on ne connaît pas de ligand utilisable, et le mélange desdites cellules et de ladite collection,
- la détection de la fluorescence dudit mélange, par excitation de ladite protéine fluorescente et mesure de la fluorescence d'émission de ladite protéine fluorescente, et la détermination du pourcentage d'extinction de fluorescence en comparant la fluorescence d'émission de ladite protéine fluorescente dans le mélange à la fluorescence moyenne de ladite protéine fluorescente en l'absence de ligand,
la fluorescence moyenne de ladite protéine fluorescente étant mesurée par des essais témoins correspondant à la mesure de la fluorescence de la protéine fluorescente en l'absence de la collection de composés, et
- la détermination des composés qui donnent un pourcentage d'extinction de la fluorescence de la protéine fluorescente d'au moins 5% et leur identification en tant que ligand.

La présente invention concerne plus particulièrement un procédé de criblage de ligands de récepteurs couplés aux protéines G (RCPG) tels que GPR50, GPR37, GPR19, GPR15, GPR31, GPR81, GPR3 et EDG7, le récepteur APJ, FPRL1, les récepteurs des chimiokines CXCR1, CXCR2, CXCR3, CXCR4, les récepteurs des peptides Glucagon-like (GLP-1R et GLP-2R), les récepteurs CRF1 et CRF2, les récepteurs métabotropiques au glutamate (mGluR) et les récepteurs au GABA.

La présente invention concerne également un procédé de criblage de ligands de RCPG dont on ne connaît pas de ligands tels que les récepteurs orphelins GPR50, GPR37, GPR19, GPR31, GPR81, GPR3 et EDG7.

La présente invention concerne aussi un procédé de criblage de ligands de RCPG dont on ne connaît pas de ligands utilisables selon le procédé de l'invention tels que le récepteur APJ, FPRL1, les récepteurs des chimiokines, CXCR1, CXCR2, CXCR3, CXCR4, les récepteurs des peptides Glucagon-like (GLP-1R et GLP2-R), CRF1, CRF2, les récepteurs métabotropiques au glutamate (mGluR) et les récepteurs au GABA.

La présente invention concerne également un procédé de préparation sur support solide d'un composé de formule (I), caractérisé en ce qu'il comprend les étapes suivantes :
- le couplage d'un support solide de formule 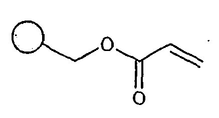 avec un composé de formule 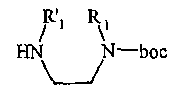 pour obtenir un composé de formule (1) suivante : 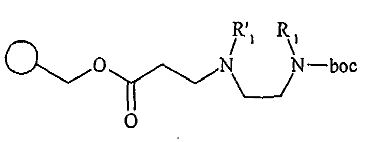 R₁ et R' ₁ étant tels que définis ci-dessus,
- la déprotection du composé de formule (1) suivie du couplage dudit composé déprotégé avec le composé de formule 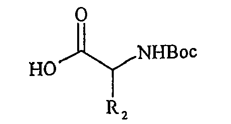 pour obtenir un composé de formule (2) suivante : 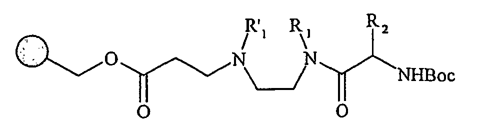 R₂ étant tel que défini ci-dessus,
- la déprotection du composé de formule (2) suivie du couplage dudit composé déprotégé avec le composé de formule 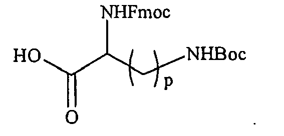 pour obtenir un composé de formule (3) suivante : 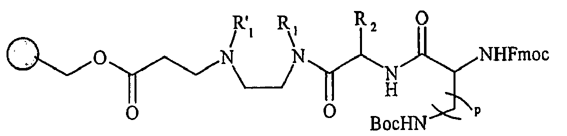 p étant tel que défini ci-dessus,
- la déprotection du groupe Fmoc du composé de formule (3) suivie du couplage dudit composé déprotégé avec le composé R₃COOH pour obtenir un composé de formule (4) suivante : 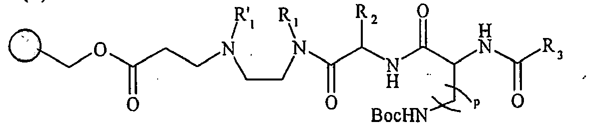 R₃ étant tel que défini ci-dessus,
- la déprotection du composé de formule (4) afin d'obtenir un composé de formule (5) suivante : 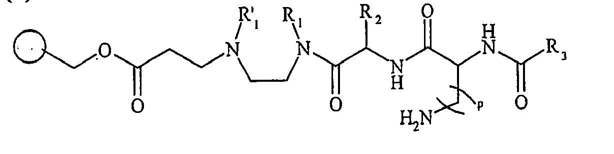
- éventuellement la réaction du composé de formule (5) avec le p-nitrophénylchloroformate puis avec la diamine de formule 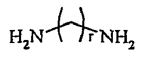 pour obtenir le composé de formule (6) suivante : 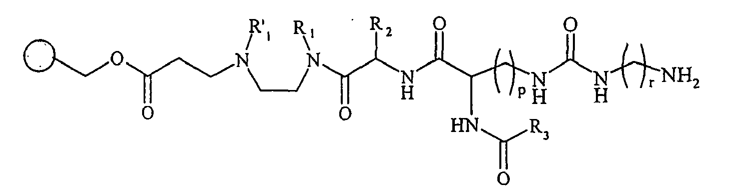 r étant tel que défini ci-dessus,
- la réaction du composé de formule (5) ou du composé de formule (6) avec des traceurs électrophiles, notamment avec A-Cl, pour obtenir le composé de formule (7) suivante : 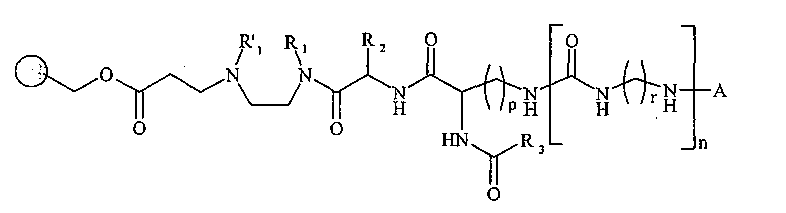 n étant égal à 0 ou 1,
   A représentant le Bodipy sous forme d'acide activé ou la lissamine sous forme d'acide sulfonique activé,
- le clivage du composé de formule (7) avec le composé R₄X, R₄ étant tel que défini ci-dessus, et X représentant un atome d'halogène, notamment I ou Br, pour obtenir un composé de formule (I) tel que défini ci-dessus.

### DESCRIPTION DES FIGURES

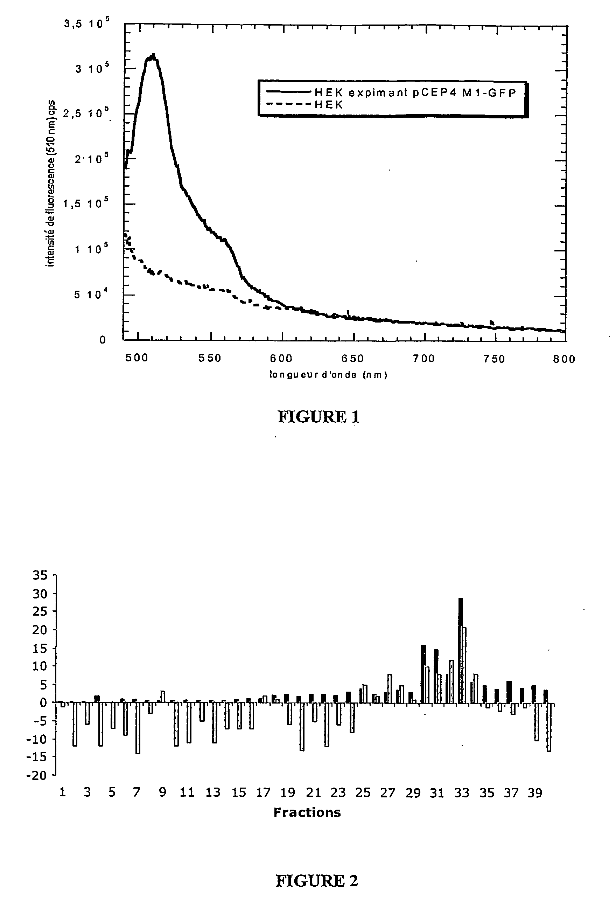

La Figure 1 représente un spectre d'émission de cellules HEK 293 transfectées par la construction pCEP4-SP-EGFP-M1 (voir exemple 1 ci-après). L'axe des ordonnées correspond à la longueur d'onde (en nm) et l'axe des abscisses à l'intensité de fluorescence (en cps). La courbe en trait plein correspond aux cellules HEK exprimant pCEP4-SP-EGFP-M1 et la courbe en trait pointillé aux cellules HEK non transfectées.
La Figure 2 représente la comparaison des absorbances à 220 nm et des pourcentages de transfert d'énergie par fluorescence (FRET) sur les différentes fractions de ligand fluorescent purifié (voir exemple 1). Les rectangles noirs représentent l'absorbance à 220 nm et les rectangles hachurés le pourcentage de FRET.

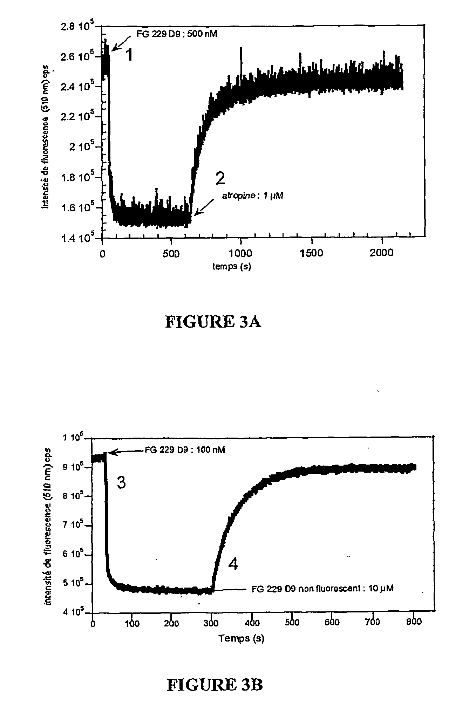

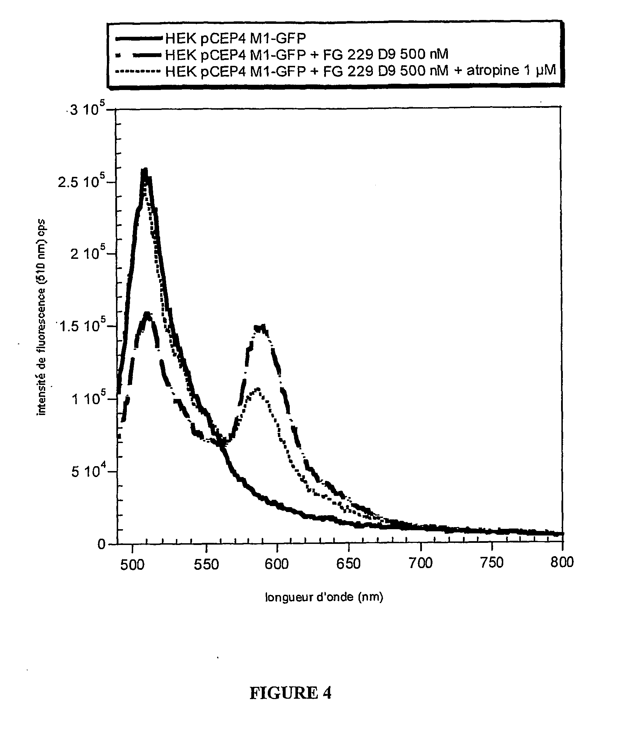

Les figures 3A et 3B représentent la mesure en temps réel du transfert d'énergie entre le ligand fluorescent purifié 500 nM (Figure 3A, 1), 100 nM (Figure 3B, 3) et le récepteur M1-EGFP et la réversion de cette liaison soit par un excès du même ligand non marqué (Figure 3B, 4), soit par un antagoniste connu, l'atropine (Figure 3A, 2). En abscisse est représenté le temps en seconde et en ordonnée l'intensité de fluorescence à 510 nm (coups par seconde), l'excitation ayant lieu à 470 nm.
La Figure 4 représente les spectres d'émission de cellules HEK 293 en suspension exprimant la construction pCEP4-M1-EGFP. En ordonnée, est représentée l'intensité de fluorescence exprimée en coups par seconde (cps) et en abscisse la longueur d'onde d'émission (nm). La courbe en trait plein représente le spectre d'émission des cellules exprimant pCEP4-M1-EGFP; la courbe en trait discontinu représente le spectre d'émission des cellules exprimant pCEP4-M1-EGFP en présence de ligand fluorescent ; la courbe en pointillé représente le spectre d'émission des cellules exprimant pCEP4-M1-EGFP en présence de ligand fluorescent et d'antagoniste connu, l'atropine.

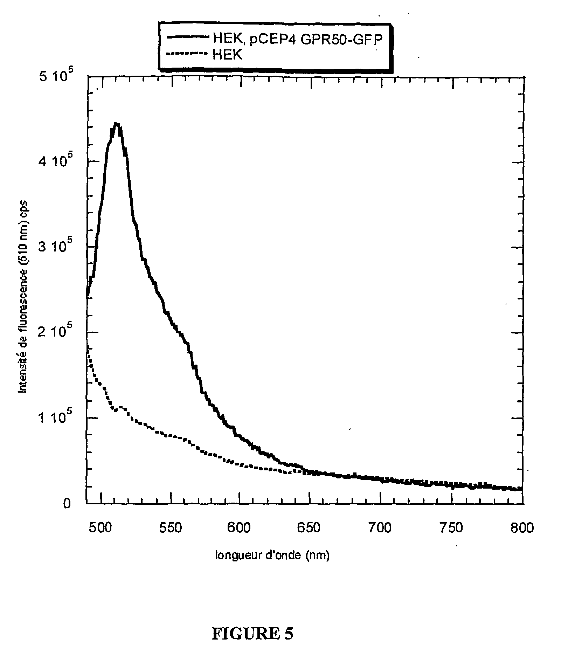

La Figure 5 représente un spectre d'émission de cellules HEK 293 transfectées par la construction pCEP4-SP-EGFP-GPR50 (voir exemple 2 ci-après). L'axe des ordonnées correspond à la longueur d'onde (en nm) et l'axe des abscisses à l'intensité de fluorescence (en cps). La courbe en trait plein correspond aux cellules HEK exprimant pCEP4-SP-EGFP-GPR50 et la courbe en trait pointillé aux cellules HEK non transfectées.

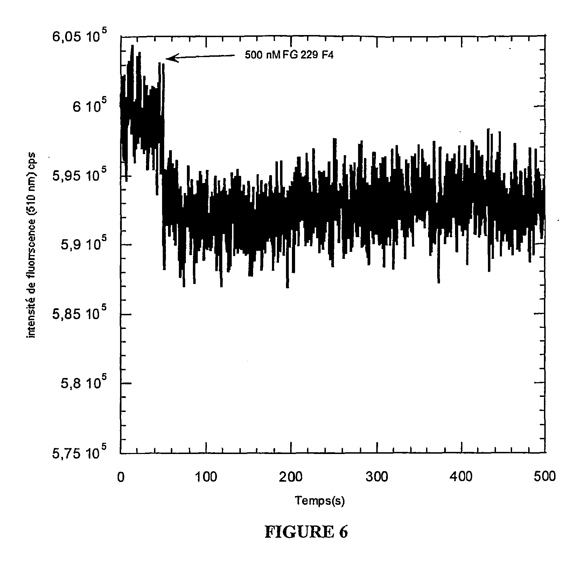

La Figure 6 représente la mesure en temps réel du transfert d'énergie entre le ligand fluorescent FG 229 F4 à 500 nM (1) et le récepteur GPR50-EGFP. En abscisse est représenté le temps en seconde et en ordonnée l'intensité de fluorescence à 510 nm (coups par seconde), l'excitation ayant lieu à 470 nm (un transfert d'énergie faible de 2 % peut être mesuré).

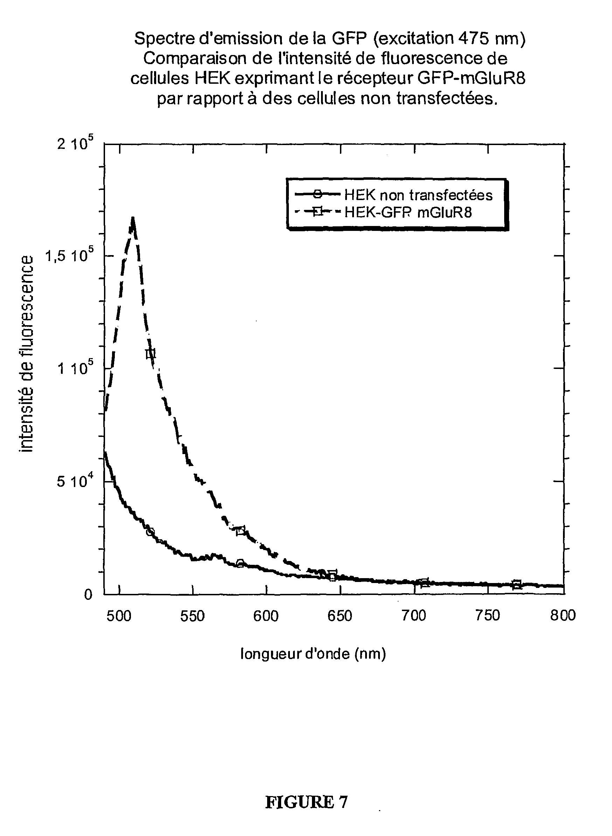

La Figure 7 représente un spectre d'émission de cellules HEK 293 transfectées par la construction pcDNA6-SP-GFP-mGluR8 (voir exemple 3 ci-après). L'axe des ordonnées correspond à la longueur d'onde (en nm) et l'axe des abscisses à l'intensité de fluorescence (en cps). La courbe en pointillés correspond aux cellules HEK exprimant pcDNA6-SP-GFP-mGluR8 et la courbe en trait plein aux cellules HEK non transfectées.

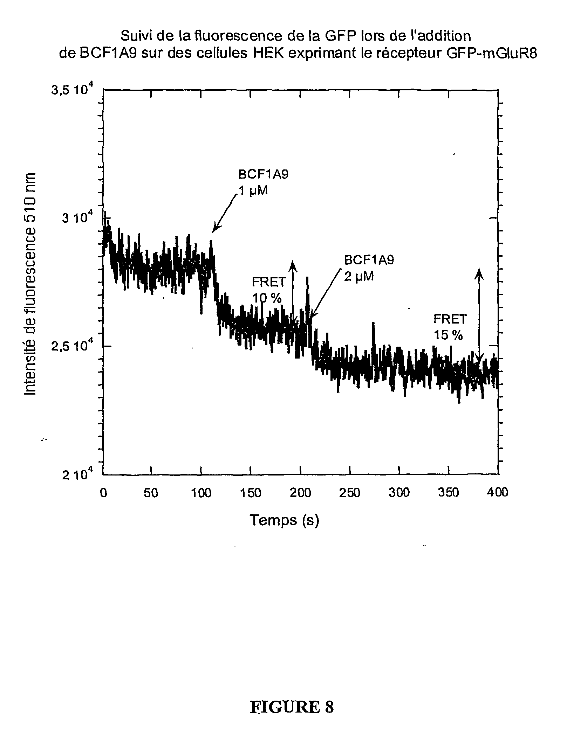

La Figure 8 représente la mesure en temps réel du transfert d'énergie entre le ligand fluorescent BCF1-A9 et le récepteur mGluR8-GFP. La position des flèches indique l'addition successive de BCF1A9. En abscisse est représenté le temps en seconde et en ordonnée l'intensité de fluorescence à 510 nm (coups par seconde), l'excitation ayant lieu à 475 nm (un transfert d'énergie de 10 et 15% est mesuré).

### EXEMPLES

### I - Développement de la Synthèse

### 1. Choix du support, conditions de lavage et monitoring des réactions

La résine de type REM **2** est choisie pour sa capacité à générer des amines tertiaires. Le clivage est effectué en 2 étapes : activation et formation d'ammonium par alkylation d'amine tertiaire suivie de β-élimination en présence de base (Morphy, J.R.; Rankovic, Z.; Rees, D.C. (1996) Tetrahedron Lett., 37, 3209-3212 ; Brown, A.R.; Rees, D.C.; Rankovic, Z.; Morphy, J.R. (1997) J. Am. Chem. Soc., 119, 3288-3295).

Les techniques classiques de synthèse sur support solide sont employées. En particulier, la résine est filtrée puis lavée après chaque étape en répétant 3 fois une séquence classique DMF/DCM/MeOH (5 à 10 mL par gramme de résine) puis DCM/MeOH/Et₂O/ Et₂O et enfin séchée sous vide de la pompe à palette pendant au moins 6 heures.

Le suivi réactionnel et le rendement sont déterminés après clivage et pesée du produit pur après chaque étape de la synthèse (en général en partant de 200 mg de résine soit environ 0,1 à 0,2 mmol de produit ou 30 à 50 mg). Lors des premières étapes, le produit est suffisamment pur et ne nécessite aucune procédure de purification. Au fur et à mesure de la synthèse, le taux d'impureté croît et le produit doit être purifié avant détermination du rendement. Le rendement est établi par rapport à la charge théorique de la résine donnée par le fournisseur.

La pseudo-pureté est estimée par RP-HPLC à 220nm ou 254nm. L'analyse de la pureté est réalisée sur un aliquot de 10 à 20 mg de résine soit 5 à 20 µmol (1 à 5 mg) de produit.

L'identification est effectuée par MS après dilution de l'échantillon analytique HPLC et par RMN ¹H et ¹³C lors des clivages réalisés sur plus grande échelle lors de la détermination des rendements.

La pureté et l'identification peuvent être obtenus par RMN ¹H lorsque la réaction est effectuée sur 50 mg de résine soit 25 à 50 µmol (5 à 15 mg) de produit. C'est généralement le cas dans la phase de développement où l'utilisation de la RMN est courante. Pour des raisons de débit d'analyse, l'utilisation de l'HPLC et MS est préférée dans les phases de sélection des "building blocks" et d'analyse de la chimiothèque.

### 2. Addition de Michael sur la résine REM

La résine REM **2** est préparée en accord avec la procédure de la littérature (schéma 1) à partir d'hydroxyméthylpolystyrène commercial **1**. On suppose que la réaction est quantitative après un double couplage de 10 équivalents de chlorure d'acryloyle [1 M] dans le DCM (10 mL par gramme de résine). L'analyse qualitative est effectuée par IR et apparition des fréquences du carbonyle (1720 cm⁻¹) et de l'insaturation αβ (1403 cm⁻¹).

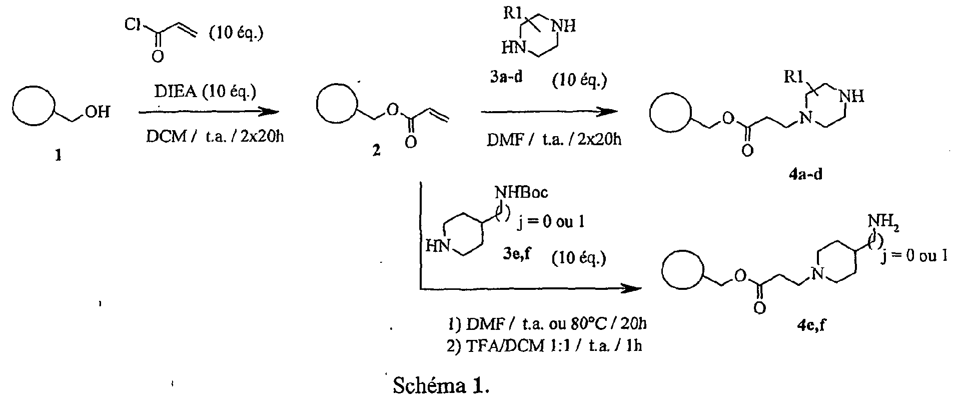

L'addition de Michael sur l'acrylate **2** est réalisée avec 10 équivalents d'amines secondaires **3** dans le DMF en procédant à un double couplage donnant ainsi accès aux β-aminoester **4.** Un facteur important est la concentration en amine qui doit être élevée, proche de 1 M (10 mL par gramme de résine). Lorsque l'amine n'est pas commerciale, et donc plus difficile d'accès, un simple couplage a été effectué sans diminution notable de conversion. A de fortes concentrations, certaines amines doivent être chauffées pour permettre la dissolution **(3a,b,e,f).** Ainsi, la réaction avec le 4-Boc-aminopipéridine **3e** est effectuée à 80°C pour des raisons de solubilisation de l'amine.

La synthèse requiert l'utilisation de diamines **3** (Tableau 1).

**Tableau 1**

| **Diamines R₁** | 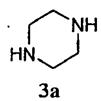 | 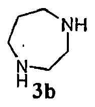 | 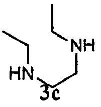 | 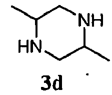 | 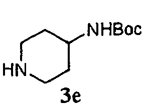 | 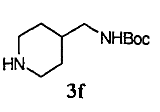 |
|---|---|---|---|---|---|---|
| 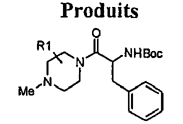 | **13a** | **13b** | **13c** | **13d** | **13e** | **13f** |
| **Rdt (%)** pdt isolé | 95 | 40 | 89 | 21 | 80 | 34 |
| **HPLC (%)** 220nm | 86 | > 95 | > 95 | > 95 | > 95 | > 95 |

Lorsque des diamines symétriques sont utilisées, une monoprotection n'est pas nécessaire du fait du principe d'isolation des sites par le support solide. Dans le cas d'utilisation d'amines dissymétriques, une monoprotection par le groupement Boc est réalisée de façon régio- et chimiosélective. On obtient ainsi la 4-Boc-aminopipéridine **3e** et la 4-Boc-aminométhylpipéridine **3f** (Contreras, J.M.; Rival, Y. M.; Chayer, S.; Bourguignon, J.J.; Wermuth, C.G. (1999) J. Med. Chem., 42, 730-741)(Schéma 2) en partant respectivement des 4-amino-1-benzylpipéridine et isonipécotamide commerciaux.

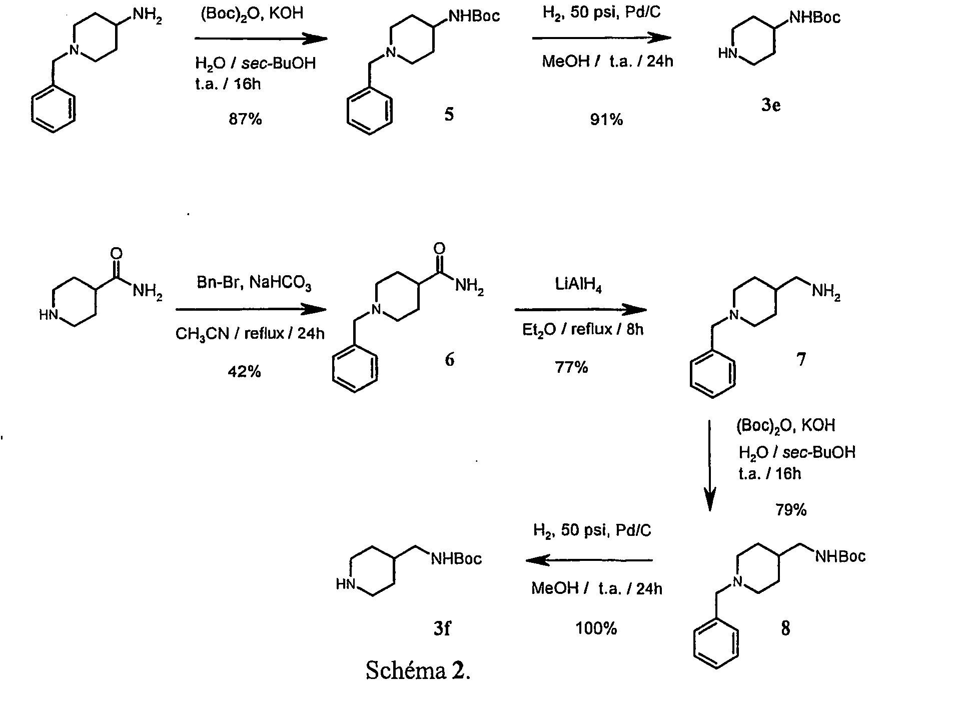

Ce type de diamines mono-protégées nécessite une étape de déprotection des groupements Boc en utilisant le TFA (Schéma **1).** Le protocole classique de déprotection au TFA implique un mélange TFA/DCM 50:50 pendant 1 heure à température ambiante en utilisant un volume de 30 mL par gramme de résine. Pratiquement la résine est pré-expansée dans du dichlorométhane (DCM)(1/3 du volume total) puis un mélange TFA/DCM 3:1 est ajouté (2/3 du volume total). Après réaction, la résine est lavée en utilisant 3 séquences de DMF/10% DIEA(DCM)/DCM/ MeOH afin de neutraliser le sel de TFA. L'amine en excès peut être récupérée après filtration et évaporation du DMF. La quantification de la charge de la résine **4** est réalisée de façon systématique, après couplage avec du Fmoc-Phe-OH, sur un aliquot de 25 mg de résine, et réalisation d'un test Fmoc (Bunin, B.A. (1998) in "The Combinatorial Index", Academie Press, San Diego) en observant la DO de l'adduit fluorénone-pipéridine à 301 nm. Les résines **4** sont ainsi prêtes pour la dérivatisation.

### 3. Couplage d'acides aminés Boc-protégés sur l'amine supportée

Du fait des conditions classiques bien établies pour le couplage d'acides aminés Boc-protégés **9** sur une amine supportée (synthèse peptidique C vers N classique), aucun développement particulier n'a été effectué (Schéma **3**). Pour des considérations de solubilisation d'un grand nombre d'acides aminés, le DMF est utilisé pur bien qu'il y ait des risques de racémisation de l'acide aminé. Classiquement 3 équivalents d'acide activé sont utilisés [0.1 M] dans le DMF (30 mL par gramme de résine). Du fait d'un encombrement stérique important, certains acides aminés sont utilisés en quantité plus importante (6 équivalents). Dans tous les cas, les acides sont préactivés sous forme d'esters d'HOBt, en agitant l'acide avec HOBt pendant 15 minutes puis en ajoutant le DIC et en poursuivant l'agitation encore 15 minutes. Alors seulement, l'acide préactivé est ajouté à l'amine sur phase solide (préalablement expansée dans le DMF ou le DCM). Les résines **4** sont ainsi transformées en résines **10.**

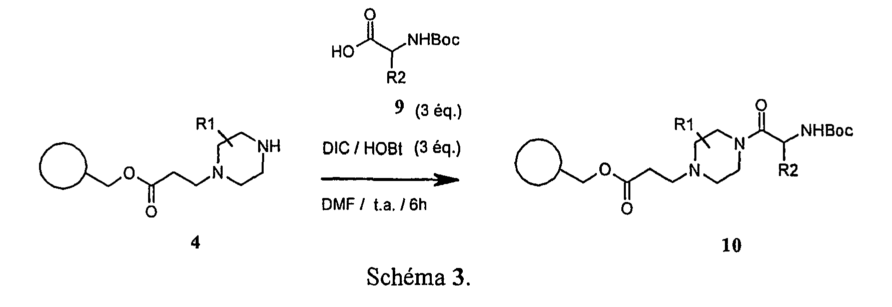

Afin d'augmenter la diversité des "building blocks", un certain nombre d'acides aminés Boc protégés **9** ont été synthétisés à partir d'acides aminés commerciaux, du fait du coût prohibitif ou de l'indisponibilité des dérivés Boc-protégés. Une méthode générale est décrite dans le Schéma **4.** (Smith, J., Liras, J.L.; Schneider, S.E.; Anslyn, E.V. (1996) J Org. Chem., 61, 8811-08818 ; Huang, W.; Kalivretenos, A.G. (1995) Tetrahedron Lett., 36, 9113-9116 ; Johnson, R.J.; Rajakumar, G.; Yu, K-L.; Mishra, R.K. (1986) J. Med. Chem., 29, 2104-2107 ; Millington, C.R.; Quarrell, R.; Lowe, G. (1998) Tetrahedron Lett., 39, 7201-7204 ; Khalil, E.M.; Subasinghe, N.L.; Johnson, R.L. (1996) Tetrahedron Lett., 37, 3441-3444; Bukholder, T.P.; Kudlacz, E.M.; Maynard, G.D.; Liu, X-G.; Le, T-B.; et al. (1997) Bioorg. Med. Chem. Lett., 7, 2531)

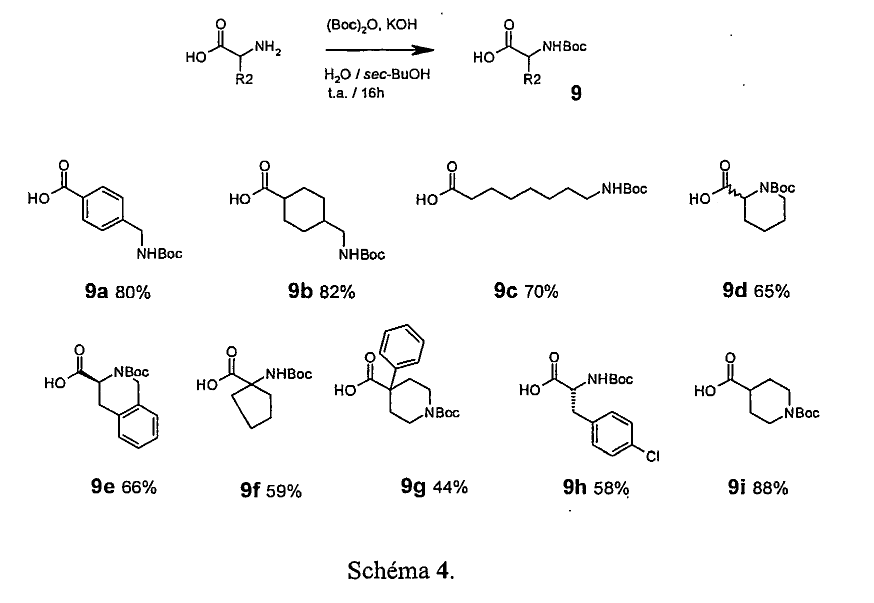

### 4. Couplage de la plateforme orthogonale : Fmoc-Lys(Boc)-OH et des acides carboxyliques basiques R₃

Le groupement protecteur Boc de la résine **10** (voir schéma 3) est traité au TFA selon les conditions décrites précédemment. Les conditions de couplage du Fmoc-Lys(Boc)-OH sont classiques et 3 équivalents d'acide activé sont utilisés dans le DMF à température ambiante pendant 6h (Schéma **5).** La préactivation de l'acide est réalisée de façon usuelle (DIC/HOBt). On accède ainsi à la plate-forme orthogonale **14.**

Après clivage dans les conditions classiques en utilisant DIEA et en effectuant un lavage aqueux, le produit **15a** est obtenu avec un rendement de 67%.

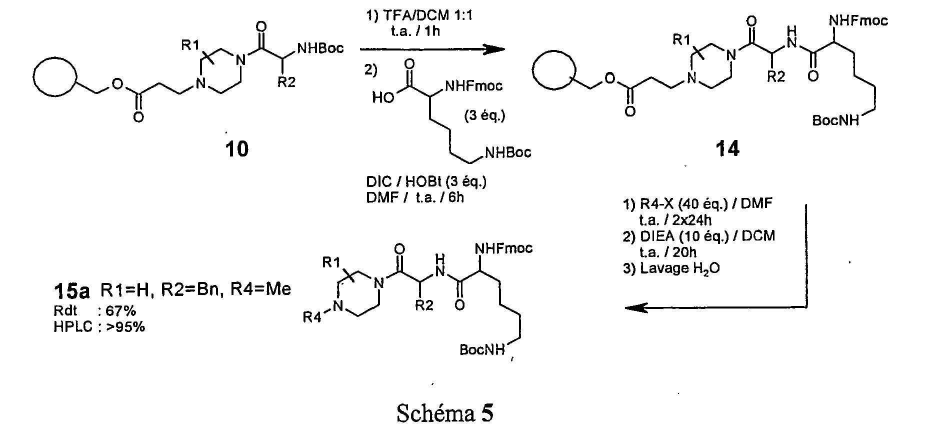

La déprotection sélective du groupement Fmoc sur la résine **14** est effectuée en utilisant un mélange 20% pipéridine dans le DMF (20 mL par gramme de résine) pendant 30 minutes et en répétant cette étape de déprotection. Cette réaction est quantitative et la présence de groupement Fmoc résiduel n'a jamais été observée.

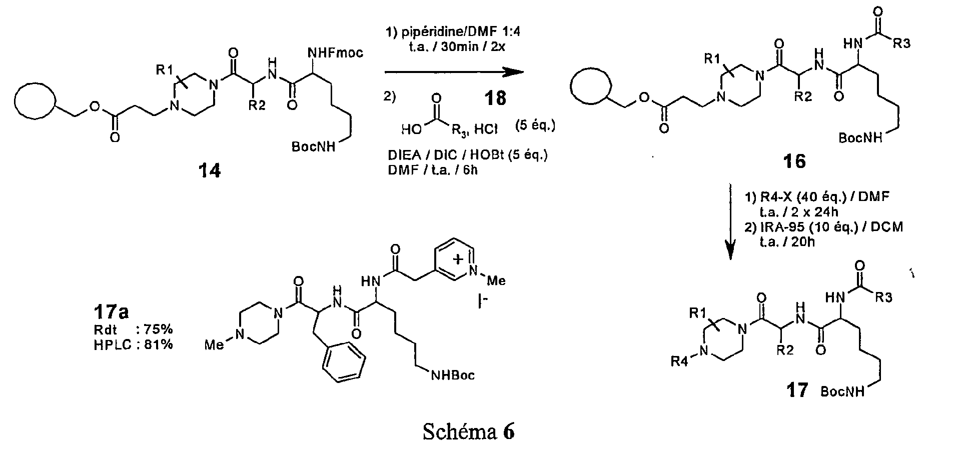

La préactivation de l'acide dont dérive R₃ est effectuée de façon usuelle (DIC/HOBt). La réaction conduit ainsi à la structure **17** après clivage, qui constitue la base de la version "blanche" de la chimiothèque (sans fluorophores)(Schéma **6**). En effet les autres étapes de préclivage n'impliquent plus dorénavant que l'incorporation du fluorophore.

Un certain nombre des acides carboxyliques dont dérivent R₃ sont disponibles sous forme de chlorhydrate du fait de leur basicité. La réaction nécessite alors l'ajout de DIEA. L'utilisation de DMF est cette fois cruciale car de nombreux acides n'ont pu être sélectionnés du fait d'une faible solubilité (même en présence de DIEA et dans le DMF chaud). Cette fois, une quantité légèrement supérieure d'acide préactivé est utilisée (5 équivalents).

Le produit **17a** est obtenu avec un rendement de 75%, après clivage en utilisant cette fois la résine IRA-95. La pureté du produit brut est estimée à 81 % par HPLC à 220 nm, les impuretés étant essentiellement constituées de sels résiduels de R₃N.HI.

Un certain nombre d'acides carboxyliques dont dérivent R₃ ont été synthétisés de façon à accroître la diversité en cette position (Schéma 7). Ainsi les acides aminés **18a,b,c** sont obtenus à partir de proline ou de sarcosine (Belokon, Y.N.; Tarasov, V.I.; Maleev, V.I.; Savel'eva, T.F.; Ryskov, M.G. (1998) Tetrahedron: Asymetrie, 9, 4249-4252), et l'arécaïdine **18d** est obtenue à partir d'arécoline commerciale (Martin, A.R.; Paradkar, V.M.; Peng, G.W.; Speth, R.C.; Yamamura, H.I.; Horn, A.S. (1980) J. Med. Chem., 23, 865-873).

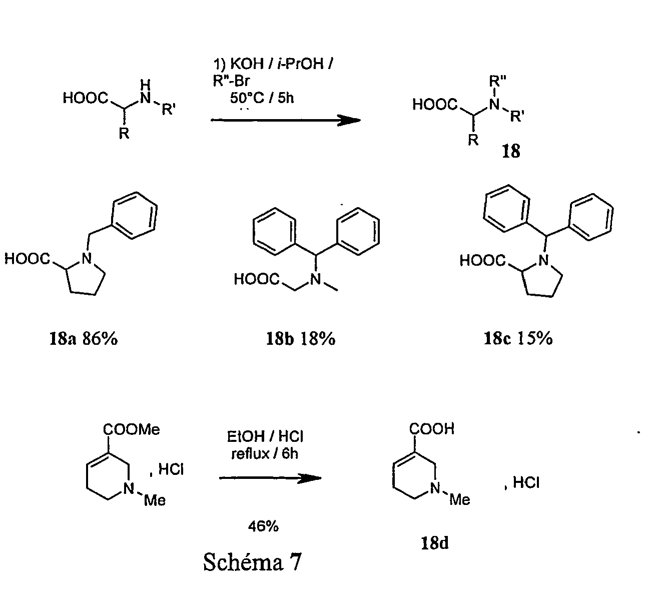

### 5. Incorporation du fluorophore

Le groupement fluorescent a été sélectionné en fonction de sa disponibilité commerciale et des caractéristiques physico-chimiques telles que sa compatibilité au FRET avec la GFP.

Le fluorophore utilisé dans le cadre de la présente invention est de préférence la Lissamine Rhodamine B³ sous forme de chlorure de sulfonyle (Schéma **8**). Celui-ci n'est cependant disponible en grande quantité que sous la forme d'un réactif dont la pureté n'est pas totale. En effet, en fin de réaction on isole un mélange de 2 isomères qui ne diffèrent par RMN 1H qu'au niveau de la Lissamine. Il semble qu'il y ait réaction avec le groupement chlorure de sulfonyle en position 4 **19f** ou 2 **19g** respectivement. Il n'est pas clair si le produit **19g** est présent au départ ou si il se forme par chloration *in situ.* Cela conduit à la formation d'un mélange de 2 produits **20a** et **20b** (80/20) qui pour l'instant semblent actifs l'un comme l'autre dans le test de FRET.

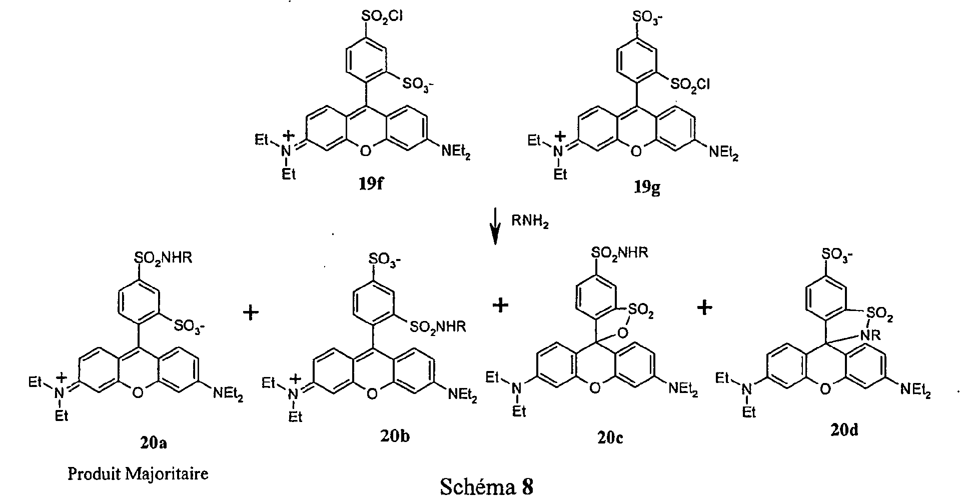

L'incorporation du fluorophore est réalisée au niveau de la chaîne latérale de la lysine sur l'amine en ε après déprotection du groupement Boc au TFA dans les conditions habituelles (Schéma **9**).

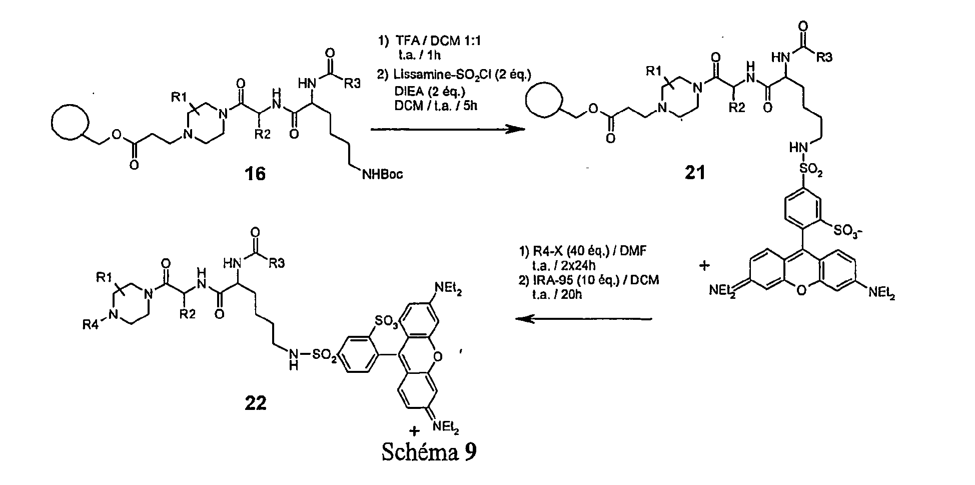

Après clivage classique, on accède aux composés fluorescents **22** à partir des composés supportés **21** (Schéma **9).**

Les composés **22a,b,c** sont ainsi obtenus après clivage de la résine **21** en utilisant l'iodure de méthyle ou le bromure de benzyle et la résine IRA95 comme base, suivi d'une purification par HPLC semi préparative en phase inverse avec des rendements globaux respectifs de 25, 20 et 27% (Schéma **10).** Ces produits sont accompagnés d'environ 5-6% de l'isomère de substitution du groupement chlorure de sulfonyle en position 2 de la lissamine.

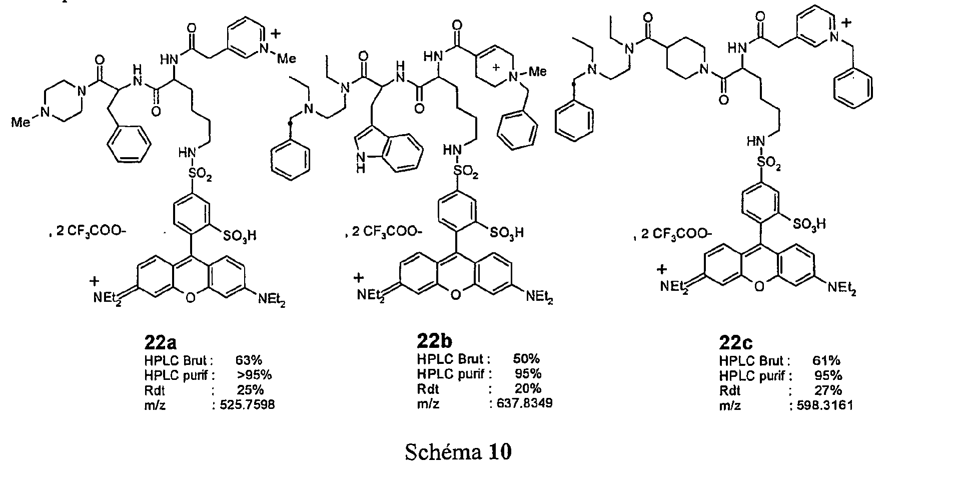

### 6. Incorporation du bras espaceur R₅

L'interaction du fluorophore et du groupement fluorescent GFP du récepteur est proportionnel à la distance entre ces deux groupes et optimale lorsque la distance est comprise entre 10 et 100Å (Vollmer, J-Y.; Alix, P.; Chollet, A.; Takeda, K.; Galzi, J-L. (1999) J. Biol. Chem., 274, 37915-37922 ; Förster (1951) dans "Fluoreszenz Organischer Verbindung" Vandenhoek and Rupprecht, Göttingen; Wu; Brand (1994) Anal Biochem, 2181, 1-13). Il apparaît donc important de disposer d'un lien de longueur différente entre le fluorophore et le pharmacophore, ceci afin d'augmenter les chances de se trouver dans la bonne gamme de distance favorisant l'interaction et le FRET.

Deux techniques d'élongation de chaîne à partir de l'amine primaire ε de la lysine ont été envisagées. Chacune des 2 techniques implique 2 étapes : soit l'incorporation d'un acide aminé Boc protégé de longueur variable type undécanoique ou caprylique (10 ou 7 unités méthylène) puis déprotection du Boc ou bien la formation d'urée en utilisant le *p*-nitrophénylchloroformate puis une diamine symétrique (Schéma 11)(Buchman, B.O.; Mohan, R. (1996) Tetrahedron Lett., 37, 4439-4442 ; Wang, F.; Haushe, J.R. (1997) Tetrahedron Lett., 38, 6529-6532).

La deuxième méthode a été utilisée du fait de la disponibilité commerciale des deux réactifs. Trois diamines ont été testées : la 1,4-diaminobutane **23a,** la 1,12-dodécanediamine **23b** et la 4,9-dioxa-1,14-dodecanediamine **23c.**

L'élongation est effectuée en utilisant un excès (10 équivalents) de p-nitrophénylchloroformate et DIEA [0.1 M] dans un mélange THF/DCM 1:1 pendant 2 heures à température ambiante conduisant aux carbamates **24**. Après un lavage en évitant d'utiliser du méthanol afin de ne pas déplacer le groupement *p*-nitrophénol, ce dernier est déplacé par la diamine **23.** Une forte concentration de diamine est utilisée (20 équivalents [0.2 M]) dans le DCM pendant 15h à température ambiante afin d'éviter toute réticulation sur 2 sites différents de la résine (Schéma **11**).

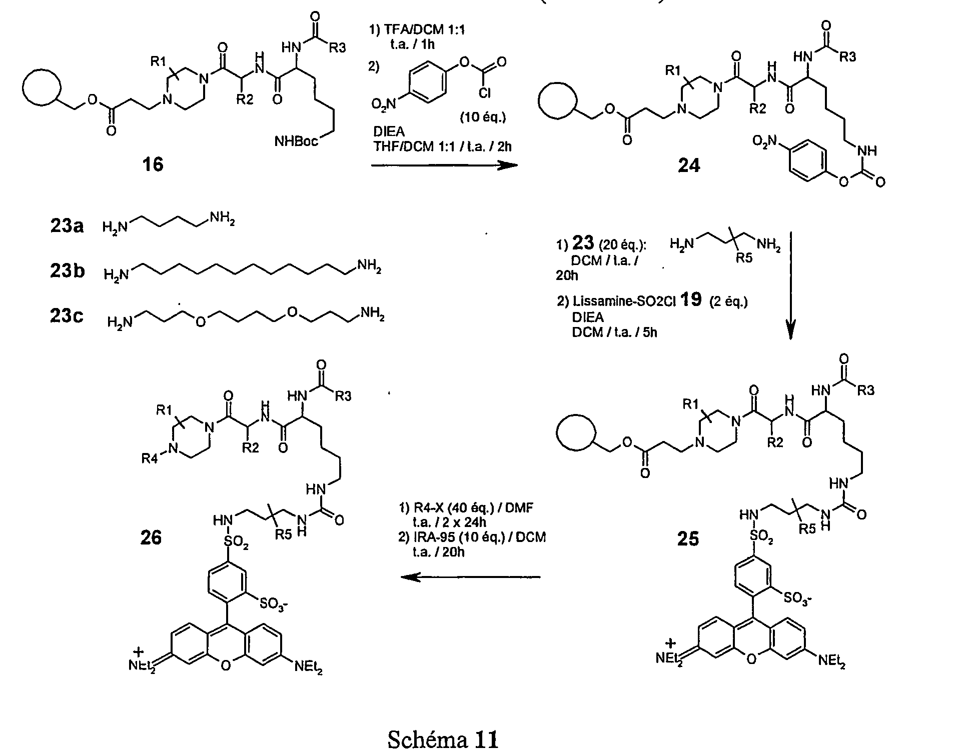

Le protocole décrit ci-dessus permet donc la synthèse de molécules fluorescentes **(22** et **26)** de type bis-cations à partir de résine REM 2. Cinq groupements (R₁ à R₅) permettent l'introduction de la diversité au niveau du pharmacophore (R₁ à R₄) et du bras espaceur (R₅) entre le pharmacophore et le fluorophore. Le fluorophore préféré est la Lissamine **19f** sous forme de chlorure de sulfonyle.

### II - Partie expérimentale

### 1 - Matériel

La Lissamine Rhodamine B sulfonyl chloride provient de chez Acros (ref 41323-0050). La résine polystyrène hydroxyméthyle (100-200mesh 1% DVB charge 0,6-1,6 mmol.g⁻¹) provient de chez Novabiochem (ref 0164-0110). Fmoc-Lys(Boc)-OH provient de chez Advanced Chem Tech (ref FK2390). La résine échangeuse d'ion IRA 95 (4,7 meq/g) provient de chez Sigma (ref A8085). Les solvants de lavage pour la synthèse des chimiothèques sont le dichlorométhane (DCM) technique (magasin de chimie), le diméthylformamide (DMF)(Riedel de Haën ref 15440) et le méthanol (MeOH)(Prolabo ref 208522.327).

La synthèse parallèle sur phase solide sur 0,01 à 0,1 mmol à température ambiante est effectuée dans des seringues en polypropylène munies d'un filtre en polyéthylène provenant de chez Supelco (ref 57023; 57024; 57026). Ces seringues sont bouchées en utilisant des septa en caoutchouc de chez Aldrich (ref Z16,725-8 ; Z10,073-0) ou Prolabo (ref 50627.152). La filtration est réalisée grâce au "Visiprep solid phase extraction vacuum manifold" (12 positions) de chez Supelco (ref 57030-u). L'agitation des seringues est réalisée par un shaker rotatif. Les réactions à hautes températures sont réalisées dans des flacons Supelco en verre transparents (15 mL) bouchés par un bouchon en polypropylène et munis d'un septum en PTFE/silicone (ref 27211). Les flacons sont positionnés sur un bloc chauffant en aluminium à 12 positions en utilisant une agitation magnétique à vitesse lente avec un barreau de forme cylindrique de chez Prolabo (ref 08553.082). La synthèse parallèle sur phase solide sur plus de 0,1 mmol à température ambiante est réalisée dans des "Peptide Vessels" de 20, 50 ou 80 mL équipés d'un fritté de porosité grossière ou dans des flacons Duran en verre transparent de 25 mL ou 50 mL de chez Prolabo (ref 09926.021 et 09926.043 respectivement). L'agitation est réalisée par un shaker rotatif. Les réactions à hautes températures sont réalisées dans des ballons mono-col classiques en verre avec une agitation mécanique (pour ne pas détériorer la résine) ou une agitation magnétique à vitesse minimale. La synthèse parallèle de chimiothèques combinatoires est effectuée en utilisant un appareil FlexChem de chez Robbins Scientific sur typiquement 0,01 mmol. Les réactions sont effectuées dans des "FlexChem polypropylene Reactor Blocks" (96-puits 2,4 mL/puits porosité moyenne 50-90 micron ref 1052-00-5). Ces "FlexChem polypropylene Reactor Blocks" sont intercalés entre deux capots métalliques (ref 1052-01-0 et 1052-02-0) et maintenus ensembles par un collier à pinces (ref 1052-06-0). L'étanchéité est assurée grâce à des septa en caoutchouc "rubber gasket" (pour une utilisation avec le DMF) (ref 1052-03-1) ou "Viton gasket" (pour une utilisation avec le DCM) (ref 1052-03-2). L'agitation des "FlexChem polypropylene Reactor Blocks" est réalisée à température ambiante ou haute température grâce à un four à agitation rotative (ref 1052-20-2). Les solutions Stock sont préparées dans des flacons Duran en verre transparents de 25 mL ou 50 mL de chez Prolabo (ref 09926.021 et 09926.043 respectivement), ainsi que des flacons VWR en verre transparents de 100 mL de chez Prolabo (ref 50637.908). La distribution des réactifs (50 à 1200 µL) depuis les solutions stock est réalisée grâce à des pipettes électroniques de chez Biohit (8 canaux ref 710800, 1 canal ref 710040) avec des pointes Biohit (50 à 1200 µL) (ref 780046). Le lavage et la distribution des solvants est réalisée en utilisant une pipette Eppendorf Multipette 4780 équipée de Combitips Plus de 50 mL de chez Prolabo (ref 01.301.611) et d'un râteau 8 canaux en polypropylène Statmatic de chez Polylabo (ref 66339). Il est préférable de verser les réactifs et les solvants dans des réservoirs à fond étroits de 60 mL de chez Prolabo (ref 08553.082). Après clivage, les molécules sont récupérées dans des plaques 96 puits en polypropylène à fond conique et contenant 2,2 mL par puits, produites par AB de chez Polylabo (ref 35379). L'évaporation est effectuée à l'aide d'un évaporateur Genevac DD4. Le stockage des produits finaux est effectué dans les plaques AB 96 puits mentionnées précédemment équipées d'un couvercle en silicone AB de chez Polylabo (ref 35858).

Les points de fusion sont déterminés sur un appareil Mettler FP62 et ne sont pas corrigés. Les spectres de masse en ionisation électronique (ESI-TOF) sont obtenus sur un spectromètre Mariner (Perceptive Biosystems). Les spectres RMN ¹H et ¹³C sont enregistrés sur un spectromètre Bruker 200 ou 300 MHz dans des solutions de CDCL₃, DMSO-d6 ou MeOD. L'HPLC analytique en phase inverse (RP-HPLC) est réalisé sur une colonne Dionex Vydac 218TP C18 (4,6 x 250 mm, 300Å, 10µ). Les éluants d'élution sont tels que gradient A: T_{0 min} 2% H₂O (0,1 % TFA), T_{30 min} 100% CH₃CN, T_{35 min} 100% CH₃CN à 0,5 mL/min. L'HPLC semi-preparative en phase inverse est réalisée sur une colonne Dionex Vydac 218TP C18 (22 × 250 mm, 300Å, 10-15µ). Les éluants d'élution sont tels que gradient B: T_{0 min} 2% H₂O (0,1% TFA), T_{5 min} 35% CH₃CN, T_{30 min} 100% CH₃CN T_{35 min} 100% CH₃CN à 10 mL/min ou gradient C tel que T_{0 min} 35% H₂O (0,1 % TFA), T_{25 min} 100% CH₃CN, T_{30 min} 100% CH₃CN à 10 mL/min.

### 2 - Préparation des motifs réactionnels

### 1-Benzyl-4-tertbutyloxycarbonylaminopipéridine 5

Dans un ballon de 250 mL équipé d'une agitation magnétique et d'une ampoule de coulée, le 4-amino-1-benzylpipéridine dichlorydrate monohydraté (16,6 g ; 63 mmol) est solubilisé dans une solution constituée de 30 mL de KOH aqueux. (7,8 g ; 139 mmol) et 60 mL de *sec*-butanol. Une solution de di-*tert*-butyldicarbonate (16,5 g : 75,6 mmol) dans 60 mL de *sec*-butanol est ajoutée lentement à température ambiante. Le mélange est ensuite agité à cette température pendant 16 heures. Après évaporation des solvants, le résidu est repris dans 200 mL d'eau et 200 mL de DCM. Le mélange est décanté puis la phase aqueuse est extraite avec 2 × 200 mL de DCM. Les phases organiques sont lavées avec une solution saturée de NaCl puis rassemblées, séchées (MgSO₄) et évaporées pour fournir 20 g d'un produit brut. Après purification par chromatographie sur silice (acétate d'éthyle/hexane 1:1 R_{f}= 0,45) ; 15,9 g (Rdt = 87%) de produit sont récupérés sous forme de cristaux blancs.
Masse (C₁₇H₂₆N₂O₂ = 290,41 ; (ESI-TOF) m/z monoisotope, calculé pour [M+H]⁺ : 291,2072 g/mol, observé : 291,2110)
Pf: 121°C
¹H RMN (EGFMH602/41, 200 MHz, CDCl₃) 7,31 (m, 5H, H_{aryl}) ; 4,43 (bs, 1H, NH) ; 3,49 (s, 3H, H_{benzyl} & H₄); 2,80 (d, 2H, *J=12Hz,* H_{2,6}); 2,09 (t, 2H, *J=12Hz,* H_{3,5}); 1,91 (d, 2H, *J=12Hz,* H_{2,6}); 1,50 (m, 2H, H_{3,5}); 1,45 (s, 9H, tBu)

### 4-tert-Butyloxycarbonylaminopipéridine 3e

Une solution de 1-Benzyl-4-tertbutyloxycarbonylaminopipéridine **5** (7,95 g ; 27,4 mmol) dans 120 mL de MeOH est versée dans un réacteur d'hydrogénation. Le catalyseur à base de Pd/c 10% est ajouté (1,46 g ; 1,38 mmol) et le mélange est soumis à une pression d'hydrogène de 3,5 bars (50 psi) pendant 24 heures dans un shaker de type Parr. Après Filtration sur célite et lavage avec du méthanol, le milieu est concentré pour fournir 4,98 g (Rdt = 91%) de produit sous forme de cristaux blancs.
Masse (C₁₀H₂₀N₂O₂ = 200,29 ; (ESI-TOF) m/z monoisotope, calculé pour [M+H]⁺: 201,1603 g/mol; observé : 201,1698)
Pf: 157°C
¹H RMN (300 MHz, CDCl₃) 4,52 (bs, 1H, NH) ; 3,51 (m, 1H, H₄) ; 3,06 (d, 2H, *J=12Hz,* H_{2,6}) ; 2,65 (t, 2H, *J=12Hz,* H_{3,5}) ; 1,95 (bs, 1H, NH) ; 1,92 (d, 2H, *J=12Hz,* H_{2,6}) ; 1,43 (s, 9H, tBu) ; 1,27 (m, 2H, H_{3,5})

### 1-Benzyl-4-carboxamidopipéridine 6

Dans un tricol de 1L muni d'un réfrigérant, d'une agitation magnétique et d'un thermomètre, l'isonipécotamide (12,5g ; 97,6 mmol) est ajoutée à 400 mL d'acétonitrile chaud. Après dissolution du réactif, le bicarbonate de sodium (14,7 g ; 175 mmol) et le bromure de benzyle (12,8 mL ; 107 mmol) sont ajoutés. Le mélange est agité au reflux pendant 24 h. Le mélange est ensuite filtré à chaud pour enlever les sels de sodium et ces sels sont lavés avec 50 mL d'acétonitrile. Après évaporation des solvants, le résidu est recristallisé dans 600 mL d'un mélange acétate d'éthyle/hexane 3:1 pour fournir 5,5 g de réactif initial et 8,69 g (Rdt = 41%) de produit sous forme de cristaux blancs.
Masse (C₁₃H₁₈N₂O = 218,31 ; (ESI-TOF) m/z monoisotope, calculé pour [M+H]⁺: 219,1497 g/mol ; observé : 219,1694)
Pf : 156°C (lit. 161°C)
¹H RMN (200 MHz, CDCl₃) 7,31 (m, 5H, H_{aryl}) ; 5,56 (bs, 2H, NH) ; 3,50 (s, 2H, H_{benzyl}) ; 2,93 (d, 2H, *J=12Hz,* H_{2,6}) ; 2,01-1,76 (m, 7H)

### 4-Aminométhyl-1-benzylpipéridine 7

Dans un bicol de 500 mL muni d'un réfrigérant, d'une agitation magnétique et placé sous balayage d'argon, le 1-benzyl-4-carboxamidopipéridine 6 (8,9 g; 40,8 mmol) est additionné par petite portions à une suspension de LiAlH₄ (2,33 g ; 61,5 mmol) dans 140 mL d'éther sec. Le mélange est ensuite porté au reflux sous argon pendant 24 heures. Le milieu est laissé refroidir à température ambiante puis versé avec précaution sur 300 mL d'un mélange eau/glace. Après filtration, 200 mL d'éther sont ajoutés puis le mélange est décanté. La phase aqueuse est extraite avec 2 × 200 mL d'éther. Après évaporation des solvants, 5,51 g de produit (Rdt = 66%) sont obtenus sous forme d'une huile incolore.
Masse (C₁₃H₂₀N₂ = 204,33 ; (ESI-TOF) m/z monoisotope, calculé pour [M+H]⁺: 205,1705 g/mol, observé : 205,1876
Pf : 88°C
¹H RMN (200 MHz, CDCl₃) 7,33 (m, 5H, Haryl) ; 3,52 (s, 2H, Hbenzyl); 2,93 (d, 2H, *J=12Hz,* H_{2,6}); 2,60 (d, 2H, *J=6Hz,* CH₂NH₂); 1,98 (t, 2H, *J=12Hz,* H_{3,5}) ; 1,71 (d, 2H, *J=9Hz,* H_{2,6}); 1,43 (bs, 2H, NH); 1,29 (m, 3H, H_{3,5} & H₄)

### 1-benzyl-4-tertbutyloxycarbonylaminométhylpipéridine 8

Dans un ballon de 100 mL équipé d'une agitation magnétique et d'une ampoule de coulée, le 4-aminométhyl-1-benzylpipéridine **7** (3,98 g ; 19,5 mmol) est dissous dans un mélange constitué de 10 mL de KOH aqueux (1,28 g ; 22,9 mmol) et 20 mL de *sec-*butanol. Une solution de di-*tert*-butyldicarbonate (5,45 g ; 25 mmol) dans 20 mL de *sec*-butanol est ajoutée lentement à température ambiante. Le mélange est ensuite agité à cette température pendant 16 h. Après évaporation des solvants, le résidu est repris dans 50 mL d'eau et 50 mL de DCM. Le mélange est décanté puis la phase aqueuse est extraite avec 2 × 50 mL de DCM. Les phases organiques sont lavées avec une solution saturée de NaCl puis rassemblées, séchées (MgSO₄) et évaporées pour fournir le produit brut. Après purification par chromatographie sur silice (acétate d'éthyle/hexane 1:1 R_{f} = 0,45), 4,79 g (Rdt = 81 %) de produit sont récupérés sous forme de cristaux blancs.
Masse (C₁₈H₂₈N₂O₂ = 304,45 ; (ESI-TOF) m/z monoisotope, calculé pour [M+H]⁺ : 305,2229 g/mol, observé : 305,2354)
Pf : 83°C
¹H RMN (300 MHz, CDCl₃) 7,33 (m, 5H, Haryl) ; 4,61 (bs, 1H, NH) ; 3,49 (s, 2H, Hbenzyl) ; 3,02 (t, 2H, *J=6Hz,* CH₂NH); 2,89 (d, 2H, *J=12Hz,* H_{2,6}); 1,94 (td, 2H, *J=12Hz, J=2.5Hz,* H_{3,5}); 1,65 (d, 2H, *J=12Hz,* H_{2,6}); 1,44 (s, 10H, tBu & H₄); 1,29 (td, 2H, *J=12Hz, J=3.5Hz,* H_{3,5})

### 4-tert-Butyloxycarbonylaminométhylpipéridine 3f

Une solution de 1-benzyl-4-tertbutyloxycarbonylaminométhylpipéridine **8** (4,79 g ; 15,7 mmol) dans 120 mL de MeOH est versée dans un réacteur d'hydrogénation. Le catalyseur à base de Pd/C 10% est ajouté (0,872 g ; 0,82 mmol) et le mélange est soumis à une pression d'hydrogène de 3,5 bars (50 psi) pendant 24 h dans un shaker de type Parr. Après filtration sur célite et lavage avec du méthanol, le milieu est concentré pour fournir 3,36 g (Rdt = 100%) de produit sous forme de cristaux blancs.
Masse (C₁₁H₂₂N₂O₂ = 214,32 ; (ESI-TOF) m/z monoisotope, calculé pour [M+H]⁺ : 215,1759 g/mol; observé : 215,1868)
Pf: 101°C
¹H RMN (200 MHz, CDCl₃) 4,68 (bs, 1H, NH) ; 3,07 (d, 2H, *J=12Hz,* H_{2,6}); 2,98 (t, 2H, *J=6Hz,* CH₂NH); 2,57 (td, 2H, *J=12Hz, J=2.5Hz,* H_{3,5}); 2,49 (bs, 1H, NH) ; 1,66 (d, 2H, *J=12Hz,* H_{2,6}); 1,43 (s, 10H, tBu & H₄); 1,14 (td, 2H, *J=12Hz, J=4Hz,* H_{3,5})

### Protocole général de formation des acides aminés Boc-protégés 9 :

Dans un ballon de 100 mL équipé d'une agitation magnétique et d'une ampoule de coulée, l'acide aminé (32 mmol) est solubilisé dans une solution constituée de 10 mL de KOH aqueux (1,88 g ; 33,6 mmol) et 20 mL de *sec*-butanol. Une solution de di*-tert-*butyldicarbonate (7,67 g ; 35,2 mmol) dans 20 mL de *sec*-butanol est ajoutée lentement à température ambiante. Le mélange est ensuite agité à cette température pendant 16 heures. Après ajout de 200 mL d'eau, le mélange est extrait avec 3 × 75 mL d'hexane. La phase aqueuse est refroidie avec 100 g de glace puis acidifiée à pH=2 avec KHSO₄ 1N. Après une extraction avec 3x100 mL d'acétate d'éthyle, les phases organiques sont séchées (MgSO₄) et concentrées pour fournir un produit généralement pur sous forme de cristaux blancs. Une recristallisation dans un mélange acétate d'éthyle / éther est effectuée dans certains cas.

### Acide 4-N-tert-butyloxycarbonylaminométhylbenzoïque 9a

(Smith, J., Liras, J.L.; Schneider, S.E.; Anslyn, E.V. (1996) J. Org. Chem., 61, 8811-08818)
Rdt = 80%
Masse (C₁₃H₁₇NO₄ = 251,29 ; (ESI-TOF) m/z monoisotope, calculé pour [M+Na]⁺ : 274,1055 g/mol ; observé : 274,1181
Pf: 164°C (Lit: 158°C)
¹H RMN (200 MHz, MeOD) 8,01 (d, 2H, *J=8Hz,* H_{2,6}) ; 7,40 (d, 2H, *J=8Hz,* H_{3,5}) ; 4,33 (s, 2H, CH₂NH) ; 1,49 (s, 9H)

### Acide 4-N-tert-butyloxycarbonylaminométhylcyclohexanoïque 9b

Rdt = 82%
Masse (C₁₃H₂₃NO₄ = 257,34; (ESI-TOF) m/z monoisotope calculé pour [M+Na]⁺ : 280,1525 g/mol ; observé : 280,1669)
Pf: 133°C
¹H RMN (200 MHz, CDCl₃) 4,65 (bs, 1H, NH) ; 2,99 (m, 2H, CH₂NH) ; 2,29 (t, 1H, *J=12Hz,* H₁); 2,07 (d, 2H, *J=12Hz,* H_{2,6}); 1,86 (d, 2H, *J=12Hz,* H_{2,6}) ; 1,47 (m, 13H, tBu, 2H_{3,5} & H₄) ; 1,02 (q, 2H, *J=12Hz,* H_{3,5})

### Acide 8-N-tert-butyloxycarbonylaminocaprylique 9c

(Huang, W.; Kalivretenos, A.G. (1995) Tetrahedron Lett., 36, 9113-9116)
Rdt = 70%
Masse (C₁₃H₂₅NO₄ = 259,36 ; (ESI-TOF) m/z monoisotope, calculé pour [M+H]⁺ : 260,1862 g/mol, observé : 260,2164)
Pf : 162°C
¹H RMN (200 MHz, CDCl₃) 4,53 (bs, 1H, NH); 3,10 (m, 2H, CH₂NH); 2,34 (t, 2H, *J=8Hz*); 1,63-1,33 (m, 10H); 1,45 (s, 9H, tBu)

### Acide N-tert-butyloxycarbonylpipécolinique 9d

(Johnson, R.J.; Rajakumar, G.; Yu, K-L.; Mishra, R.K. (1986) J. Med. Chem., 29, 2104-2107)
Rdt = 65%
Masse (C₁₁R₁₉NO₄ = 229,29 ; (ESI-TOF) m/z monoisotope, calculé pour [M+H]⁺: 230,1392 g/mol, observé : 230,1532)
Pf: 129°C
¹H RMN (300 MHz, CDCl₃) 9,42 (bs, 1H, OH) ; 4,94-4,78 (bm, 1H, H₂) ; 3,97 (m, 1H, H₆); 2,97 (m, 1H, H₆); 2,24 (d, 1H, *J=16Hz*); 1,69 (m, 3H) ; 1,47 (s, 9H tBu) ; 1,43-1,33 (m, 2H)

### Acide N-tert-butyloxycarbonyl-tetrahydroisoquinoléïne-3-carboxylique 9e

Rdt = 66%
Masse (C₁₅H₁₉NO₄ = 277,33 ; (ESI-TOF) m/z monoisotope, calculé pour [M+H (Cl³⁵)]⁺ : 278,1392 g/mol, observé : 278,1529)
Pf : 123°C
¹H RMN (200 MHz, CDCl₃) 7,17 (m, 4H, H_{aryl}) ; 6,50 (bs, 1H, OH) ; 5,10 (bs, 1H, NH) ; 4,70 (d, 2H, *J=16Hz,* CH₂NH) ; 4,48 (d, 1H, *J=16Hz,* CHα) ; 3,17 (m, 2H, CH₂β) ; 1,51-1,41 (m, 9H tBu)

### N-tert-butyloxycarbonyl-4-chlorophénylalanine 9h

(Millington, C.R.; Quarrell, R.; Lowe, G. (1998) Tetrahedron Lett., 39, 7201-7204)
Rdt = 58%
Masse (C₁₄H₁₈NO₄Cl= 299,77 ; (ESI-TOF) m/z monoisotope, calculé pour [M+H (Cl³⁵)]⁺: 300,1002 g/mol, observé : 300,1529)
Pf : 147°C
¹H NMR (300 MHz, CDCl₃) 9,81 (bs, 1H, OH) ; 7,28 (d, 2H, *J=8Hz,* H_{2,6}) ; 7,13 (d, 2H, *J=8Hz,* H_{2,6}) ; 5,00 (d, 1H, *J=7Hz,* NH) ; 4,62-4,37 (m, 1H, CHα) ; 3,20-3,02 (m, 2H, CH₂N) ; 1,43-1,35 (m, 9H tBu)

### Acide N-tert-butyloxycarbonylisonipécotinique 9i

Rdt = 88%
Masse (C₁₁H₁₉NO₄=229,29 ; (ESI-TOF) m/z monoisotope, calculé pour [M+H]⁺ : 230,1392 g/mol, observé : 230,1621)
Pf : 149 °C
¹H RMN (200 MHz, CDCl₃) 9,58 (bs, 1H, OH) ; 4,01 (d, 2H, *J=12Hz,* H_{2,6}) ; 2,83 (dt, 2H, *J=12Hz, J=3Hz,* H_{2,6}) ; 2,48 (m, 1H, H₁) ; 1,91 (dd, 2H, *J=12Hz, J=3Hz,* H_{3,5}) ; 1,68 (dt, 2H, *J=12Hz, J=3Hz,* H_{3,5}) ; 1,46 (s, 9H tBu)

### Acide 1-N-tert-butyloxycarbonylaminocyclopentane-1-carboxylique 9f

(Khalil, E.M.; Subasinghe, N.L.; Johnson, R.L. (1996) Tetrahedron Lett., 37, 3441-3444)

Un mélange de 1-aminocyclopentane-1-carboxylique (4,13 g; 32 mmol) et d'hydroxyde de tetraméthylammonium pentahydraté (5,79 g ; 32 mmol) est agité à température ambiante dans 200 mL d'acétonitrile. Le di-*tert*-butyldicarbonate (10,46 g ; 48 mmol) est ajouté et le mélange agité pendant 48 heures à température ambiante. Un excès de di-*tert*-butyldicarbonate (3,49 g ; 16 mmol) est ajouté et le mélange agité pendant encore 24 heures à température ambiante. Le solvant est évaporé sous pression réduite. Après ajout de 200 mL d'eau, le mélange est extrait avec 3 × 75 mL d'hexane. La phase aqueuse est refroidie avec 100 g de glace puis acidifiée à pH = 2 avec KHSO₄ 1N. Après une extraction avec 3 × 100 mL d'acétate d'éthyle, les phases organiques sont séchées (MgSO₄) et concentrées pour fournir 4,35 g de produit (Rdt = 59%) sous forme de cristaux blancs.
Masse (C₁₁H₁₉NO₄ = 229,29; (ESI-TOF) m/z monoisotope, observé pour [M+H]⁺ : 230,1532 g/mol)
Pf : 131°C (Lit.: 133°C)
¹H RMN (200 MHz, CDCl₃) 8,22 (bs, 1H, OH) ; 5,01 (bs, 1H, NH) ; 2,38-2,24 (m, 2H) ; 2,00-1,79 (m, 6H) ; 1,48 (s, 9H, tBu)

### Acide-4-phényl-N-tert-butyloxycarbonylpipéridine-4-carboxylique 9g

(Bukholder, T.P.; Kudlacz, E.M.; Maynard, G.D.; Liu, X-G.; Le, T-B.; et al. (1997) Bioorg. Med. Chem. Lett., 7, 2531)

Dans un ballon de 100 mL équipé d'une agitation magnétique et d'une ampoule de coulée, le sel de tosylate de l'acide 4-phényl-4-pipéridine carboxylique (12,06 g ; 32 mmol) est solubilisé dans une solution constituée de 20 mL de KOH aqueux (3,76 g ; 67,1 mmol) et 40 mL de *sec*-butanol. Une solution de di-*tert*-butyldicaxbonate (7,67 g ; 35,2 mmol) dans 20 mL de *sec*-butanol est ajoutée lentement à température ambiante. Le mélange est ensuite agité à cette température pendant 16 heures. Après ajout de 200 mL d'eau, le mélange est extrait avec 3 × 75 mL d'hexane. La phase aqueuse est refroidie avec 100 g de glace puis acidifiée à pH = 2 avec KHSO₄ 1N. Après une extraction avec 3 × 100 mL d'acétate d'éthyle, les phases organiques sont séchées (MgSO₄) et concentrées pour fournir 6,84 g d'un produit brut qui est recristallisé dans l'acétate d'éthyle pour fournir 4,32 g de produit (Rdt = 44%) sous forme de cristaux blancs.
Masse (C₁₇H₂₃NO₄ = 305,39; (ESI-TOF) m/z monoisotope, calculé pour [M+H]⁺: 306,1705 g/mol, observé : 306,1970)
Pf : 187°C
¹H RMN (300 MHz, CDCl₃) 7,43-7,28 (m, 5H, H_{aryl}) ; 3,93 (d, 2H, *J=12Hz,* H_{2,6}) ; 3,10 (t, 2H, *J=12Hz,* H_{2,6}) ; 2,50 (d, 2H, *J=12Hz,* H_{3,5}), ; 1,88 (t, 2H, *J=12Hz,* H_{3,5}) ; 1,45 (s, 9H tBu)

### N-benzylproline 18a (cf. schéma 6)

Dans un tricol de 100 mL muni d'une agitation magnétique, d'un thermomètre, d'une ampoule de coulée et d'un réfrigérant, une solution de proline (5,76 g ; 50 mmol) et de KOH (8,47 g ; 151 mmol) dans 35 mL d'isopropanol est préparée en chauffant le mélange à 50°C. Le milieu devient homogène après 30 minutes puis le bromure de benzyle (7,2 mL ; 60 mmol) est additionné en 30 minutes à 50°C. Le mélange est chauffé pendant 5 heures à cette température. Le mélange est ensuite laissé refroidir à température ambiante puis le pH est ajusté à 5-6 avec de l'HCl concentré. Après ajout de 15 mL de CHCl₃, le mélange est agité pendant 16h à température ambiante. Le mélange est ensuite filtré et le précipité est lavé avec 2 × 20mL de CHCl₃. Le filtrat est concentré sous pression réduite pour fournir un solide qui est lavé à l'acétone. Après séchage sous vide de la pompe à palette on obtient 8,80 g de produit (Rdt = 86%) sous forme de cristaux blancs.
Masse (C₁₂H₁₅NO₂ = 205,27 ; (ESI-TOF) m/z monoisotope, calculé pour [M+H]⁺: 206,1181 g/mol, observé : 206,1423)
Pf : 170°C (lit. 174°C)
¹H RMN (300 MHz, DMSO-d6) 8,71 (bs, 1H, OH); 7,41-7,34 (m, 5H, H_{aryl}); 4,18 (d, 1H, *J=12Hz*); 3,90 (d, 1H, *J=12Hz*); 3,52 (m, 1H); 3,16 (m, 1H); 2,76 (q, 1H *J=9Hz*); 2,15 (q, 1H *J=9Hz*); 1,90-1,71 (m, 3H)

### N-diphénylméthylproline 18b

Dans un tricol de 100 mL munit d'une agitation magnétique, d'un thermomètre, d'une ampoule de coulée et d'un réfrigérant, une solution de proline (5,76 g ; 50 mmol) et de KOH (8,47 g ; 151 mmol) dans 35 mL d'isopropanol est préparée en chauffant le mélange à 50°C. Le milieu devient homogène après 30 minutes puis le chlorodiphénylméthane (10,7 mL ; 60 mmol) est additionné en 30 minutes à 50°C. Le mélange est chauffé pendant 5h à cette température. Le mélange est ensuite laissé refroidir à température ambiante. Après ajout de 100 mL d'eau, le pH est ajusté à 10 et 9,80 g de chlorodiphénylméthane (80%) sont récupérés après extraction avec du CHCl₃. Le pH est ajusté à 5-6 avec de l'HCl concentré et le mélange est extrait à nouveau avec du chloroforme pour fournir 2,15 g de produit (Rdt = 15%) sous forme de cristaux de couleur marron.
Masse (C₁₈H₁₉NO₂ = 281,36 ; (ESI-TOF) m/z monoisotope, calculé pour [M+H]⁺: 282,14939 g/mol, observé : 282,1784)
Pf : 167°C
¹H RMN (300 MHz, CDCl₃) 8,85 (bs, 1H, OH) ; 7,47-7 ;30 (m, 10H, H_{aryl}) ; 4,82 (s, 1H, CHPh₂) ; 4,18 (d, 1H, *J=12Hz*) ; 3,68 (dd, 1H, *J=9Hz, J=3,5Hz*) ; 3,32 (m, 1H) ; 2,69 (q, 1H *J=9Hz*) ; 2,21 (m, 2H) ; 1,89 (m, 2H)

### N-diphénylméthylsarcosine 18c

Dans un tricol de 250 mL munit d'une agitation magnétique, d'un thermomètre, d'une ampoule de coulée et d'un réfrigérant, un mélange de sarcosine (4,45 g ; 50 mmol) et de KOH (8,47 g; 151 mmol) dans 70 mL d'isopropanol est préparée en chauffant le mélange à 50°C. Le milieu reste trouble après 30 minutes puis le chlorodiphénylméthane (10,7 mL ; 60 mmol) est additionné en 30 minutes à 50°C. Le mélange est chauffé pendant 5h à cette température. Le mélange est ensuite laissé refroidir à température ambiante. Après ajout de 100 mL d'eau, le pH est ajusté à 10 et le chlorodiphénylméthane qui n'a pas réagi est récupéré après extraction avec du CHCl₃. Le pH est ajusté à 5-6 avec de l'HCl concentré et le mélange est extrait à nouveau avec du chloroforme pour fournir 2,23 g de produit (Rdt = 18%) sous forme de gomme incolore.
Masse (C₁₆H₁₇NO₂ = 255,33 ; (ESI-TOF) m/z monoisotope, calculé pour [M+H]^{+:} 256,1337 g/mol, observé : 256,1545)
¹H RMN (200 MHz, CDCl₃) 7,43-7,30 (m, 11H, H_{aryl} & OH); 4,89 (s, 1H, CHPh₂) ; 3,34 (s, 2H, Hα) ; 2,46 (s, 3H, CH₃N)

### Chlorhydrate d'arécaïdine 18d

(Martin, A.R.; Paradkar, V.M.; Peng, G.W.; Speth, R.C.; Yamamura, H.I.; Horn, A.S. (1980) J. Med. Chem., 23, 865-873)

Dans un ballon de 250 mL muni d'une agitation magnétique et d'un réfrigérant, le chlorhydrate d'arécoline (4 g ; 20,9 mmol) est ajouté à une solution de 50 mL d'éthanol à 95% et 60 mL d'HCl concentré. Le mélange est chauffé à 100°C pendant 12h puis au reflux pendant 6h. Les solvants sont évaporés sous pression réduite laissant un résidu solide. Le résidu est recristallisé dans un mélange éthanol à 95% / éther pour fournir 1,69 g de produit (Rdt = 46%) sous forme de cristaux blancs.
Masse (C₇H₁₁NO₂+HCl = 177,63 ; (ESI-TOF) m/z monoisotope, calculé pour [M+H]⁺ : 142,0867 g/mol, observé : 142,0817)
Pf : 247°C (lit. 261°C)
¹H RMN (300 MHz, MeOD) 7,15 (s, 11H) ; 4,14 (bm, 1H) ; 3,81 (bm, 1H) ; 3,57 (bm, 1H) ; 3,25 (bm, 1H) ; 3,02 (s, 3H) ; 2,70 (bs, 2H)

### 3 - Synthèse multi-étapes

### Résine REM 2

La résine hydroxyméthylpolystyrène **1** (10,3 g; 12,8 mmol)[Novabiochem 1.24 mmol/g, 100-200 Mesh] est placée dans un ballon de 250 mL. La résine est expansée avec 100 mL de dichlorométhane puis la diisopropyléthylamine (16,5 g ; 128 mmol) est ajoutée. La suspension est agitée avec une agitation magnétique réglée à la plus basse vitesse puis le chlorure d'acryloyle (11,6 g ; 128 mmol) est ajouté en 4 portions. L'agitation est poursuivie à température ambiante pendant 24h puis la résine est filtrée et lavée avec du DMF et du dichlorométhane. La réaction est répétée une fois avant la filtration. Le lavage final est réalisé avec 2 séquences de DMF, dichlorométhane et MeOH terminé par Et₂O. La résine est séchée sous vide de la pompe à palette pendant 24h pour fournir 11,2 g fr résine (charge théorique 1,16 mmol.g⁻¹). La formation de l'acrylate est confirmée par analyse IR (vibration du carbonyle à 1727 cm⁻¹ et de l'alcène à 1403 cm⁻¹).

### Protocole général pour l'addition de Michael sur la résine REM

La résine REM **2** (3,5 g; 4,74 mmol; 1,35 mmol.g⁻¹ charge théorique) est expansée dans 10 mL de DMF, puis une solution de diamine 3 (47,4 mmol) dans le DMF (40 mL) est ajoutée à la suspension. Après agitation sur le shaker rotatif pendant 24h à température ambiante, la résine est filtrée et lavée avec du DMF et du dichlorométhane. La réaction est répétée une fois. Le lavage final est effectué avec 2 séquences de DMF, dichlorométhane et MeOH terminé par Et₂O. La résine est séchée sous vide de la pompe à palette pendant 16h. La détermination de la charge est obtenue sur un aliquot de 25 mg de résine par la méthode du test Fmoc (après couplage avec 5 équivalents de Fmoc-Phe-OH/DIC/HOBt pendant 6h à température ambiante, suivi d'une deprotection en utilisant la pipéridine ; l'absorbance de l'adduit fulvàne-pipéridine généré en solution est mesurée à 301 nm, ε = 7800 mol.L⁻¹.m⁻¹). Pour des raisons de solubilité, le protocole est modifié pour la 4-*tert*-butyloxycarbonylaminopipéridine **3e.** La réaction est effectuée dans un ballon équipé d'une agitation magnétique et une solution chaude (80°C) d'amine Boc-monoprotégée dans le DMF est ajoutée à la résine. L'addition de Michael est poursuivie pendant 16h à 80°C et n'est pas répétée. Les composés Boc-monoprotégés de façon régiosélective sont employés dans le cas des diamines asymétriques. Une étape de Boc-deprotection est donc nécessaire après l'addition de Michael. La résine (2 g) est expansée dans le DCM (20 mL) puis une solution de TFA/DCM 3:1 (40 mL) est ajoutée. Après 1h de réaction à température ambiante, la résine est filtrée puis lavée avec 3 séquences de DMF, DCM, 10% DIEA dans le DCM, MeOH et terminée par Et₂O. La résine est ensuite séchée sous vide de la pompe à palette pendant 16h.

### Protocole général pour le couplage d'acide aminé sur une résine fonctionnalisée par un groupement amino

Une solution d'acide préactivé est préparée en mélangeant l'acide carboxylique (13,5 mmol) et HOBt (13,5 mmol) dans du DMF (30 ml) pendant 15 min à température ambiante. Une solution de DIC (13,5 mmol) dans du DMF (10 mL) est ensuite ajoutée et le mélange est encore agité pendant 15 min à température ambiante. Pendant ce temps, la résine (3,75 g ; 4,5 mmol ; 1,20 mmol.g⁻¹ charge théorique) est expansée dans du DMF (20 mL) pendant 30 min sans agitation. La solution d'acide préactivé (13,5 mmol) est ajoutée à la résine et l'ensemble est agité sur un shaker rotatif pendant 6h à température ambiante. La résine est filtrée et lavée en utilisant 3 séquences de DMF, dichlorométhane et MeOH puis Et₂O. La résine est ensuite séchée sous vide de la pompe à palette pendant 16h.

### Protocole général pour le clivage d'esters par transestérification

Les resines du type N,N-dialkylaminoethanoate de polystyryle 10 (300 mg ; 0,277 mmol ; 0,93 mmol.g⁻¹ charge théorique) sont expansées dans 4 mL de THF dans un ballon de 50 mL équipé d'une agitation magnétique et d'un condenseur. Une solution de méthylate de sodium (15 mg ; 0,277 mmol) dans 16 mL de MeOH sec est ajoutée à la suspension et le mélange est agité à 65°C pendant 20 h. Après refroidissement, la résine est filtrée puis lavée avec du MeOH et DCM (3 × 5 mL). Les filtrats sont collectés et évaporés. Le résidu est repris dans DCM (30 mL) et une solution de NaHCO₃ 5% (30 mL). La phase organique est séparée et la phase aqueuse est réextraite avec du DCM (2 × 30 mL). Les phases organiques sont rassemblées, séchées (MgSO₄) et évaporées à sec pour fournir les esters méthyliques 11 correspondants.

### Ester éthylique de l'acide 3-[4-(2-tert-Butoxycarbonylamino-3-phénylpropionyl)-pipérazin-1-yl]-propionique 11

Rdt : 95 mg de gomme jaune (82%).
HPLC : 82% dionex, gradient A, Rt 24,3 min.
Masse (C₂₂H₃₃N₃O₅ = 419,54; (ESI-TOF) m/z monoisotope, calculé pour [M+H]⁺ : 420,2498 g/mol, observé : 420,2776)
¹H NMR (300MHz, CDCl₃) 7,29-7,19 (m, 5H) ; 5,45 (brd d, *J*=8,3 Hz, 1H, NH) ; 4,81 (q, *J*=6,0 Hz, 1H, HαPhe) ; 3,62 (s, 3H, MeO) ; 3,52 (m, 2H, H_{2,6}pipérazine) ; 3,28 (m, 1H, HβPhe) ; 2,98 (m, 1H, HβPhe) ; 2,96 (t, *J*=8,3 Hz, 2H, H_{2,6}pipérazine) ; 2,58 (t, *J*=7,2 Hz, 2H, CH₂CH₂) ; 2,42 (t, *J*=6,8 Hz, 2H, CH₂CH₂) ; 2,36 (m, 1H, Hpipérazine) ; 2,23 (m, 2H, H_{3,5}pipérazine) ; 1,77 (m, 1H, Hpipérazine) ; 1,42 (s, 9H, *t*Bu).
¹³C RMN (50MHz, CDCl₃) 173,04 (Cester); 170,31 (Camide); 155,43 (Ccarbam) ; 136,83 (C₁ph) ; 129,97 (C_{3,5}ph) ; 128,92 (C_{2,6}ph) ; 127,35 (C₄ph) ; 80,10 (C*t*Bu) ; 53,58 (C_{MeO}) ; 52,74 (C_{3,5pipérazine}) ; 52,54 (C_{3,5pipérazine}) ; 52,06 (N-CH₂CH₂) ; 51,24 (CαPhe) ; 45,83 (C_{2,6pipérazine}) ; 42,21 (C_{2,6pipérazine}) ; 40,79 (C_{βPhe}) ; 32,30 (CO-CH₂CH₂) ; 28,73 (C*t*Bu).

### Protocole général pour le clivage de résine REM par quaternarisation et élimination D'hofmann

La résine de type amine tertiaire (300 mg, 279 µmol, 0.93 mmol.g⁻¹ charge théorique) est expansée dans le DMF (8 mL) et l'iodure de méthyle ou le bromure de benzyle (11,2 mmol) est ajouté. L'ensemble est agité pendant 20h sur un shaker rotatif à température ambiante. La résine est ensuite filtrée et lavée avec du DMF, DCM et DMF. La réaction est répétée une fois puis après filtration, le lavage final est réalisé en utilisant 3 séquences de DMF, dichlorométhane et MeOH puis Et₂O. La résine est ensuite séchée sous vide de la pompe à palette pendant 6h. La résine est suspendue dans le DCM (8 mL) et la base (DIEA (2,79 mmol) ou résine IRA-95 (4,7 méqu/g ; 0,59 g ; 2,79 mmol)) est ajoutée. L'ensemble est agité pendant 20h sur un shaker rotatif à température ambiante. La résine est filtrée puis lavée avec du MeOH et DCM (3 × 5 mL). Les filtrats sont collectés et évaporés. Le résidu est repris dans DCM (30 mL) et une solution de NaHCO₃ 5% (30 mL). La phase organique est séparée et la phase aqueuse est reextraite avec du DCM (2 × 30 mL). Les phases organiques sont rassemblées, séchées (MgSO₄) et évaporées à sec pour fournir les amines tertiaires correspondantes.

### Ester tert-butylique de l'acide 1-Benzyl-2-(4-méthylpipérazin-1-yl)-2-oxo-éthyl]-carbamique 13a

Rdt : 95 mg de gomme jaune (95%).
HPLC : 86% dionex, gradient A, Rt 23,0 min.
Masse (C₁₉H₂₉N₃O₃= 347,47 ; (ESI-TOF) m/z monoisotope, calculé pour [M+H]⁺ : 348,2287 g/mol ; observé : 348,1748)
¹H RMN (300MHz, CDCl₃) δ 7,24-7,19 (m, 5H, Ph) ; 5,47 (brd d, *J*=8,7 Hz, 1H, NH) ; 4,81 (q, *J*=6,4 Hz, 1H, HαPhe) ; 3,54 (m, 2H, H_{2,6}pipérazine) ; 3,31 (m, 1H, HβPhe) ; 2,95-3,05 (m, 3H, HβPhe & H_{2,6}pipérazine) ; 2,27 (m, 1H, H_{pipérazine}) ; 2,15-2,23 (m, 2H, H_{3,5pipérazine}) ; 2,17 (s, 3H, MeN) ; 1,74 (m, 1H, H_{pipérazine}) ; 1,42 (s, 9H, *t*Bu).
¹³C RMN (50MHz, CDCl₃) 170,35 (C_{amide}); 155,45 (C_{carbam}) ; 136,87 (C₁ₚₕ) ; 129,96 (C_{3,5}ph) ; 128,91 (C_{2,6ph}) ; 127,34 (C₄ph); 80,03 (C_{*t*Bu}) ; 54,82 (C_{3,5}pipérazine) ; 54,73 (C_{3,5}pipérazine); 51,27 (CαPhe) ; 46,18 (C_{MeN}) ; 45,72 (C_{2,6}pipérazine) ; 42,16 (C_{2,6}pipérazine) ; 40,81 (C_{βPhe}) ; 28,73 (C*t*Bu).

### Ester tert-butyliqzce de l'acide [1-Benzyl-2-(4-méthyl-[1,4]-diazepan-1-yl)2-oxoéthyl]-carbamique 13b

Rdt : 30 mg d'huile jaune (40%).
HPLC : > 95% dionex, gradient A, Rt 23,5 min.
Masse (C₂₀H₃₁N₃O₃ = 361,50 ; (ESI-TOF) m/z monoisotope, calculé pour [M+H]⁺ : 362,2443 g/mol, observé : 362,1955)
¹H NMR (300MHz, CDCl₃) δ 7,37-7,19 (m, 5H, Ph) ; 5,34 (brd, 1H, NH) ; 4,79 (m, 1H, HαPhe) ; 3,64 (m, 2H, Hₕₒₘₒₚᵢₚ) ; 3,37 (m, 1H, HβPhe) ; 2,96-3,15 (m, 3H, HβPhe & Hₕₒₘₒₚᵢₚ); 2,18-2,72 (m, 4H, Hₕₒₘₒₚᵢₚ) ; 2,36 (s, 3H, MeN) ; 1,91 (m, 2H, Hₕₒₘₒₚᵢₚ) ; 1,87 (s, 9H, *t*Bu).

### Ester tert-butylique de l'acide [1-(Éthyl-[2-éthylméthylamino)-éthyl]-carbamoyl)-2-phényléthyl)-carbamique 13c

Rdt : 50 mg d'huile jaune (89%).
HPLC : > 95% dionex, gradient A, Rt 25,0 min.
Masse (C₂₁H₃₅N₃O₃= 377,55 ; (ESI-TOF) m/z monoisotope, calculé pour [M+H]⁺: 378,2756 g/mol, observé : 378,2136)
¹H RMN (300MHz, CDCl₃) 7,23-7,19 (m, 5H, Ph) ; 5,30 (brd, 1H, NH) ; 4,72 (m, 1H, HαPhe) ; 3,83 (m, 1H) ; 3,60 (m, 1H, HβPhe) ; 3,34 (m, 1H, HβPhe) ; 2,96-3,13 (m, 3H) ; 2,64 (m, 3H) ; 2,40 (s, 3H, MeN) ; 1,40 (s, 9H, *t*Bu) ; 1,16 (t, *J*=7,2 Hz, 2H) ; 1,01 (t, *J*=7,2 Hz, 4H).

### Ester tert-buylique de l'acide [1-Benzyl-2-oxo-2-(2,4,5-triméthylpipérazin-1-yl)éthyl]-carbamique 13d

Rdt : 12 mg d'huile jaune (21%)
HPLC : > 95% dionex, gradient A, Rt 24,2 min.
Masse (C₂₁H₃₃N₃O₃ = 375,53 ; (ESI-TOF) m/z monoisotope, calculé pour [M+H]⁺ : 376,2599 g/mol, observé : 376,2008)

### Ester tert-butylique de l'acide [1-(1-méthylpipéridin-4-ylcarbamoyl)-2-phényléthyl]-carbamique 13e

Rdt : 47 mg de solide blanc (80%).
HPLC : > 95% dionex, gradient A, Rt 22,7 min.
Masse (C₂₀H₃₁N₃O₃ = 361,50 ; (ESI-TOF) m/z monoisotope, calculé pour [M+H]⁺: 362,2443 g/mol, observé : 362,2758)
¹H NMR (200MHz, CDCl₃) 7,24-7,19 (m, 5H, Ph) ; 5,47 (brd d, *J*=8,7 Hz, 1H, NH) ; 4,81 (q, *J*=6,4 Hz, 1H, HαPhe) ; 3,54 (m, 2H, H_{2,6}pipérazine) ; 3,31 (m, 1H, HβPhe) ; 2,95-3,05 (m, 3H, HβPhe & H_{2,6}pipérazine) ; 2,27 (m, 1H, Hpipérazine) ; 2,15-2,23 (m, 2H, H_{3,5}pipérazine) ; 2,17 (s, 3H, MeN); 1,74 (m, 1H, Hpipérazine) ; 1,42 (s, 9H, *t*Bu).
¹³C RMN (75MHz, CDCl₃) 170,78 (C_{amide}) ; 155,73 (C_{carbam}) ; 137,19 (C₁ph) ; 129,71 (C_{3,5}ph) ; 129,08 (C_{2,6}ph) ; 127,39 (C₄ph) ; 80,58 (C*t*Bu) ; 56,50 (CαPhe) ; 54,51 (C_{2,6}) ; 46,36 (C_{Me}N) ; 46,13 (C₄) ; 39,27 (C_{βPhe}) ; 32,07 (C_{3,5}) ; 28,69 (C*t*Bu).

### Ester tert-butylique de l'acide (1-[(1-méthylpipéridin-4-ylméthyl)-carbamoyl]-2-phényléthyl)-carbamique 13f

Rdt : 27 mg de solide blanc (34%).
HPLC : > 95% dionex, gradient A, Rt 23,4 min.
Masse (C₂₁H₃₂N₃O₃ = 375.53 ; (ESI-TOF) m/z monoisotope, calculé pour [M+H]⁺ : 376,2600 g/mol, observé : 376,2112)
¹H RMN (200MHz, CDCl₃) 7,37-7,19 (m, 5H, Ph) ; 5,94 (brd t, *J*=6,1 Hz, 1H, NH) ; 5,10 (brd, 1H, NH) ; 4,30 (q, *J*=7,1 Hz, 1H, HαPhe) ; 3,09 (m, 4H, H_{2,6} & HβPhe) ; 2,88 (brd d, *J*=11,7 Hz, 1H, H₇) ; 2,32 (s, 3H, MeN) ; 1,97 (td, *J*=11,5 Hz, *J*=2,9 Hz, 2H, H_{2,6}) ; 1,54-1,28 (m, 14H, H_{3,4,5} & *t*Bu).

### Protocole général pour la déprotection de groupement Boc

La résine fonctionnalisée par un groupement N-*tert*-butyloxycarbonyle (3,89 g ; 3,62 mmol ; 0,93 mmol.g⁻¹ charge théorique) est expansée dans 15 mL de DCM puis une solution de TFA/DCM 3:1 (35 mL) est ajoutée. Après une heure de deprotection à température ambiante, la résine est filtrée, et lavée en utilisant 3 séquences de DMF, 10% DIEA (DCM), dichlorométhane et MeOH puis Et₂O. La résine est ensuite séchée sous vide de la pompe à palette pendant 6h.

### Protocole général pour le couplage de la plate-forme de dérivatisation Fmoc-Lys(Boc)-OH

Une solution d'acide préactivé (ester d'HOBt) est préparé en mélangeant en premier la N-α-Fmoc-N-ε-Boc-Lysine (5,08 g ; 10,9 mmol) et HOBt (1,66 g ; 10,9 mmol) dans le DMF (30 ml) pendant 15 min à température ambiante. Le DIC (1,70 mL ; 10,9 mmol) dans le DMF (10 mL) est ensuite ajouté au mélange et le milieu est agité pendant 15 min à température ambiante. Pendant ce temps, l'amine primaire supportée (3,62 mmol) est expansée dans le DMF (30 mL) pendant 30 minutes sans agitation. La solution d'acide préactivé (10,9 mmol) est maintenant ajoutée à la résine et l'ensemble est agité sur le shaker rotatif pendant 6h à température ambiante. La résine est ensuite filtrée et lavée en utilisant 3 séquences de DMF, dichlorométhane et MeOH puis Et₂O. La résine est ensuite séchée sous vide de la pompe à palette pendant 6h.

### Ester tert-butylique de l'acide [5-[1-Benzyl-2-(4-méthylpipérazin-1-yl)-2-oxoéthyl carbamoyl]-5-(9H-fluorén-9-ylméthoxycarbonylamino)-pentylcarbamique 15a

Le composé **15a** est obtenu après quaternarisation en utilisant l'iodure de méthyle et élimination d'Hofmann en accord avec le protocole général.
Rdt : 99 mg de solide blanc (67%).
HPLC : > 95% dionex, gradient A, Rt 29,1 min.
Masse (C₄₀H₅₁N₅O₆ = 697,91 ; (ESI-TOF) m/z monoisotope, calculé pour [M+H]⁺ : 698,3917 g/mol, observé : 698,4302)
¹H RMN (200MHz, CDCl₃) 7.77-7,19 (m, 13H, Ph & Fmoc) ; 7,04 (brd d, *J*=7,3 Hz, 1H, NH) ; 5,55 (brd d, *J*=7,3 Hz, 1H, NH) ; 5,10 (q, *J*=7,3 Hz, 1H, HαLys) ; 4,76 (m, 1H, HαPhe) ; 4,39 (d, *J*=6,4 Hz, 2H, HFmoc) ; 4,21 (t, *J*=6,8 Hz, 1H, HFmoc) ; 3,54 (m, 2H, H_{2,6}pipérazine) ; 3,30 (m, 1H, HβPhe) ; 3,10-2,94 (m, 5H, HεLys, HβPhe & H_{2,6}pipérazine) ; 2,35-2,17 (m, 4H, H_{3,5}pipérazine) ; 2,18 (s, 3H, MeN) ; 1,81 (m, 2H, HβLys) ; 1,48-1,42 (m, 13H, HχLys, HδLys & tBu).
¹³C RMN (50MHz, CDCl₃) 171,54 (C_{amide}) ; 169,66 (C_{amide}) ; 156,58 (C_{carbam}) ; 144,21 (C_{fmoc}) ; 141,71 (C_{fmoc}) ; 136,30 (C₁ₚₕ) ; 129,98 (C_{3,5}ph) ; 129,02 (C_{2,6}ph) ; 128,14 (C_{fmoc}); 127,52 (C₄ₚₕ); 125,53 (C_{fmoc}); 120,39 (C_{fmoc}); 79,60 (C*t*Bu); 67,47 (C_{fmoc}); 55,18 (CαLys); 54,69 (C_{2,6}pipérazine); 50,23 (CαPhe); 47,58 (C_{fmoc}); 46,18 (C_{MeN}); 45,81 (C_{3,5}pipérazine); 42,34 (C_{3,5}pipérazine); 40,10 (CpPhe) ; 40,06 (C_{ε}Lys); 32,88 (C_{β}Lys); 29,99 (C_{δ}Lys); 28,86 (CtBu) ; 22,83 (C_{χ}Lys).

### Protocole général pour la déprotection de groupement Fmoc

La résine fonctionnalisée par un groupement N-9-fluorénylméthoxycarbonyle (4 g ; 2,8 mmol ; 0,7 mmol.g⁻¹ charge théorique) est expansée dans le DMF (20 mL) pendant 15 minutes puis l'excès de DMF est enlevé par filtration. Une solution de pipéridine dans le DMF (20% v/v) est ajoutée à la résine et l'ensemble est agité sur le shaker rotatif pendant 30 minutes à température ambiante. La résine est filtrée puis lavée en utilisant DMF, DCM et DMF puis la réaction est répétée une fois. Après 30 minutes, la résine est filtrée et le lavage final est réalisé en utilisant 3 séquences de DMF, dichlorométhane et MeOH puis Et₂O. La résine est ensuite séchée sous vide de la pompe à palette pendant 6h.

### Protocole général pour le couplage de l'acide carboxylique basique dont dérive R₃

Une solution d'acide carboxylique basique dont dérive R3 préactivé (ester d'HOBt) est préparée en mélangeant en premier le chlorhydrate d'acide (4,15 mmol) et HOBt (0,635 g ; 4,15 mmol) dans le DMF (3 ml) ainsi qu'une solution de DIEA (4,15 mmol) dans le DMF (3 mL) et en agitant pendant 15 min à température ambiante. Le DIC (0,523 g ; 4,15 mmol) dans le DMF (4 mL) est ensuite ajouté au mélange et le milieu est agité pendant 15 min à température ambiante. Pendant ce temps, l'amine primaire supportée (1 g ; 0,83 mmol ; 0,83 mmol.g⁻¹ charge théorique) est expansée dans le DMF (30 mL) pendant 30 minutes sans agitation. La solution d'acide préactivé (4,15 mmol) est maintenant ajoutée à la résine et l'ensemble agité sur le shaker rotatif pendant 6h à température ambiante. La résine est ensuite filtrée et lavée en utilisant 3 séquences de DMF, dichlorométhane et MeOH puis Et₂O. La résine est ensuite séchée sous vide de la pompe à palette pendant 6h.

Lorsque l'acide carboxylique n'est pas sous forme de chlorhydrate, la solution de DIEA dans le DMF n'est pas nécessaire et doit être remplacée par du DMF seul.

### Iodure de (5-[1-Benzyl-2-(4-méthylpipérazin-1-yl)-2-oxoéthylcarbamoyl]-5-(tert-butoxy-carbonyl aminopentylcarbamoyl)-méthyl)-1-méthylpyridinium 17a

Le composé **17a** est obtenu après quaternarisation en utilisant l'iodure de méthyle et élimination d'Hofmann en accord avec le protocole général.
Rdt : 121 mg de solide blanc (75%).
HPLC : 81% dionex, gradient A, Rt 21,9 min.
Masse (C₃₃H₄₉N₆O₅ = 609,81 ; (ESI-TOF) m/z monoisotope, calculé pour [M]⁺ : 609,3764 g/mol, observé : 609,3073)
¹H RMN (200MHz, CDCl₃) 9,52 (brd s, 1H, Py2) ; 8,83 (d, *J*=7,1 Hz, 1H, NH) ; 8,80 (d, *J*=5,6 Hz, 1H, Py4) ; 8,51 (d, *J*=7,8 Hz, 1H, Py6) ; 7,96 (t, *J*=7,3 Hz, 1H, Py5) ; 7,54 (d, *J*=7,8 Hz, 1H, NH) ; 7,30-7,15 (m, 5H, Ph) ; 5,05 (q, *J*=7,6 Hz, 1H, HαLys) ; 4,52 (s, 3H, PyMe) ; 4,98 (m, 1H, HαPhe) ; 4,26-4,33 (m, 2H, CH₂Py) ; 3,84 (d, *J*=14,7 Hz, 1H, NH) ; 3,46-3,34 (m, 3H, H_{2,6}pipérazine & HβPhe) ; 3,17-2,89 (m, 9H, HεLys, HβPhe & H_{2,6}pipérazine) ; 2,32-2,18 (m, 4H, H_{3,5}pipérazine) ; 2,18 (s, 3H, MeN) ; 1,81 (m, 2H, HβLys) ; 1,48-1,42 (m, 13H, HχLys, HδLys & *t*Bu).
¹³C RMN (50MHz, CDCl₃) 171,65 (C_{amide}) ; 170,11 (C_{amide}); 168,87 (C_{amide}); 156,58 (C_{carbam}); 146,68 (C_{6py}) 143,12 (C_{4py}) ; 138,37 (C_{2py}); 136,80 (C₁ₚₕ) ; 130,07 (C_{3,5ph}) ; 128,94 (C_{2,6ph}) ; 127,77 (C₄ₚₕ) ; 127,35 (C_{5py}) ; 126,28 (C_{3py}); 79,39 (C*t*Bu) ; 55,37 (C_{pyMe}); 55,14 (CαLys); 54,64 (C_{2,6}pipérazine); 50,14 (CαPhe); 49,36 (CH₂py); 46,18 (C_{MeN}); 45,81 (C_{3,5}pipérazine); 42,21 (C_{3,5}pipérazine); 40,61 (C_{β}Phe); 39,51 (C_{ε}Lys); 31,64 (C_{β}Lys); 29,72 (C_{δ}Lys); 28,90 (C*t*Bu) ; 23,60 (C_{χ}Lys)

### Protocole général pour la deprotection du groupement Boc sur l'azote ε de la Lysine et l'élongation de la chaîne latérale.

La résine fonctionnalisée par un groupement N-ε-*tert*-butyloxycarbonyl-lysine (0,735 g) est expansée dans 6 mL de DCM dans un « peptide vessel » puis une solution de TFA/DCM 3:1 (14 mL) est ajoutée. Après une heure de deprotection à température ambiante, la résine est filtrée, et lavée en utilisant 3 séquences de DMF, 10% DIEA (DCM), dichlorométhane et MeOH puis Et₂O. La résine est ensuite séchée sous vide de la pompe à palette pendant 6h. L'élongation de la chaîne latérale peut être réalisée en 2 étapes en faisant premièrement réagir l'amine déprotégée avec le *p-*nitrophénylchloroformate puis en déplaçant le groupe partant *p*-nitrophénol par une diamine (par exemple : 1,4-diaminobutane, 1,12-diaminododécane...). Ainsi, l'amine Boc-déprotégée et séchée (0,012 g ; 0,01 mmol ; 0,83 mmol.g⁻¹ charge théorique) est expansée dans une quantité minimale de dichlorométhane sec pendant 15 minutes dans une seringue Supelco. A la suspension est ajoutée 0,5 mL (0,1 mmol) d'une solution de DIEA [0,2 M] dans DCMsec/THF 1:1 puis 0,5 mL (0,1 mmol) d'une solution de p-nitrophénylchloroformate [0,2 M] dans DCMsec/THF 1:1. La réaction est effectuée à température ambiante pendant 2 heures. La résine est ensuite filtrée et lavée avec 2 séquences de dichlorométhane et THF puis 1 mL (0,2 mmol) d'une solution de diamine [0,2 M] est ajoutée et l'agitation est poursuivie pendant 15 heures à température ambiante. La résine est ensuite filtrée et lavée en utilisant 3 séquences de DMF, dichlorométhane et MeOH puis Et₂O. La résine est ensuite séchée sous vide de la pompe à palette pendant 6h.

### Protocole général pour le couplage du groupement fluorescent chlorure de Lissamine sulfonyle sur l'amine primaire supportée.

La résine sèche (0,3 g ; 0,243 mmol ; 0,81 mmol.g⁻¹ charge théorique) est expansée dans une quantité minimale de DCM pendant 15 minutes dans un ballon équipé d'une agitation magnétique. A la suspension est ajoutée 12 mL d'une solution de DIEA [0,04 M] dans le DCM (0,48 mmol) puis 12 mL d'une solution de chlorure de lissamine sulfonyle [0,04 M] dans le DCM (0,48 mmol). La réaction est effectuée à température ambiante avec une agitation lente pendant 5h. La résine est ensuite filtrée et lavée en utilisant 3 séquences de DMF, 10% AcOH (DCM), 10% DIPEA (DCM), dichlorométhane et MeOH puis 2 séquences de DMF et finalement Et₂O. La résine est ensuite séchée sous vide de la pompe à palette pendant 6h.

### (5-[1-Betazyl-2-(4-méthylpipérazin-1-yl)-2-oxoéthylcarbantoyl]-5-(3',6'-bis(diéthyl amino)-9'-(2-sulfophénylxanthyl-4-sulfonyl)aminopentylcarbantoyl)-méthyl)-1-méthyl pyridinium bis trifluoroacétate 22a

Le composé **22a** est obtenu après quaternarisation en utilisant l'iodure de méthyle et élimination d'Hofmann en accord avec le protocole général. Le clivage permet d'obtenir 182 mg de produit brut sous forme de solide violet foncé. Une purification est réalisée par RP-HPLC semi-préparative. Le mélange brut est solubilisé dans 7 mL de MeOH/H₂O 1:2 et 7 injections de 1 mL sont réalisées (gradient B).
Rdt : 77 mg de solide violet (25%).
HPLC : 96% dionex, gradient A, Rt 25,0 min.
Masse (C₅₅H₇₀N₈O₉S₂, 2TFA- = 1051,34 + 226,03 = 1277,37 ; (ESI-TOF) m/z monoisotope, calculé pour [M]⁺ : 1050,5043 g/mol, observé : 525,7598)
¹H RMN (200MHz, MeOD) 8,94 (brd s, 1H, Py2) ; 8,83 (d, *J*=5,6 Hz, 1H, Py4); 8,71 (d, *J*=1,5 Hz, 1H, liss) ; 8,51 (d, *J*=8,1 Hz, 1H, Py6) ; 8,16 (dd, *J*=2 Hz, *J*=8,1 Hz, 1H, liss) ; 8,06 (dd, *J*=6,1 Hz, *J*=7,8 Hz, 1H, Py5) ; 7,58 (d, *J*=7,8 Hz, 1H, liss) ; 7,40-7,15 (m, 5H, Ph) ; 6,95-7,21 (m, 6H, liss) ; 5,05 (m, 1H, HαLys caché par HOD) ; 4,43 (s, 3H, PyMe); 4,30 (m, 1H, HαPhe); 3,95 (d, *J*=2,5 Hz, 2H, CH₂Py); 3,69 (t, *J*=6,7 Hz, 8H, liss) ; 3,20-3,60 (m, 7H, caché par MeOH) ; 3,09-2,83 (m, 8H, Hεys, HβPhe, MeN & H_{pipérazine}); 1,73 (m, 2H, HβLys); 1,42-1,58 (m, 4H, HγLys & HδLys); 1,33 (m, 12H, liss).

Une autre fraction est obtenue correspondant à l'isomère de position du groupement sulfonamide sur la lissamine
Rdt : 20 mg de solide violet (6%).
Masse (C₅₅H₇₀N₈O₉S₂, 2TFA-=1051,34+226,03=1277,37 ; (ESI-TOF) m/z monoisotope, calculé pour [M]⁺ : 1050,5043 g/mol)
HPLC : 80% dionex, gradient A, Rt 24,4 min.
¹H RMN (200MHz, MeOD) 9,01 (brd s, 1H, Py2) ; 8,86 (d, *J*=6,1 Hz, 1H, Py4) ; 8,67 (s, 1H, liss) ; 8,55 (d, *J*=7,1 Hz, 1H, Py6) ; 8,27 (dd, *J*=1,7 Hz, *J*=7,8 Hz ,1H, liss) ; 8,08 (dd, *J*=6,4 Hz, *J*=8,3 Hz, 1H, Py5) ; 7,60 (d, *J*=7,8 Hz, 1H, liss) ; 7,40-7,25 (m, 5H, Ph) ; 6,95-7,20 (m, 6H, liss) ; 5,00 (m, 1H, HαLys caché par HOD) ; 4,49 (s, 3H, PyMe) ; 4,30 (m, 1H, HαPhe) ; 3,98 (m, 2H, CH₂Py) ; 3,73 (q, *J*=7,1 Hz, 8H, liss) ; 3,20-3,60 (m, 5H, caché par MeOH); 3,10-2,85 (m, 10H, HεLys, HβPhe, MeN & H_{pipérazine}) ; 1,67 (m, 2H, HβLys) ; 1,35-1,45 (m, 4H, HγLys & HδLys) ; 1,35 (t, *J*=7,1 Hz, 12H, liss).

### Composé 22b

Le composé **22b** est obtenu après quaternarisation en utilisant le bromure de benzyle et élimination d'Hofmann en accord avec le protocole général. Le clivage permet d'obtenir 264 mg de produit brut sous forme de solide violet foncé. Une purification est réalisée par RP-HPLC semi-préparative. Le mélange brut est solubilisé dans 6 mL de MeOH et 6 injections de 1 mL sont réalisées (gradient C).
Rdt : 44 mg de solide violet (20%).
HPLC : 95% dionex, gradient A, Rt 20,8 min.
Masse (C₇₁H₈₉N₉O₉S₂, 2TFA-=1276,67+226,03=1502,70; (ESI-TOF) m/z monoisotope, calculé pour [M]⁺ : 1275,6250 g/mol, observé : 637,8349)

Une autre fraction est obtenue correspondant à l'isomère de position du groupement sulfonamide sur la lissamine
Rdt : 16 mg de solide violet (7%).
HPLC : >95% dionex, gradient A, Rt 19,8 min.
Masse (C₆₆H₈₄N₈O₉S₂, 2TFA- = 1197,57 + 226,03 = 1423,60 ; (ESI-TOF) m/z monoisotope, calculé pour [M]⁺ : 1196,5778 g/mol)

### Composé 22c

Le composé **22c** est obtenu après quaternarisation en utilisant le bromure de benzyle et élimination d'Hofmann en accord avec le protocole général. Le clivage permet d'obtenir 264 mg de produit brut sous forme de solide violet foncé. Une purification est réalisée par RP-HPLC semi-préparative. Le mélange brut est solubilisé dans 6 mL de MeOH et 6 injections de 1 mL sont réalisées (gradient C).
Rdt: 83 mg de solide violet (27%).
HPLC : 95% dionex, gradient A, Rt 20,0 min.
Masse (C₆₆H₈₄N₈O₉S₂, 2TFA- = 1197,57 + 226,03 = 1423,60 ; (ESI-TOF) m/z monoisotope, calculé pour [M]⁺: 1196,5778 g/mol, observé : 598,3161)

Une autre fraction est obtenue correspondant à l'isomère de position du groupement sulfonamide sur la lissamine
Rdt : 28 mg de solide violet (9%).
HPLC : 85% dionex, gradient A, Rt 18,8 min.
Masse (C₆₆H₈₄N₈O₉S₂, 2TFA- = 1197,57 + 226,03 = 1423,60 ; (ESI-TOF) m/z monoisotope, calculé pour [M]⁺ : 1196,5778 g/mol)

### Exemple 1 : Expression du récepteur M1 fusionné à son extrémité amino-terminale à la protéine EGFP et criblage de chimiothèques fluorescentes.

### I) Fusion de la séquence codante du récepteur M1 à la EGFP

La séquence codante du récepteur muscarinique M1 humain (Genbank accession N° : X15263) est amplifiée par PCR en utilisant les amorces :

| | |
|---|---|
| Amorces sens : | 5' acc gcc gcc ggg atc tca gat ctc gga aag ggt ccc tgg |
| Amorce anti-sens : | 5' gca gga cgc agg ctc gag tca gca ttg gcg ggaggg |

Cette séquence est fusionnée en phase avec le peptide signal de la sous-unité alpha 7 de poulet (Genbank accession N° : **X52295**) du récepteur nicotinique de l'acétylcholine, qui est amplifié par PCR à l'aides des amorces :

| | |
|---|---|
| Amorces sens : | 5' ggt cgg ctg cgg ccg cat ggg cct ccg ggc gct |
| Amorce anti-sens : | 5' cca ggg acc ctt tcc gag atc tga gat ccc ggc ggc ggt |

Les séquences obtenues sont clivées par les enzymes Bg1II et Xho1 pour la séquence du récepteur M1, et par les enzymes Not1 et Bgl II pour le fragment peptide signal-EGFP. Ces fragments sont ensuite placés dans le vecteur KS ouvert par les enzymes Not1 et Xho1. La séquence SP-EGFP-M1 est alors sous clonée dans le vecteur d'expression pCEP4 (Invitrogen) avant d'être envoyée en séquençage **ainsi que décrit dans** Ilien et al. (J. Neurochem., 2003, 85: 768-78**).** Dans cette construction, le récepteur M1 est tronqué de 17 acides aminés dans sa partie amino-terminale, par rapport à sa séquence sauvage, et un bras espaceur long de 6 acides aminés sépare la GFP du récepteur.

### II) Expression des protéines recombinantes

Des cellules HEK 293 sont transfectées, par la méthode de la précipitation au phosphate de calcium (Chen & Okayama 1987, Mol.Cell.Biol.7 : 2745-2752) par la construction pCEP4-SP-EGFP-M1. Des lignées stables sont établies par sélection des cellules transfectées résistantes à l'hygromycine (100 µg/ml, Clontech). Les cellules sont cultivées dans un milieu MEM (Gibco) supplémenté de 10% de sérum de veau foetal (Sigma), de Pénicilline (100 unités/ml), streptomycine (100 µg/ml) et de glutamine (4 mM).

### III) Mesures de l'expression du récepteur M1 fusionné à la EGFP, par fluorimétrie

Les expériences de fluorescence sont effectuées dans une cuvette de 1ml pourvue d'un système d'agitation magnétique et placée dans un spectrofluorimètre Fluorolog (SPEX) équipé d'une lampe Xe 450 W (Osram) et de monochromateurs Spex 1680 0.22 m (excitation) et Spex 1681 0.22 m (émission). Les cellules sont mises en suspension dans un tampon physiologique : Hepes 10 mM, NaCl 137,5 mM, MgCl₂ 1,25 mM, CaCl₂ 1,25 mM, KCl 6 mM, glucose 5,6 mM, NaH₂PO₄ 0,4 mM, BSA 0,1 % (W/v), pH 7,4.

Les cellules entières sont récoltées après traitement à la trypsine-EDTA (1x Gibco) à température ambiante. Les cellules sont centrifugées 5 minutes à 100 g et sont remises en suspension à une concentration de 1 à 2.10⁶ cellules/ml dans le tampon physiologique.

Un spectre d'émission des cellules transfectées par le pCEP4-SP-EGFP-M1 est représenté dans la figure 1. Un spectre est réalisé avant chaque criblage. Les spectres font clairement apparaître le signal de la EGFP, indiquant que ces cellules expriment bien la EGFP en comparaison des cellules non transfectées.

### IV) Criblage de collections de composés fluorescents sur la lignée HEK exprimant le récepteur M1 fusionné à la EGFP

### IV.1. Procédure de criblage

La distribution des ligands et des cellules est réalisée à l'aide d'un robot pipetteur distributeur (Biomek 2000) et la fluorescence est mesurée dans un spectrofluorimètre (Victor II, Perkin Elmer).

Dans une plaque noire, 96 puits (Labsystem) sont distribués, chronologiquement, 98 µl de tampon physiologique, 2 à 4 µl de ligand fluorescent (correspondant à un composé marqué selon la présente invention) et 100 000 cellules (100 µl) d'une suspension cellulaire.

Les ligands fluorescents sont criblés à une concentration finale variant de 500 nM à 1 µM. Ces ligands sont dissous dans le DMSO, dont la concentration finale dans l'essai varie entre 1 et 2 %. Les cellules sont distribuées depuis un réservoir, dans lequel les cellules sont gardées en suspension par une alternance d'aspiration et refoulement effectuée par le robot avant chaque distribution. Afin de mélanger l'ensemble des composés dans la plaque, le robot aspire et refoule une fois un volume de 50 µl, soit la moitié du puits. La plaque est ensuite incubée 10 minutes à température ambiante, puis centrifugée 5 minutes à 100 g. Elle est ensuite placée dans le détecteur de fluorescence Victor. La EGFP est excitée à 465 ± 7 nm et son émission est mesurée à 510 ± 7 nm, pendant 2 secondes, à 0,8 mm du fond de la plaque.

Dans une plaque 96 puits, 16 puits servent de contrôle et 80 ligands fluorescents sont testés, soit un par puits. L'émission de la EGFP (510 nm) de chacun des puits traités est comparée à la fluorescence moyenne de la EGFP (510 nm) des 16 puits contrôle. Un pourcentage d'extinction de fluorescence est ainsi calculé et correspond au critère d'identification des touches.

400 ligands fluorescents ont été testés suivant ce protocole, quarante ligands ont été identifiés comme ligands potentiels, appelés touches. Les deux ligands qui donnaient un pourcentage d'extinction de la fluorescence (à 510 nm) le plus élevé ont été re-synthétisés et purifiés par fraction. Chaque fraction a été testée d'une part, en liaison sur des cellules exprimant le récepteur M1 fusionné à la EGFP et d'autre part en absorbance (220 nm). Cette expérience (figure 2) démontre que c'est le produit fluorescent principal de la synthèse, qui induit l'extinction de fluorescence de la EGFP.

### IV.2. Confirmation des touches et structure des ligands détectés

Les touches identifiées lors du criblage sont confirmées au spectrofluorimère (matériel précédemment décrit). Les mesures sont effectuées dans une cuvette de 1 ml avec une suspension de 1 à 2.10⁶ cellules/ml de tampon physiologique (même composition citée précédemment)(voir Figures 3A et 3B ; Figure 4).

D'après la figure 4, on observe clairement l'extinction de fluorescence de la EGFP à 510 nm, dû au transfert d'énergie au ligand fluorescent. On observe un retour à une émission maximale de la EGFP à 510 nm. Le ligand fluorescent est chassé de son site de liaison par l'antagoniste. Le transfert d'énergie vers le ligand fluorescent est interrompu et l'émission de la EGFP est à nouveau maximale.

Un des ligands préférés découverts par criblage, nommé FG 229 D9, possède la structure suivante :

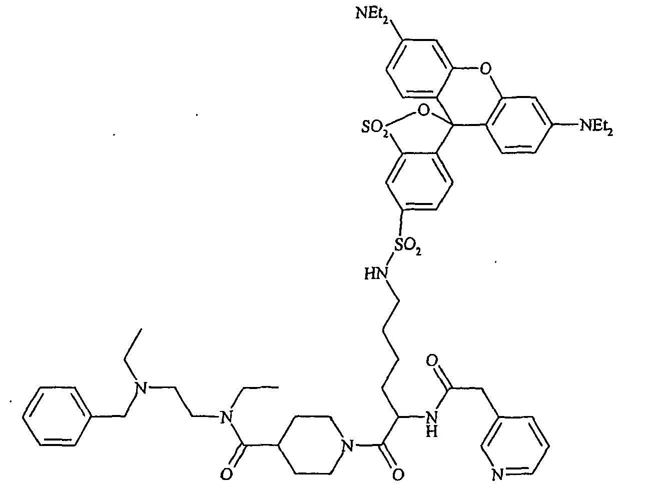

Un tel composé correspond à un composé de formule (I) selon l'invention, dans laquelle n = 0, p = 4, R₁ et R' ₁ représentent un groupe éthyle, R₂ représente la chaîne latérale de l'acide isonipécotinique, R₃ représente

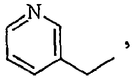

R₄ représente un groupe benzyle et A représente un dérivé de la lissamine.

L'activité biologique de ce composé sur le test de FRET est illustrée sur les figures 3 et 4.

### Exemple 2 : Expression du récepteur orphelin GPR 50 fusionné à son extrémité amino-terminale à la protéine EGFP et criblage de chimiothèque fluorescente.

### I) Fusion de la séquence codante du récepteur GPR 50 à la EGFP

La séquence codante du récepteur GPR50 (Genbank accession N°: U 52219) est placée dans le vecteur pCEP4-SP-EGFP (précédemment décrit dans l'exemple 1). Ces extrémités 3' et 5' contiennent des séquences complémentaires aux extrémités 5' et 3' du vecteur pCEP4-SP-EGFP digéré par Fse1. Ces séquences complémentaires ont été introduites dans les amorces utilisées lors de la PCR (séquences soulignées) pour amplifier la séquence codante du récepteur.
Amorce sens : 5' G GCC GGG GCC GGG ACC CCC TAT GGC TGT ATT GGC
Amorce anti-sens : 5' CTC GTT CTC GTT GGA TCA CAC AGC CAT TTC ATC AGG ATC

Ce procédé d'insertion directionnelle de fragments de PCR dans un vecteur, ne fait pas intervenir d'enzyme de restriction (Aslanidis C et al ; 1990, Nucleic Acids Res.18, 6069-6074, Haun R.S. et al ; 1992, BioTechniques 13, 515-518).

Dans la construction pCEP4-SP-EGFP-GPR50, un bras espaceur long de 6 acides aminés sépare la EGFP du récepteur. Quant au récepteur, il possède une extrémité amino-terminale longue de 30 acides aminés, qui a été tronquée de 9 acides aminés par rapport à sa séquence sauvage.

### II) Expression des protéines recombinantes

Des cellules HEK 293 sont transfectées, par la méthode de la précipitation au phosphate de calcium (Chen & Okayama 1987, Mol.Cell.Biol.7 : 2745-2752) par la construction pCEP4-SP-EGFP-GPR50. Des lignées stables sont établies par sélection des cellules transfectées résistantes à l'hygromycine (500 µg/ml, Clontech). Les cellules sont cultivées dans un milieu MEM (Gibco) supplémenté de 10% de sérum de veau foetal (Sigma), de Pénicilline (100 unités/ml), streptomycine (100 µg/ml) et de glutamine (4 mM).

### III) Mesures de l'expression du récepteur GPR50 fusionné à la EGFP, par fluorimétrie

Les expériences de fluorescence sont effectuées dans une cuvette de 1ml pourvue d'un système d'agitation magnétique et placée dans un spectrofluorimètre Fluorolog (SPEX) équipé d'une lampe Xe 450 W (Osram) et de monochromateurs Spex 1680 0.22 m (excitation) et Spex 1681 0.22 m (émission). Les cellules sont mises en suspension dans un tampon physiologique : Hepes 10 mM, NaCl 137,5 mM, MgCl₂ 1,25 mM, CaCl₂ 1,25 mM, KCl 6 mM, glucose 5,6 mM, NaH₂PO₄ 0,4 mM, BSA 0,1 % (W/v), pH 7,4.

Les cellules entières sont récoltées après traitement à la trypsine-EDTA (1x Gibco) à température ambiante. Les cellules sont centrifugées 5 minutes à 100 g et sont remises en suspension à une concentration de 1 à 2.10⁶ cellules/ml dans le tampon physiologique.

Un spectre d'émission des cellules transfectées par le pCEP4-SP-EGFP-GPR50 est représenté dans la figure 5. Un spectre est réalisé avant chaque criblage. Les spectres font clairement apparaître le signal de la EGFP, indiquant que ces cellules expriment bien la EGFP en comparaison des cellules non transfectées.

### IV) Criblage de collections de composés fluorescents sur la lignée HEK exprimant le récepteur GPR50 fusionné à la EGFP

### IV.1. Procédure de criblage

La distribution des ligands et des cellules est réalisée à l'aide d'un robot pipetteur distributeur (Biomek 2000) et la fluorescence est mesurée dans un spectrofluorimètre (Victor II, Perkin Elmer).

Dans une plaque noire, 96 puits (Labsystem), sont distribués, chronologiquement, 98 µl de tampon physiologique, 2 à 4 µl de ligand fluorescent et 100 000 cellules (100 µl) d'une suspension cellulaire.

Les ligands fluorescents sont criblés à une concentration finale variant de 500 nM à 1 µM. Ces ligands sont dissous dans le DMSO, dont la concentration finale dans l'essai varie entre 1 et 2 %. Les cellules sont distribuées depuis un réservoir, dans lequel les cellules sont gardées en suspension par une alternance d'aspiration et refoulement effectuée par le robot avant chaque distribution. Afin de mélanger l'ensemble des composés, le robot aspire et refoule une fois, la moitié du puits. La plaque est ensuite incubée 10 min à température ambiante, puis centrifugée 5 min à 100g. Elle est ensuite placée dans le détecteur de fluorescence Victor. La EGFP est excitée à 465 ± 7 nm et son émission est mesurée à 510 ± 7 nm, pendant 2s, à 0.8 mm du fond de la plaque.

Dans une plaque 96 puits, 16 puits servent de contrôle et 80 ligands fluorescents sont testés, soit un par puits. L'émission de la EGFP (510 nm) de chacun des puits traités est comparée à la fluorescence moyenne de la EGFP (510 nm) des 16 puits contrôle. Un pourcentage d'extinction de fluorescence est ainsi calculé et correspond au critère d'identification des touches.

Trois mille ligands fluorescents ont été testés suivant ce protocole.

### IV.2. Confirmation et structure des touches potentielles

La liaison de certaines molécules a été vérifiée au spectrofluorimère (matériel précédemment décrit (voir exemple 1)). Les mesures sont effectuées dans une cuvette de 1 ml avec une suspension de 1 à 2.10⁶ cellules/ml de tampon physiologique (composition citée dans l'exemple 1).

Un des ligands préférés découverts par criblage (FG 229 F4 ; voir Figure 6) possède la structure suivante :

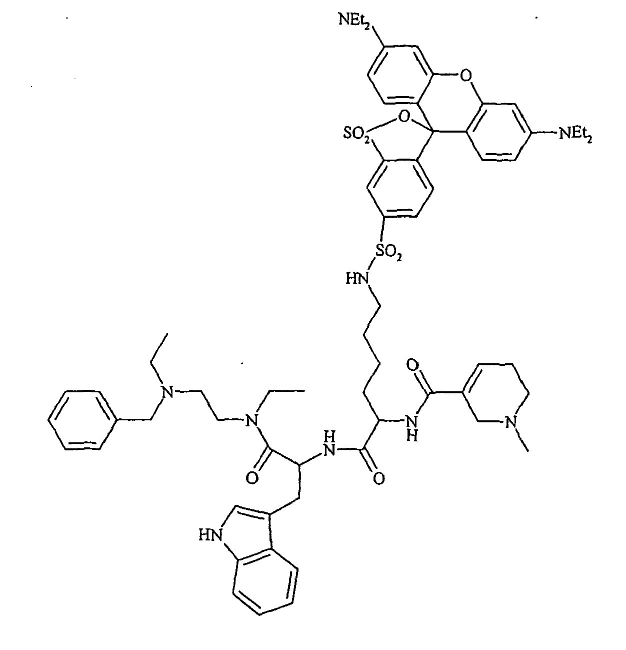

Un tel composé correspond à un composé de formule (I) selon l'invention, dans laquelle n = 0, p = 4, R₁ et R' ₁ représentent un groupe éthyle, R₂ représente la chaîne latérale du tryptophane, R₃ représente

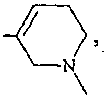

R₄ représente un groupe benzyle et A représente un dérivé de la lissamine.

### Exemple 3 : Expression du récepteur mGluR8 fusionné à son extrémité amino-terminale à la protéine GFP et criblage de chimiothèques fluorescentes.

### I) Fusion de la séquence codante du récepteur mGluR8 à la GFP

La séquence codante du récepteur métabotropique au glutamate sous type 8 (mGluR8) (Genbank accession N° : P70579) est placée dans le vecteur pcDNA6 (Invitrogen) en aval de la séquence codante de la protéine GFP, elle-même placée en aval de la séquence codant pour le peptide signal de la sous-unité alpha 7 de poulet du récepteur nicotinique de l'acétylcholine (correspondant à la séquence en acide aminé 1 à 25 de la séquence de la protéine décrite dans la Genbank accession N°X52295). La construction obtenue est appelée pcDNA6-SP-GFP-mGluR8.

Dans la construction pcDNA6-SP-GFP-mGluR8, un bras espaceur long de 6 acides aminés sépare la GFP du récepteur. Quant au récepteur, son extrémité amino-terminale, composée de 550 acides aminés, a été tronquée de 547 acides aminés. Dans cette construction, il y a donc 9 acides aminés qui séparent la GFP du premier domaine transmembranaire du récepteur.

### II) Expression des protéines recombinantes

Des cellules HEK 293 sont transfectées par un agent de transfection la lipofectamine 2000 (Invitrogen) selon les instructions du fournisseur avec la construction pcDNA6-SP-GFP-mGluR8. Des lignées stables sont établies par sélection des cellules transfectées résistantes à la blasticidine (5 µg/ml, Invivogen). Les cellules sont cultivées dans un milieu MEM (PAA) contenant de la L-glutamine (2 mM) et supplémenté de 10% de sérum de veau foetal (PAA), de Pénicilline (100 unités/ml), streptomycine (100 µg/ml).

### III) Mesures de l'expression du récepteur mGluR8 fusionné à la GFP, par fluorimétrie

Les expériences de fluorescence sont effectuées dans une cuvette de 1ml pourvue d'un système d'agitation magnétique et placée dans un spectrofluorimètre Fluorolog-3 (Jobin-Yvon Horiba). Les cellules sont mises en suspension dans un tampon physiologique : Hepes 10 mM ; NaCl 137,5 mM ; MgCl₂ 1,25 mM ; CaCl₂ 1,25 mM ; KCl 6 mM ; glucose 5,6 mM ; NaH₂PO₄ 0,4 mM; BSA 0,1% (W/v) pH 7,4.

Les cellules entières sont récoltées après traitement au PBS (PAA), EDTA 5 mM à température ambiante. Les cellules sont centrifugées 5 minutes à 100g et sont remises en suspension à une concentration de 1 à 2.10⁶ cellules/ml dans le tampon physiologique.

Un spectre d'émission des cellules transfectées par le pcDNA-SP-GFP-mGluR8 est représenté dans la figure 7. Un spectre est réalisé avant chaque criblage. Les spectres font clairement apparaître le signal de la GFP, indiquant que ces cellules expriment bien la GFP en comparaison des cellules non transfectées.

### IV) Criblage de collections de composés fluorescents sur la lignée HEK exprimant le récepteur mGluR8 fusionné à la GFP

### IV.1. Procédure de criblage

Vingt quatre heures avant le criblage, les cellules sont ensemencées dans des plaques 96 puits. Au moment de l'expérience, les puits sont rincés et les cellules incubées dans un tampon physiologique dont la composition est décrite dans le paragraphe III. Les ligands fluorescents à tester sont distribués sur les cellules par la Flexstation (Molecular Devices) qui suit l'évolution de la fluorescence de la GFP (excitation 475 +/- 8 nm, émission 510 +/- 8 nm) au cours du temps.

Les ligands fluorescents sont criblés à une concentration finale variant de 500 nM à 1 µM. Ces ligands sont dissous dans le DMSO, dont la concentration finale dans l'essai varie entre 1 et 2%. Un pourcentage d'extinction de fluorescence de la GFP est calculé pour chaque puits et correspond au critère d'identification des touches.

Environ 800 ligands fluorescents issus de la chimiothèque décrite dans la présente invention ont été testés suivant ce protocole, un ligand a été identifié comme ligand potentiel du mGluR8.

### IV.2. Confirmation des touches et structure des ligands détectés

Les touches identifiées lors du criblage sont confirmées au spectrofluorimère (matériel précédemment décrit). Les mesures sont effectuées dans une cuvette de 1 ml sur des cellules HEK exprimant le récepteur GFP-mGluR8, diluées dans du tampon physiologique (même composition citée précédemment). La figure 8 illustre la cinétique d'extinction de fluorescence de la GFP lors de l'interaction d'un ligand fluorescent appelé BCF1A9 avec le récepteur GFP-mGluR8.

### Exemple 4 : Expression du récepteur du Glucagon-like peptide -1 (GLP-1) fusionné à son extrémité amino-terminale à la protéine GFP et criblage de chimiothèque fluorescente.

### I) Fusion de la séquence codante du récepteur GLP-1 à la GFP

Un récepteur GLP-1R fluorescent est obtenu par fusion d'une partie de sa séquence codante à celle de la GFP. La séquence codante du récepteur GLP-1 (Genbank accession N°: NM_002062) est placée dans un vecteur pcDNA6 (Invitrogen) en aval de la séquence codante de la protéine autofluorescente GFP, elle-même placée en aval de la séquence codante pour le peptide signal de la sous-unité alpha 7 de poulet du récepteur nicotinique de l'acétylcholine (correspondant à la séquence en acide aminé 1 à 25 de la séquence de la protéine décrite dans la Genbank accession N° : NP_989512). Dans la construction ainsi obtenue, appelée pCDNA6-SP-GFP-GLP1R, un bras espaceur correspondant à la séquence codant pour deux acides aminés sépare la GFP du récepteur. Quant au récepteur, une version tronquée de 134 acides aminés dans sa partie amino-terminale a été utilisée pour cette étude. Cette variante du récepteur possède la caractéristique de ne plus permettre la liaison de son ligand endogène, le peptide apparenté au Glucagon, GLP-1.

### II) Expression des protéines recombinantes

Des cellules HEK 293 sont transfectées par un agent de transfection la lipofectamine 2000 (Invitrogen) selon les instructions du fournisseur avec la construction pcDNA6-SP-GFP-GLP-1R. Des lignées stables sont établies par sélection des cellules transfectées résistantes à la blasticidine (5 µg/ml, Invivogen). Les cellules sont cultivées dans un milieu MEM (PAA) contenant de la L-glutamine (2 mM) et supplémenté de 10% de sérum de veau foetal (PAA), de Pénicilline (100 unités/ml) et de streptomycine (100 µg/ml).

### III) Mesures de l'expression du récepteur GLP-1 fusionné à la GFP, par fluorimétrie

Les mesures de l'expression du récepteur GLP-1 fusionné à la GFP par fluorimétrie sont réalisées comme décrit précédemment dans l'exemple 3.

### IV) Criblage de collections de composés fluorescents sur la lignée HEK exprimant le récepteur GLP1R fusionné à la GFP

Les procédures de criblage et de confirmation des touches ont été réalisées comme précédemment décrit dans l'exemple 3.

## Revendications

1. Utilisation d'une collection de composés répondant à la formule générale (I) suivante : 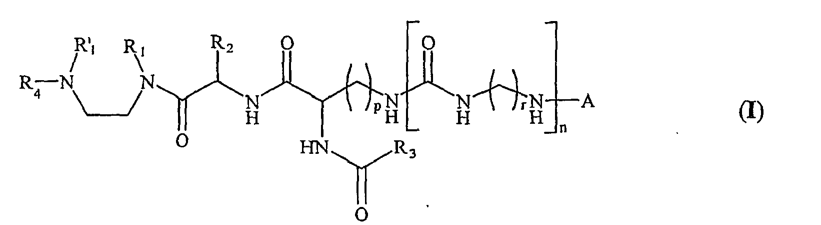 dans laquelle :
- n est égal à 0 ou 1,
- p représente un nombre entier variant de 1 à 6, et est de préférence égal à 4,
- r représente un nombre entier variant de 1 à 12, et est de préférence égal à 4,
- R₁ et R'₁ représentent indépendamment l'un de l'autre un atome d'hydrogène, un groupe alkyle comprenant de 1 à 6 atomes de carbone, ou un groupe aromatique, notamment un groupe benzyle ou un groupe phénéthyle, éventuellement substitués par un atome d'halogène, un groupe méthoxy ou un groupe alkyle comprenant de 1 à 6 atomes de carbone,
ou R₁ et R'₁ peuvent former un cycle comprenant de 2 à 4 atomes de carbone, notamment de 2 atomes de carbone,
- R₂ représente une chaîne latérale d'acide aminé ou d'un dérivé d'acide aminé,
- R₃ représente un groupe dérivé d'un acide carboxylique, porteur d'une entité basique, notamment choisi parmi les groupes suivants : 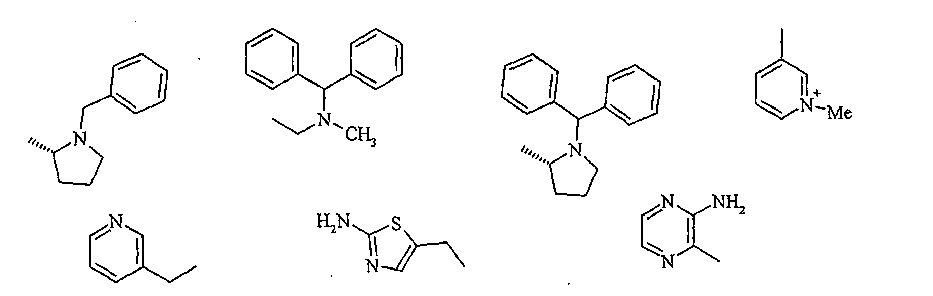
- R₄ représente un groupe alkyle comprenant de 1 à 10 atomes de carbone, notamment de 1 à 6 atomes de carbone, un groupe phényle substitué par un groupe alkyle comprenant de 1 à 10 atomes de carbone, notamment de 1 à 6 atomes de carbone, R₄ étant de préférence un groupe méthyle, un groupe benzyle ou un groupe allyle, et
- A représente un atome d'hydrogène, un groupe protecteur ou un groupement traceur, notamment un fluorophore, un colorant ou un "quencher",
pour la détermination *in vitro,* par étude de liaison, de ligands de récepteurs dont on ne connaît pas de ligand ou dont on ne connaît pas de ligand utilisable pour effectuer des essais de liaison d'affinité spécifique.

2. Composé de formule (I) suivante : 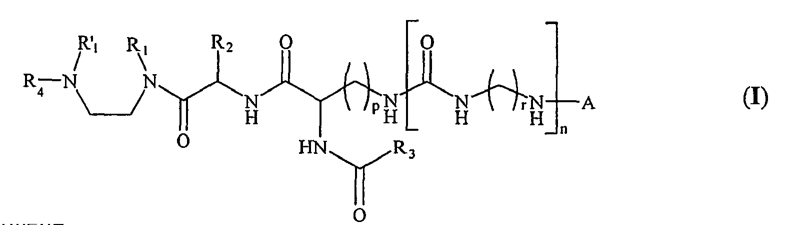 dans laquelle :
- n est égal à 0 ou 1,
- p représente un nombre entier variant de 1 à 6, et est de préférence égal à 4,
- r représente un nombre entier variant de 1 à 12, et est de préférence égal à 4,
- R₁ et R'₁ représentent indépendamment l'un de l'autre un atome d'hydrogène, un groupe alkyle comprenant de 1 à 6 atomes de carbone, ou un groupe aromatique, notamment un groupe benzyle ou un groupe phénéthyle, éventuellement substitués par un atome d'halogène, un groupe méthoxy ou un groupe alkyle comprenant de 1 à 6 atomes de carbone,
ou R₁ et R' ₁ peuvent former un cycle comprenant de 2 à 4 atomes de carbone, notamment de 2 atomes de carbone,
- R₂ représente une chaîne latérale d'acide aminé ou d'un dérivé d'acide aminé,
- R₃ représente un groupe dérivé d'un acide carboxylique, porteur d'une entité basique, notamment choisi parmi les groupes suivants : 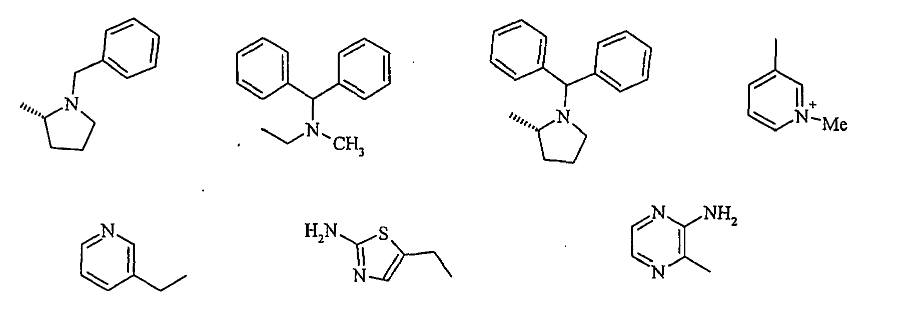
- R₄ représente un groupe alkyle comprenant de 1 à 10 atomes de carbone, notamment de 1 à 6 atomes de carbone, un groupe phényle substitué par un groupe alkyle comprenant de 1 à 10 atomes de carbone, notamment de 1 à 6 atomes de carbone, R₄ étant de préférence un groupe méthyle, un groupe benzyle ou un groupe allyle, et
- A représente un atome d'hydrogène, un groupe protecteur ou un groupement traceur, notamment un fluorophore, un colorant ou un "quencher".

3. Composé selon la revendication 2, **caractérisé en ce que** R₂ représente la chaîne latérale d'un acide aminé ou dérivé d'acide aminé choisi dans le groupe comprenant : l'alanine, la glutamine, la leucine, la glycine, le tryptophane, la β-alanine, la phénylalanine, la 4-chloro-phénylalanine, l'acide isonipécotinique, l'acide 4-aminométhylbenzoïque, l'acide 3-tétrahydroisoquinoléinique et l'histidine libre ou benzylée.

4. Composé selon la revendication 2 ou 3, **caractérisé en ce que** n est égal à 0.

5. Composé selon la revendication 2 ou 3, **caractérisé en ce que** n est égal à 1.

6. Composé selon la revendication 4, **caractérisé en ce que** A représente un atome d'hydrogène ou un groupe protecteur, notamment un groupe Boc.

7. Composé selon la revendication 6, répondant à l'une des formules suivantes: 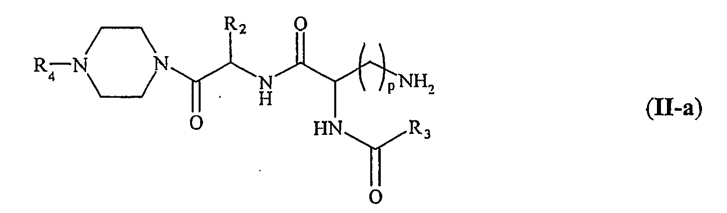 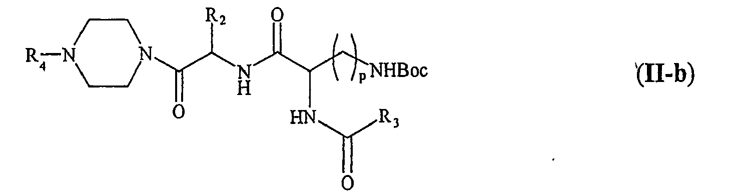 dans lesquelles R₂, R₃, R₄ et p sont tels que définis dans la revendication 2, p étant de préférence égal à 4.

8. Composé selon la revendication 4 ou 5, **caractérisé en ce que** A représente un groupe fluorophore choisi parmi le groupe comprenant les dérivés du Bodipy et de la lissamine.

9. Composé selon la revendication 8, **caractérisé en ce que** A représente l'un des groupes suivants :

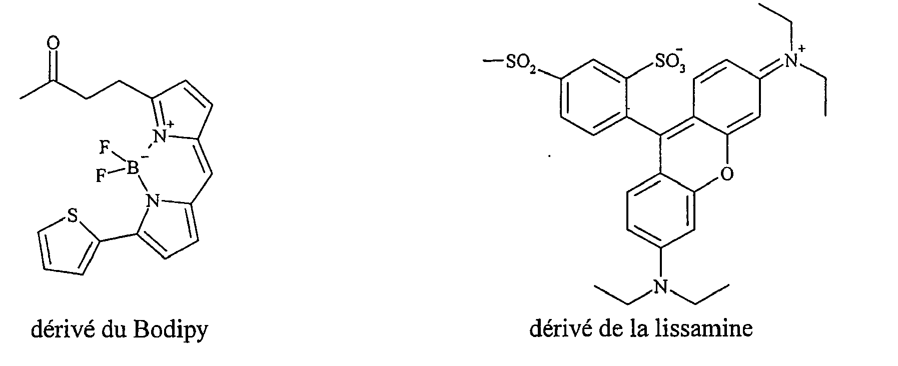

10. Composé selon la revendication 8 ou 9, répondant à la formule (III) suivante : 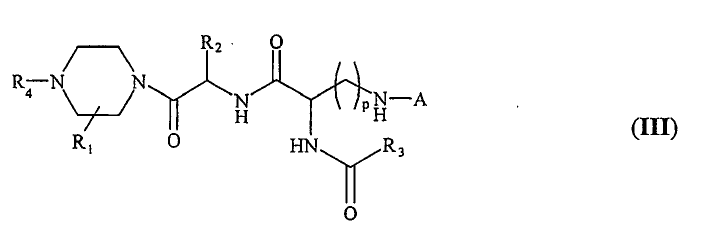 dans laquelle :
- R₁, R₂, R₃, R₄ et p sont tels que définis dans la revendication 2 et
- A est tel que défini dans la revendication 8 ou 9.

11. Composé selon la revendication 10, **caractérisé en ce que** p = 4 et répondant à la formule suivante : 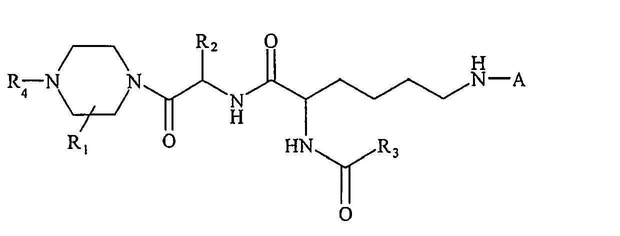 dans laquelle R₁, R₃ et R₄ sont tels que définis dans la revendication 2 et R₂ est tel que défini dans la revendication 3.

12. Composé selon la revendication 10 ou 11, répondant à la formule (III-a) suivante : 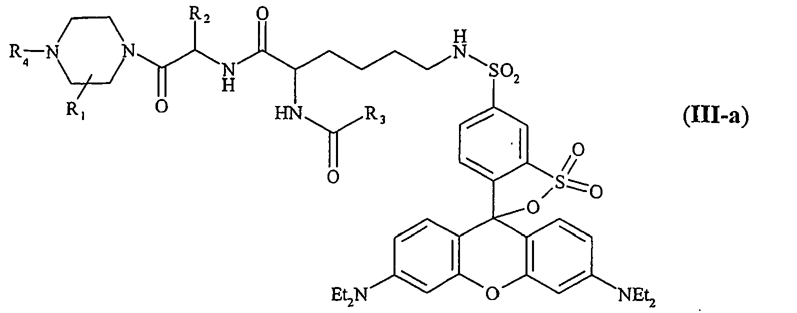 dans laquelle R₁, R₃ et R₄ sont tels que définis dans la revendication 2 et R₂ est tel que défini dans la revendication 3.

13. Composé selon la revendication 8 ou 9, répondant à la formule (IV) suivante : 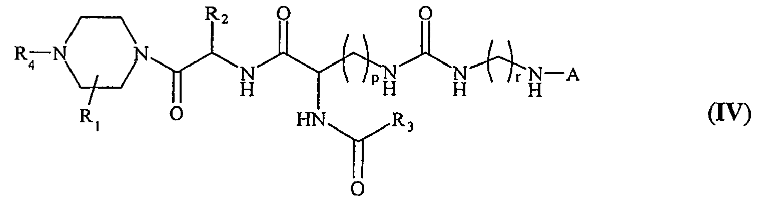 dans laquelle :
- R₁, R₂, R₃, R₄, p et r sont tels que définis dans la revendication 2 et
- A est tel que défini dans la revendication 8 ou 9.

14. Composé selon la revendication 13, **caractérisé en ce que** p = 4 et répondant à la formule suivante :

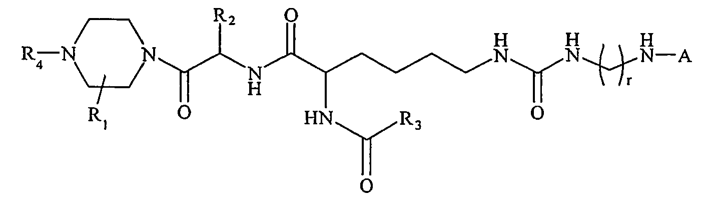

15. Composé selon la revendication 13, **caractérisé en ce que** r = 4 et répondant à la formule suivante :

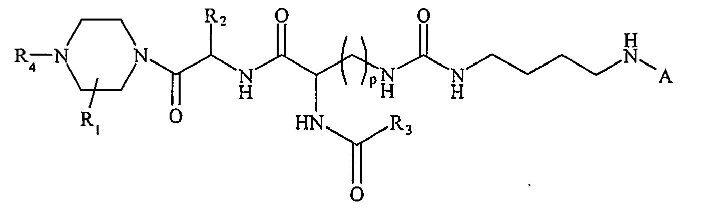

16. Composé selon la revendication 13, **caractérisé en ce que** p et r sont égaux à 4 et répondant à la formule suivante :

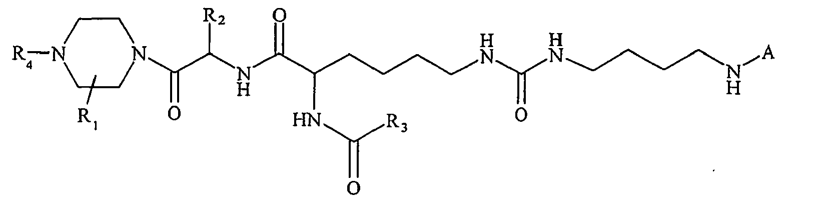

17. Composé selon la revendication 13, répondant à la formule (IV-a) suivante : 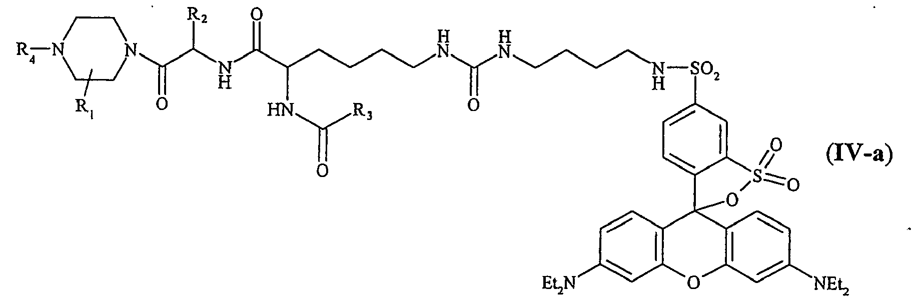 dans laquelle R₁, R₃ et R₄ sont tels que définis dans la revendication 2 et R₂ est tel que défini dans la revendication 3.

18. Collection comprenant une pluralité de composés de formule (I) telle que définie dans la revendication 2.

19. Collection comprenant une pluralité de composés de formule (III-a) telle que définie dans la revendication 12.

20. Collection comprenant une pluralité de composés de formule (IV-a) telle que définie dans la revendication 17.

21. Utilisation d'une collection selon la revendication 19 ou 20, pour la détermination *in vitro,* par étude de liaison, de ligands de récepteurs dont on ne connaît pas de ligand ou dont on ne connaît pas de ligand utilisable pour effectuer des études de liaison d'affinité spécifique.

22. Procédé de criblage de ligands de récepteurs dont on ne connaît pas de ligand ou dont on ne connaît pas de ligand utilisable, ledit procédé comprenant les étapes suivantes :
- la mise en présence d'une collection de composés traçables de formule (I) selon la revendication 2, avec des cellules transfectées par une construction contenant la fusion de la séquence codant pour une protéine fluorescente, avec la séquence nucléotidique codant pour un récepteur dont on ne connaît pas de ligand ou dont on ne connaît pas de ligand utilisable, et le mélange desdites cellules et de ladite collection,
- la détection de la fluorescence dudit mélange, par excitation de ladite protéine fluorescente et mesure de la fluorescence d'émission de ladite protéine fluorescente, et la détermination du pourcentage d'extinction de fluorescence en comparant la fluorescence d'émission de ladite protéine fluorescente à la fluorescence moyenne de ladite protéine fluorescente,
la fluorescence moyenne de ladite protéine fluorescente étant mesurée par des essais témoins correspondant à la mesure de la fluorescence de la protéine fluorescente en l'absence de la collection de composés, et
- la détermination des composés qui donnent un pourcentage d'extinction de la fluorescence de la protéine fluorescente d'au moins 5% et leur identification en tant que ligand

23. Procédé de préparation sur support solide d'un composé de formule (I) selon la revendication 2, **caractérisé en ce qu'**il comprend les étapes suivantes :
- le couplage d'un support solide de formule 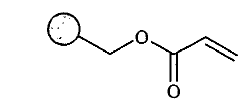 avec un composé de formule 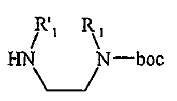 pour obtenir un composé de formule (1) suivante : 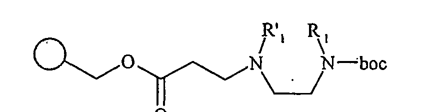 R₁ et R' ₁ étant tels que définis dans la revendication 2,
- la déprotection du composé de formule (1) suivie du couplage dudit composé déprotégé avec le composé de formule 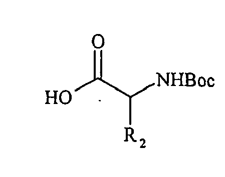 pour obtenir un composé de formule (2) suivante : 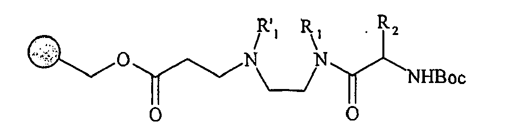 R₂ étant tel que défini dans la revendication 2,
- la déprotection du composé de formule (2) suivie du couplage dudit composé déprotégé avec le composé de formule 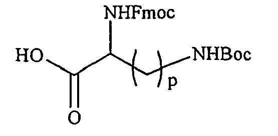 pour obtenir un composé de formule (3) suivante : 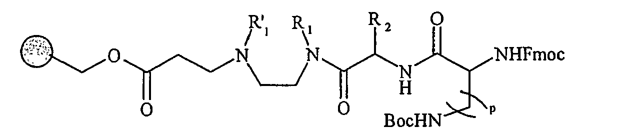 p étant tel que défini dans la revendication 2,
- la déprotection du groupe Fmoc du composé de formule (3) suivie du couplage dudit composé déprotégé avec le composé R₃COOH pour obtenir un composé de formule (4) suivante : 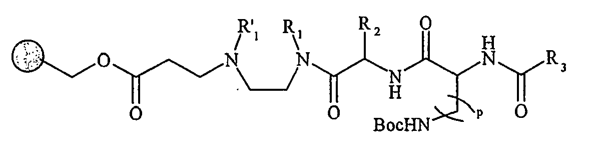 R₃ étant tel que défini dans la revendication 2,
- la déprotection du composé de formule (4) afin d'obtenir un composé de formule (5) suivante : 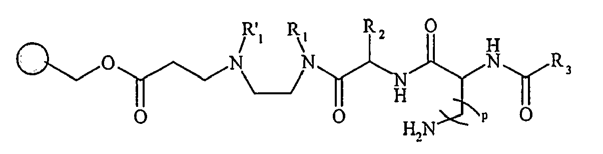
- éventuellement la réaction du composé de formule (5) avec le p-nitrophénylchloroformate puis avec la diamine de formule 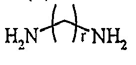 pour obtenir le composé de formule (6) suivante : 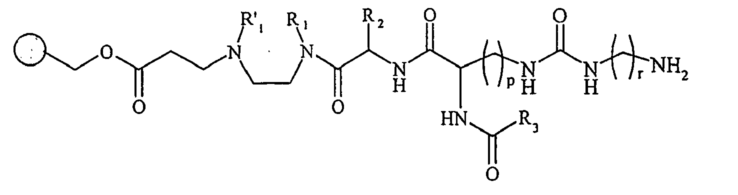 r étant tel que défini dans la revendication 2,
- la réaction du composé de formule (5) ou du composé de formule (6) avec des traceurs électrophiles, notamment avec A-Cl, pour obtenir le composé de formule (7) suivante : 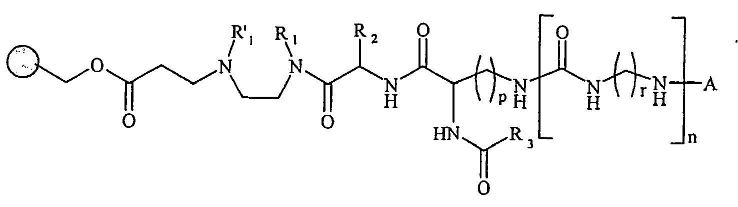 n étant égal à 0 ou 1,
A représentant le Bodipy sous forme d'acide activé ou la lissamine sous forme d'acide sulfonique activé
- le clivage du composé de formule (7) avec le composé R₄X, R₄ étant tel que défini dans la revendication 2 et X représentant un atome d'halogène, notamment I ou Br,
pour obtenir un composé de formule (I) selon la revendication 2.

## Claims

1. Use of a collection of compounds corresponding to the following general formula (I): 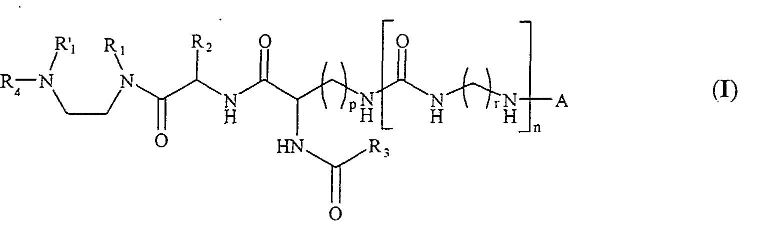 in which:
- n is equal to 0 or 1,
- p represents an integer varying from 1 to 6, and is preferably equal to 4,
- r represents an integer varying from 1 to 12, and is preferably equal to 4,
- R₁ and R'₁ represent independently of each other a hydrogen atom, an alkyl group comprising from 1 to 6 carbon atoms, or an aromatic group, in particular a benzyl group or a phenethyl group, optionally substituted by a halogen atom, a methoxy group or an alkyl group comprising from 1 to 6 carbon atoms,
or R₁ and R'₁ can form a ring comprising from 2 to 4 carbon atoms, in particular 2 carbon atoms,
- R₂ represents a side chain of an amino acid or of an amino acid derivative,
- R₃ represents a group derived from a carboxylic acid, carrying a basic entity, in particular selected from the following groups: 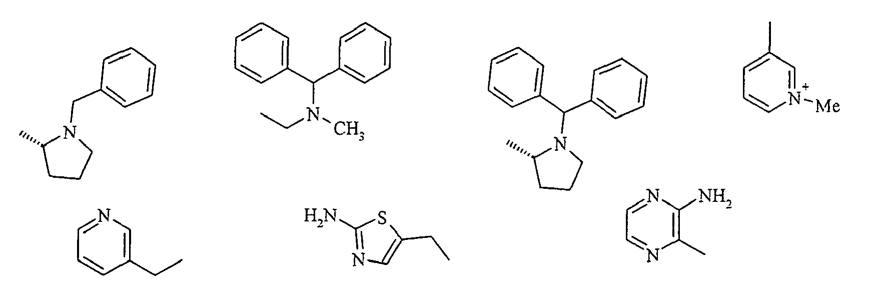
- R₄ represents an alkyl group comprising from 1 to 10 carbon atoms, in particular from 1 to 6 carbon atoms, a phenyl group substituted by an alkyl group comprising from 1 to 10 carbon atoms, in particular from 1 to 6 carbon atoms, R₄ preferably being a methyl group, a benzyl group or an allyl group, and
- A represents a hydrogen atom, a protective group or a tracer group, in particular a fluorophore, a dye or a "quencher",
for the *in vitro* determination, by binding studies, of ligands of receptors no ligand of which is known or no usable ligand of which is known in order to carry out specific affinity binding studies.

2. A compound of the following formula (I): 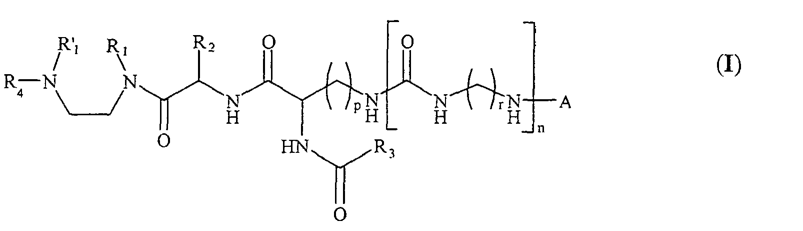 in which:
- n is equal to 0 or 1,
- p represents an integer varying from 1 to 6, and is preferably equal to 4,
- r represents an integer varying from 1 to 12, and is preferably equal to 4,
- R₁ and R'₁ represent independently of each other a hydrogen atom, an alkyl group comprising from 1 to 6 carbon atoms, or an aromatic group, in particular a benzyl group or a phenethyl group, optionally substituted by a halogen atom, a methoxy group or an alkyl group comprising from 1 to 6 carbon atoms,
or R₁ and R'₁ can form a ring comprising from 2 to 4 carbon atoms, in particular 2 carbon atoms,
- R₂ represents a side chain of an amino acid or of an amino acid derivative,
- R₃ represents a group derived from a carboxylic acid, carrying a basic entity, in particular selected from the following groups: 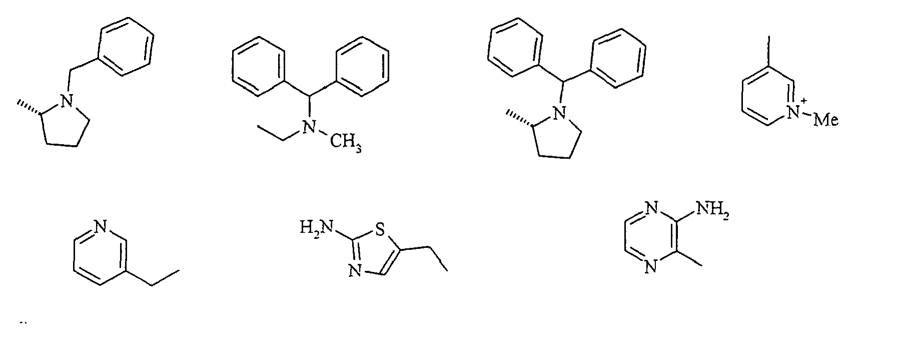
- R₄ represents an alkyl group comprising from 1 to 10 carbon atoms, in particular from 1 to 6 carbon atoms, a phenyl group substituted by an alkyl group comprising from 1 to 10 carbon atoms, in particular from 1 to 6 carbon atoms, R₄ preferably being a methyl group, a benzyl group or an allyl group, and
- A represents a hydrogen atom, a protective group or a tracer group, in particular a fluorophore, a dye or a "quencher''.

3. The compound according to claim 2, **characterized in that** R₂ represents the side chain of an amino acid or of amino acid derivative selected from the group comprising: alanine, glutamine, leucine, glycine, tryptophan, β-alanine, phenylalanine, 4-chloro-phenylalanine, isonipecotinic acid, 4-aminomethylbenzoic acid, 3-tetrahydroisoquinolinic acid and free or benzylated histidine.

4. The compound according to claim 2 or 3, **characterized in that** n is equal to 0.

5. The compound according to claim 2 or 3, **characterized in that** n is equal to 1.

6. The compound according to claim 4, **characterized in that** A represents a hydrogen atom or a protective group, in particular a Boc group.

7. The compound according to claim 6, corresponding to one of the following formulae: 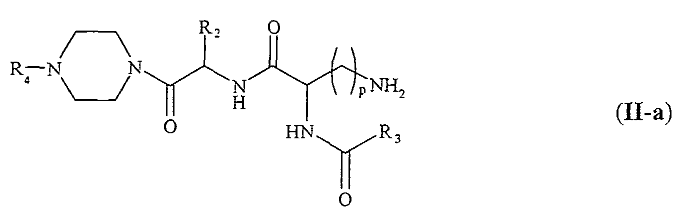 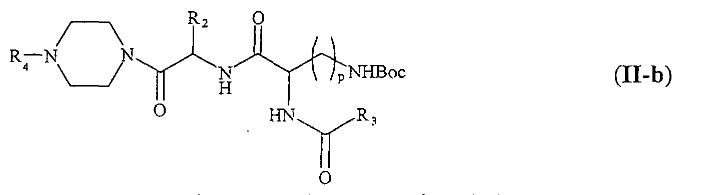 in which R₂, R₃, R₄ and p are as defined in claim 2, p preferably being equal to 4.

8. The compound according to claim 4 or 5, **characterized in that** A represents a fluorophore group selected from the group comprising Bodipy and lissamine derivatives.

9. The compound according to claim 8, **characterized in that** A represents one of the following groups:

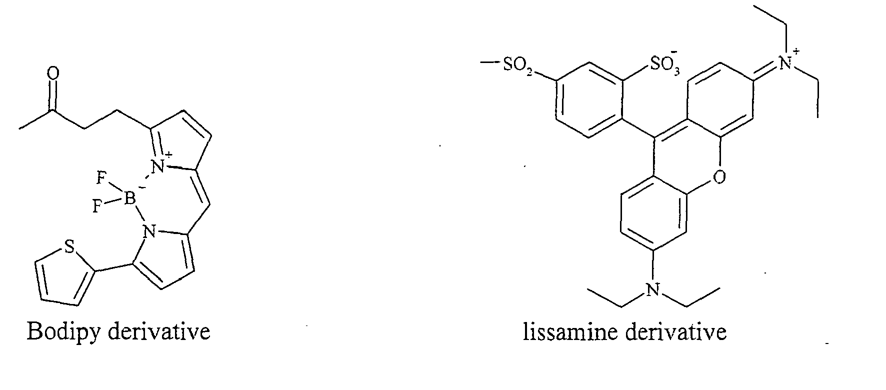

10. The compound according to claim 8 or 9, corresponding to the following formula (III): 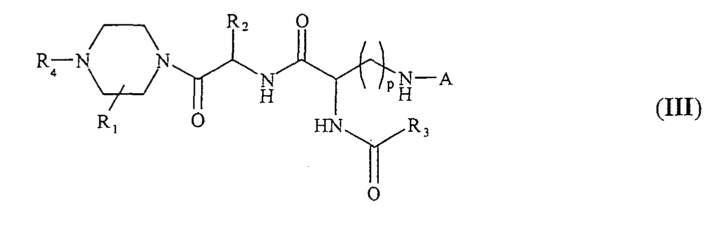 in which:
- R₁, R₂, R₃, R₄ and p are as defined in claim 2 and
- A is as defined in claim 8 or 9.

11. The compound according to claim 10, **characterized in that** p = 4 and corresponding to the following formula: 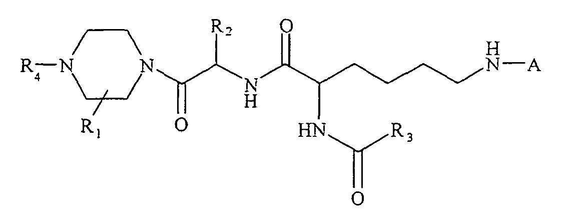 in which R₁, R₃ and R₄ are as defined in claim 2 and R₂ is as defined in claim 3.

12. The compound according to claim 10 or 11, corresponding to the following formula (III-a): 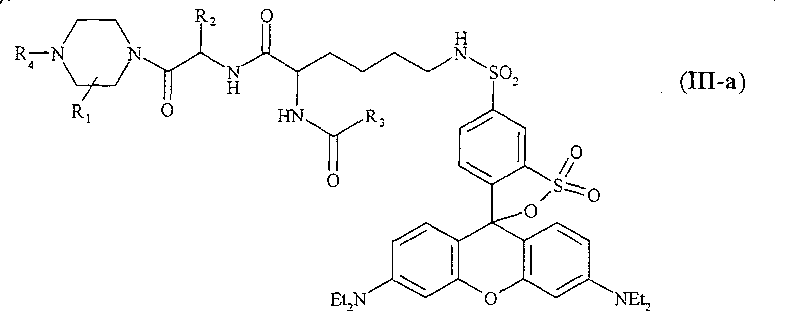 in which R₁, R₃ and R₄ are as defined in claim 2 and R₂ is as defined in claim 3.

13. The compound according to claim 8 or 9, corresponding to the following formula (IV): 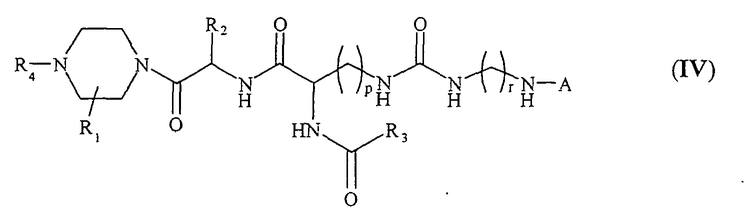 in which:
- R₁, R₂, R₃, R₄, p and r are as defined in claim 2 and
- A is as defined in claim 8 or 9.

14. The compound according to claim 13, **characterized in that** p = 4 and corresponding to the following formula:

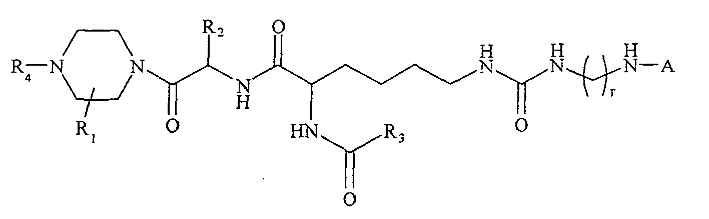

15. The compound according to claim 13, **characterized in that** r = 4 and corresponding to the following formula:

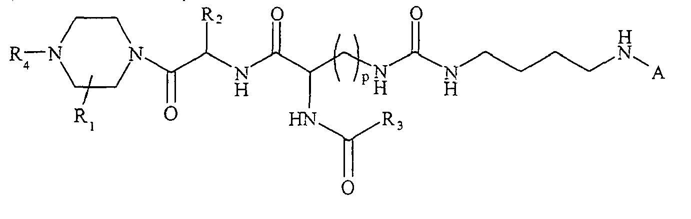

16. The compound according to claim 13, **characterized in that** p and r are equal to 4 and corresponding to the following formula:

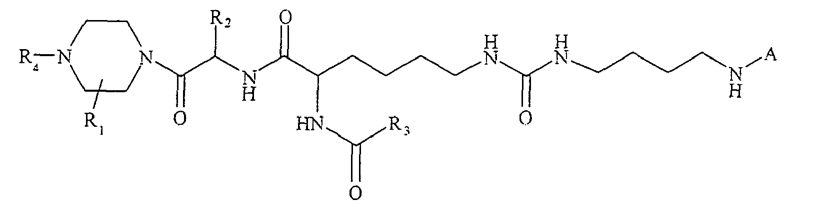

17. The compound according to claim 13, corresponding to the following formula (IV-a): 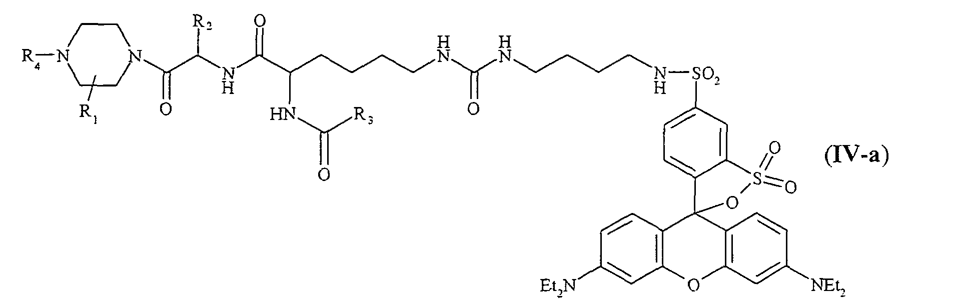 in which R₁, R₃ and R₄ are as defined in claim 2 and R₂ is as defined in claim 3.

18. A collection comprising a plurality of compounds of formula (I) as defined in claim 2.

19. A collection comprising a plurality of compounds of formula (III-a) as defined in claim 12.

20. A collection comprising a plurality of compounds of formula (IV-a) as defined in claim 17.

21. The use of a collection according to claim 19 or 20, for the *in vitro* determination, by binding studies, of ligands of receptors no ligand of which is known or no usable ligand of which is known to carry out specific affinity binding studies.

22. A process for screening ligands of receptors no ligand of which is known or no usable ligand of which is known, said process comprising the following steps:
- bringing a collection of traceable compounds of formula (I) according to claim 2, together with cells transfected by a construction containing the fusion of the sequence coding for a fluorescent protein with the nucleotide sequence coding for a receptor no ligand of which is known or no usable ligand of which is known, and the mixture of said cells and of said collection,
- detection of the fluorescence of said mixture, by excitation of said fluorescent protein and measurement of the emission fluorescence of said fluorescent protein, and determination of the fluorescence extinction percentage by comparing the emission fluorescence of said fluorescent protein to the average fluorescence of said fluorescent protein,
the average fluorescence of said fluorescent protein being measured by control tests corresponding to the measurement of the fluorescence of the fluorescent protein in the absence of the collection of compounds, and
- determination of the compounds which produce a fluorescence extinction percentage of the fluorescent protein of at least 5% and their identification as ligand.

23. A process for the preparation on solid support of a compound of formula (I) according to claim 2, **characterized in that** it comprises the following steps:
- the coupling of a solid support of formula 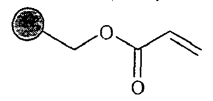 to a compound of formula 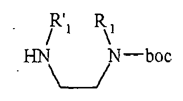 in order to obtain a compound of the following formula (1): 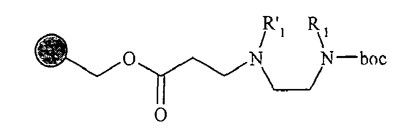 R₁ and R' ₁ being as defined in claim 2,
- the deprotection of the compound of formula (1) followed by the coupling of said deprotected compound to the compound of formula 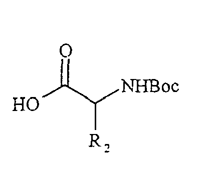 in order to obtain a compound of the following formula (2): 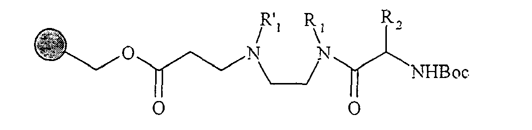 R₂ being as defined in claim 2,
- the deprotection of the compound of formula (2) followed by the coupling of said deprotected compound to the compound of formula 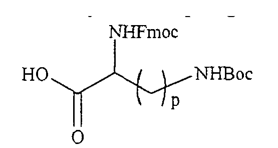 in order to obtain a compound of the following formula (3): 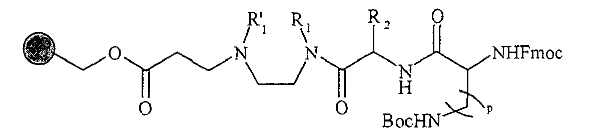 p being as defined in claim 2,
- the deprotection of the Fmoc group of the compound of formula (3) followed by the coupling of said deprotected compound to the compound R₃COOH in order to obtain a compound of the following formula (4): 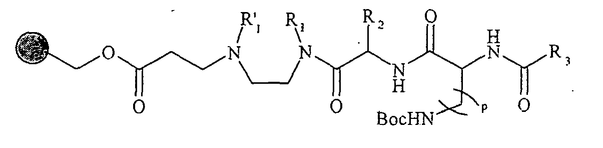 R₃ being as defined in claim 2,
- the deprotection of the compound of formula (4) in order to obtain a compound of the following formula (5): 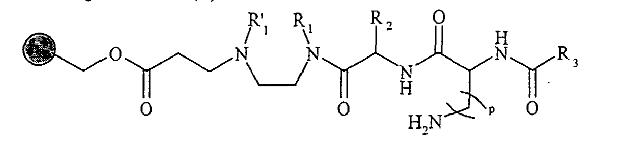
- optionally the reaction of the compound of formula (5) with p-nitrophenylchloroformate then with the diamine of formula 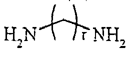 in order to obtain the compound of the following formula (6): 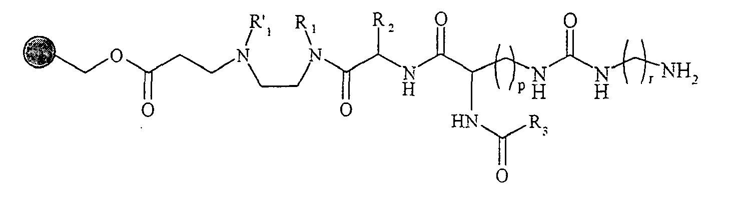 r being as defined in claim 2,
- the reaction of the compound of formula (5) or of the compound of formula (6) with electrophilic tracers, in particular with A-Cl, in order to obtain the compound of the following formula (7): 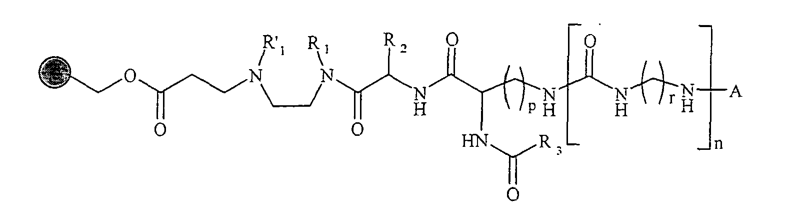 n being equal to 0 or 1,
A representing Bodipy in the form of activated acid or lissamine in the form of activated sulphonic acid,
- the cleavage of the compound of formula (7) with the compound R₄X, R₄ being as defined in claim 2, and X representing a halogen atom, in particular I or Br, in order to obtain a compound of formula (I) according to claim 2.

## Patentansprüche

1. Verwendung einer Kollektion von Verbindungen, die der folgenden allgemeinen Formel (I) entsprechen: 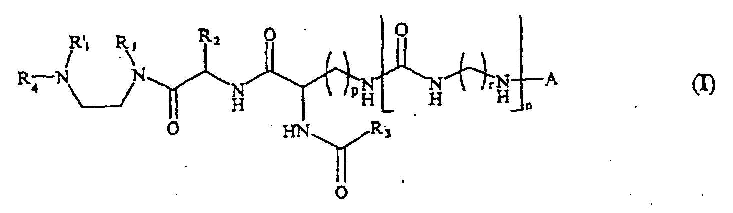 wobei:
- n gleich 0 oder 1 ist,
- p eine ganze Zahl zwischen 1 und 6 darstellt und vorzugsweise gleich 4 ist,
- r eine ganze Zahl zwischen 1 und 12 darstellt und vorzugsweise gleich 4 ist,
- R₁ und R'₁ unabhängig voneinander ein Wasserstoffatom, eine Alkylgruppe, umfassend 1 bis 6 Kohlenstoffatome, oder eine aromatische Gruppe, insbesondere eine Benzylgruppe oder eine Phenethylgruppe, die eventuell durch ein Halogenatom, eine Methoxygruppe oder eine Alkylgruppe, umfassend 1 bis 6 Kohlenstoffatome substituiert sind, darstellt,
oder R₁ und R'₁ einen Zyklus bilden können, umfassend 2 bis 4 Kohlenstoffatome, insbesondere 2 Kohlenstoffatome,
- R₂ eine seitliche Aminosäurenkette oder ein Aminosäurenderivat darstellt;
- R₃ eine von einer Karboxylsäure abgeleitete Gruppe darstellt, die Träger einer basischen Einheit ist, die insbesondere unter folgenden Gruppen ausgewählt wird: 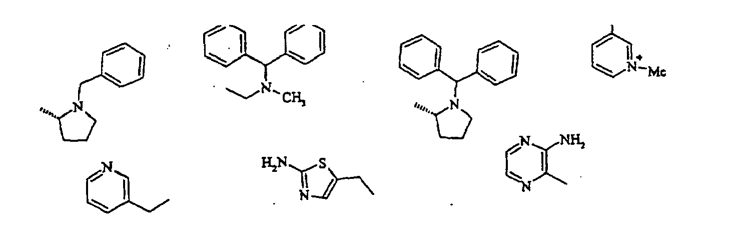
- R₄ eine Alkylgruppe, umfassend 1 bis 10 Kohlenstoffatome, insbesondere 1 bis 6 Kohlenstoffatome, eine Phenylgruppe, die durch eine Alkylgruppe substituiert ist, umfassend 1 bis 10 Kohlenstoffatome, insbesondere 1 bis 6 Kohlenstoffatome, wobei R₄ vorzugsweise eine Methylgruppe, eine Benzylgruppe und eine Allylgruppe ist, und
- A ein Wasserstoffatom, eine Schutzgruppe oder eine Zuggruppierung, insbesondere ein Fluorphor, einen Farbstoff oder einen "Quencher" darstellt,
um in vitro durch Verbindungsstudie Rezeptorliganden zu bestimmen, von denen kein Ligand bekannt ist oder von denen kein Ligand bekannt ist, der verwendbar ist, um Verbindungstests von spezifischer Affinität durchzuführen.

2. Verbindung mit folgender Formel (I): 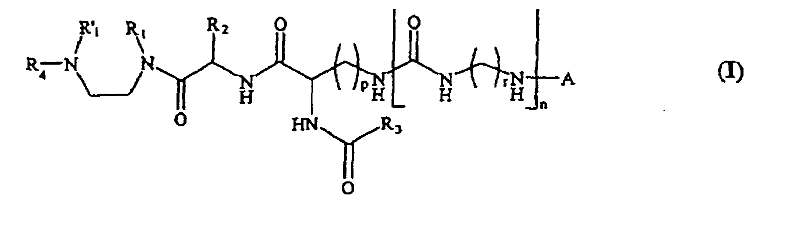 wobei:
- n gleich 0 oder 1 ist,
- p eine ganze Zahl zwischen 1 und 6 darstellt und vorzugsweise gleich 4 ist,
- r eine ganze Zahl zwischen 1 und 12 darstellt und vorzugsweise gleich 4 ist,
- R₁ und R'₁ unabhängig voneinander ein Wasserstoffatom, eine Alkylgruppe, umfassend 1 bis 6 Kohlenstoffatome, oder eine aromatische Gruppe, insbesondere eine Benzylgruppe oder eine Phenethylgruppe, die eventuell durch ein Halogenatom, eine Methoxygruppe oder eine Alkylgruppe, umfassend 1 bis 6 Kohlenstoffatome substituiert sind, darstellt,
oder R₁ und R'₁ einen Zyklus bilden können, umfassend 2 bis 4 Kohlenstoffatome, insbesondere 2 Kohlenstoffatome,
- R₂ eine seitliche Kette einer Aminosäure oder eines Aminosäurenderivats darstellt;
- R₃ eine von einer Karboxylsäure abgeleitete Gruppe darstellt, die Träger einer basischen Einheit ist, die insbesondere unter folgenden Gruppen ausgewählt wird: 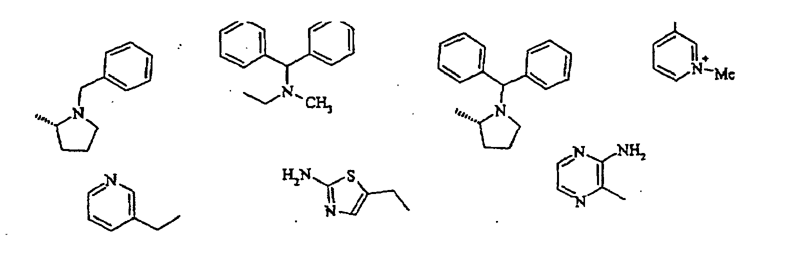
- R₄ eine Alkylgruppe, umfassend 1 bis 10 Kohlenstoffatome, insbesondere 1 bis 6 Kohlenstoffatome, eine Phenylgruppe, die durch eine Alkylgruppe substituiert ist, umfassend 1 bis 10 Kohlenstoffatome, insbesondere 1 bis 6 Kohlenstoffatome, wobei R₄ vorzugsweise eine Methylgruppe, eine Benzylgruppe und eine Allylgruppe ist, und
- A ein Wasserstoffatom, eine Schutzgruppe oder eine Zuggruppierung, insbesondere ein Fluorphor, einen Farbstoff oder einen "Quencher" darstellt.

3. Verbindung nach Anspruch 2, **dadurch gekennzeichnet, dass** R₂ die seitliche Kette einer Aminosäure oder eines Aminosäurenderivats darstellt, das in folgender Gruppe ausgewählt wird: Alanin, Glutamin, Leukin, Glycin, Tryptophan, β-Alanin, Phenylalanin, 4-Chlor-Phenylalanin, Isonipekotinsäure, 4-Aminomethylbenzolsäure, 3-Tetrahydroisoquinoleinsäure und freies oder benzyliertes Histidin.

4. Verbindung nach Anspruch 2 oder 3, **dadurch gekennzeichnet, dass** n gleich 0 ist.

5. Verbindung nach Anspruch 2 oder 3, **dadurch gekennzeichnet, dass** n gleich 1 ist.

6. Verbindung nach Anspruch 4, **dadurch gekennzeichnet, dass** A ein Wasserstoffatom oder eine Schutzgruppe, insbesondere eine Boc-Gruppe darstellt.

7. Verbindung nach Anspruch 6, die einer der folgenden Formeln entspricht: 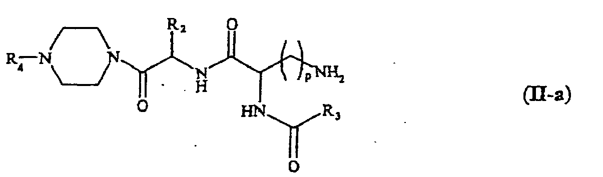 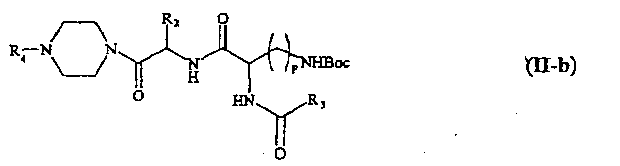 wobei R₂, R₃, R₄ und p wie in Anspruch 2 definiert sind, wobei p vorzugsweise gleich 4 ist.

8. Verbindung nach Anspruch 4 oder 5, **dadurch gekennzeichnet, dass** A eine Fluorphorgruppe darstellt, die in der Gruppe ausgewählt wird, die die Derivate des Bodipy und des Lissamins enthält.

9. Verbindung nach Anspruch 8, **dadurch gekennzeichnet, dass** A eine der folgenden Gruppen darstellt:

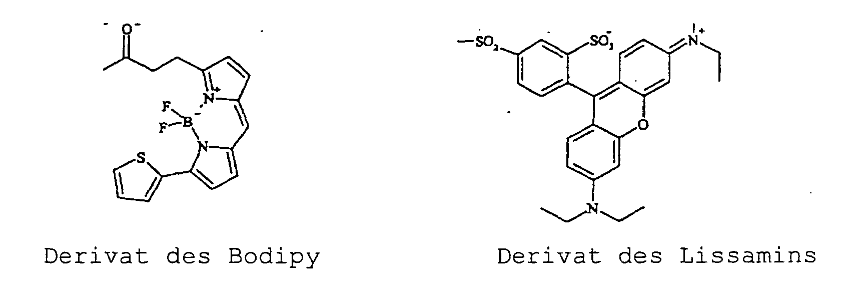

10. Verbindung nach Anspruch 8 oder 9, die der folgenden Formel (III) entspricht: 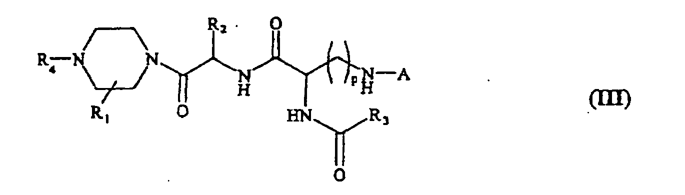 wobei
- R₁, R₂, R₃, R₄ wie in Anspruch 2 definiert sind, und
- A wie in Anspruch 8 oder 9 definiert ist.

11. Verbindung nach Anspruch 10, **dadurch gekennzeichnet, dass** p = 4 und der folgenden Formel entspricht: 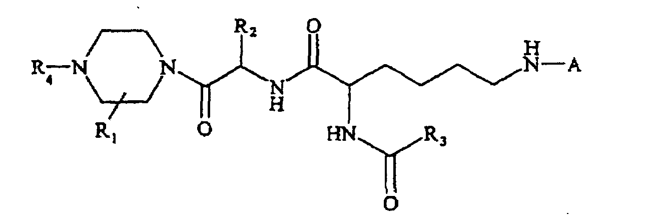 wobei R₁, R₃, R₄ wie in Anspruch 2 definiert sind und R₂ wie in Anspruch 3 definiert ist.

12. Verbindung nach Anspruch 10 oder 11, die der folgenden Formel (III-a) entspricht: 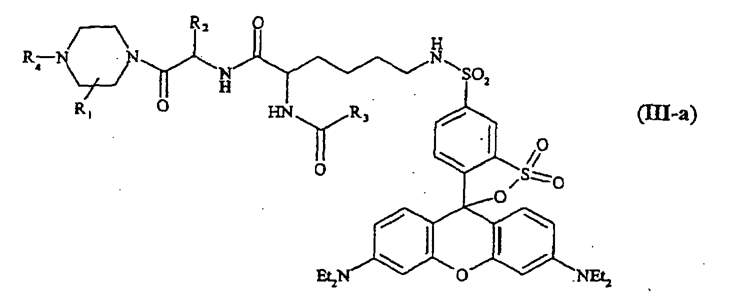 wobei R₁, R₃, R₄ wie in Anspruch 2 definiert sind und R₂ wie in Anspruch 3 definiert ist.

13. Verbindung nach Anspruch 8 oder 9, die der folgenden Formel (IV) entspricht: 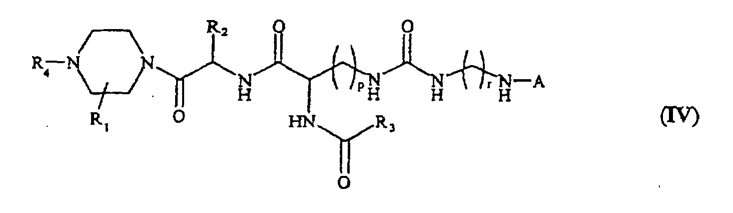 wobei
- R₁, R₂, R₃, R₄, p und r wie in Anspruch 2 definiert sind, und
- A wie in Anspruch 8 oder 9 definiert ist.

14. Verbindung nach Anspruch 13, **dadurch gekennzeichnet, dass** p = 4 und der folgenden Formel entspricht:

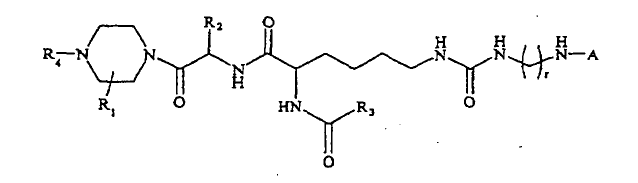

15. Verbindung nach Anspruch 13, **dadurch gekennzeichnet, dass** r = 4 und der folgenden Formel entspricht:

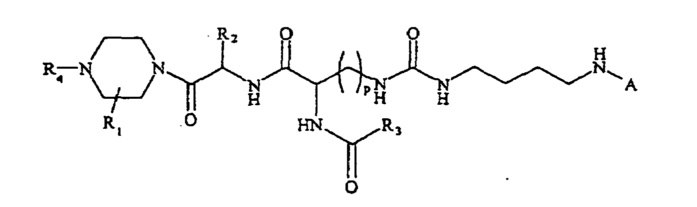

16. Verbindung nach Anspruch 13, **dadurch gekennzeichnet, dass** p und r gleich 4 sind und der folgenden Formel entsprechen:

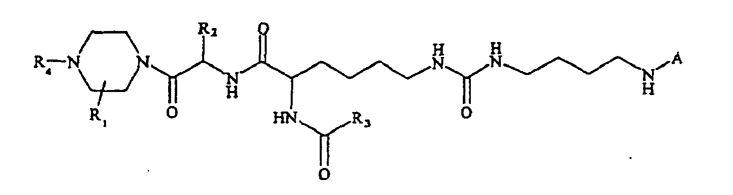

17. Verbindung nach Anspruch 13, die der folgenden Formel (IV-a) entspricht: 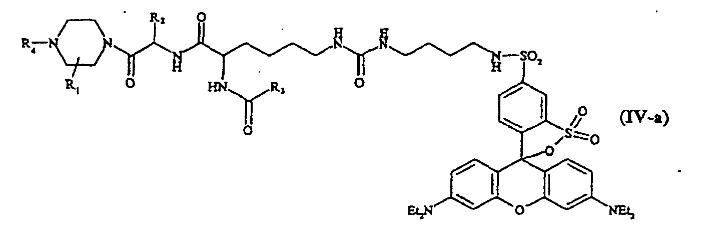 wobei R₁, R₃, R₄ wie in Anspruch 2 definiert sind und R₂ wie in Anspruch 3 definiert ist.

18. Kollektion, umfassend eine Vielzahl von Verbindungen mit der Formel (I), wie in Anspruch 2 definiert.

19. Kollektion, umfassend eine Vielzahl von Verbindungen mit der Formel (III-a), wie in Anspruch 12 definiert.

20. Kollektion, umfassend eine Vielzahl von Verbindungen mit der Formel (IV-a), wie in Anspruch 17 definiert.

21. Verwendung einer Kollektion nach Anspruch 19 oder 20 für die In-vitro-Bestimmung von Rezeptorliganden durch Verbindungsstudie, von denen kein Ligand bekannt ist oder von denen kein Ligand bekannt ist, der verwendet werden kann, um Verbindungsstudien von spezifischer Affinität durchzuführen.

22. Auswahlverfahren von Rezeptorliganden, von denen kein Ligand bekannt ist, oder von denen kein verwendbarer Ligand bekannt ist, wobei das Verfahren die folgenden Schritte umfasst:
- Zusammenbringen einer Kollektion von nachvollziehbaren Verbindungen mit der Formel (I) nach Anspruch 2, mit transfizierten Zellen durch eine Konstruktion, die die Fusion der für ein fluoreszierendes Protein codierenden Sequenz enthält, mit der Nukleotidsequenz, die für einen Rezeptor codiert, dessen Ligand nicht bekannt ist oder von dem kein verwendbarer Ligand bekannt ist, und Mischen dieser Zellen und der Kollektion,
- Erfassung der Fluoreszenz der Mischung durch Erregung des fluoreszierenden Proteins und Messen der Sendefluoreszenz des fluoreszierenden Proteins, und Bestimmung des Löschprozentsatzes der Fluoreszenz durch Vergleichen der Sendefluoreszenz des fluoreszierenden Proteins mit der durchschnittlichen Fluoreszenz des fluoreszierenden Proteins,
wobei die durchschnittliche Fluoreszenz des fluoreszierenden Proteins durch Beweistests gemessen wird, die der Messung der Fluoreszenz des fluoreszierenden Proteins bei Nichtvorhandensein der Kollektion von Verbindungen entspricht, und
- Bestimmung der Verbindungen, die einen Löschprozentsatz der Fluoreszenz des fluoreszierenden Proteins von mindestens 5 % und ihre Identifikation als Ligand ergeben.

23. Verfahren zur Herstellung eines festen Trägers einer Verbindung mit der Formel (I) nach Anspruch 2, **dadurch gekennzeichnet, dass** es die folgenden Schritte umfasst:
- Kopplung eines festen Trägers mit der Formel 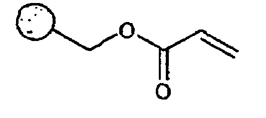 mit einer Mischung mit der Formel 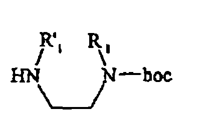 um eine Verbindung mit folgender Formel (1) zu erhalten: 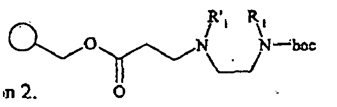
wobei R₁ und R'₁ wie in Anspruch 2 definiert sind,
- Aufheben des Schutzes der Verbindung mit der Formel (1), gefolgt von der Kopplung der ungeschützten Verbindung mit der Verbindung mit der Formel 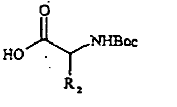 um eine Verbindung mit folgender Formel (2) zu erhalten: 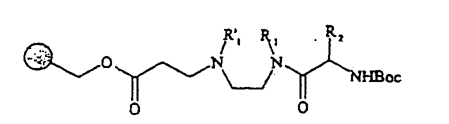
wobei R₂ wie in Anspruch 2 definiert ist,
- Aufheben des Schutzes der Verbindung mit der Formel (2), gefolgt von der Kopplung der ungeschützten Verbindung mit der Verbindung mit der Formel 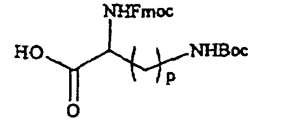
um eine Verbindung mit folgender Formel (3) zu erhalten: 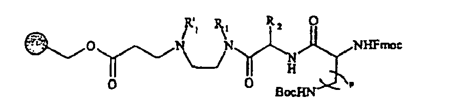 wobei p wie in Anspruch 2 definiert ist,
- Aufheben des Schutzes der Gruppe Fmoc der Verbindung mit der Formel (3), gefolgt von der Kopplung der ungeschützten Verbindung mit der Verbindung R₃COOH, um eine Verbindung mit folgender Formel () zu erhalten: 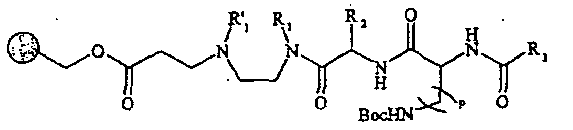
wobei R₃ wie in Anspruch 2 definiert ist,
- Aufheben des Schutzes der Verbindung mit der Formel (4), um eine Verbindung mit folgender Formel (5) zu erhalten: 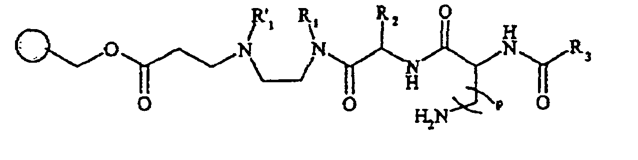
- eventuell Reaktion der Verbindung mit der Formel (5) mit p-Nitrophenylchlorformat, dann mit Diamin 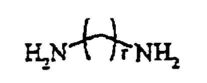 mit der Formel, um die Verbindung mit folgender Formel (6) zu erhalten: 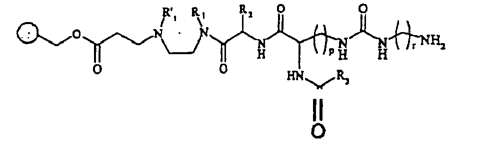
wobei r wie in Anspruch 2 definiert ist,
- Reaktion der Verbindung mit der Formel (5) oder der Verbindung mit der Formel (6) mit elektrophilen Indikatoren, insbesondere mit A-Cl, um die Verbindung mit folgender Formel (7) zu erhalten: 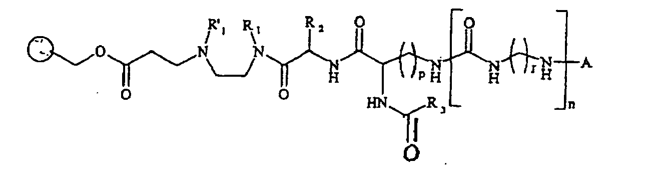
wobei n gleich 0 oder 1 ist,
wobei A das Bodipy in Form einer aktivierten Säure oder das Lissamin in Form einer aktivierten Sulfosäure darstellt,
- Spaltung der Verbindung mit der Formel (7) mit der Verbindung R₄X, wobei R₄ wie in Anspruch 2 definiert ist, und X ein Halogenatom, insbesondere I oder Br, darstellt,
um eine Verbindung mit der Formel (I) nach Anspruch 2 zu erhalten.
